# EUROPEAN PATENT SPECIFICATION

(11) **EP 2 104 501 B1**
(45) Date of publication and mention of the grant of the patent: **12.03.2014**
(21) Application number: 07862806.2
(22) Date of filing: 11.12.2007
(51) Int. Cl.: A61K 31/519, A61K 39/395, A61K 45/06, A61P 35/00, A61P 35/02

(54) **METHODS OF CANCER TREATMENT WITH IGF1R INHIBITORS**
VERFAHREN ZUR KREBSBEHANDLUNG MIT IGF1R-INHIBITOREN
METHODES DE TRAITEMENT DU CANCER AVEC DES INHIBITEURS IGF1R

(30) Priority: 13.12.2006 US 874589 P; 20.12.2006 US 870937 P; 25.06.2007 US 946011 P; 11.10.2007 US 979274 P
(43) Date of publication of application: 30.09.2009
(73) Proprietor: Merck Sharp & Dohme Corp., Rahway, NJ 07065-0907 (US)
(72) Inventor: WANG, Yan, Scotch Plains, NJ 07076 (US); ZONG, Chen, Metuchen, NJ 08840 (US); SEIDEL-DUGAN, Cynthia, Mountainside, NJ 07902 (US); WANG, Yaolin, Edison, NJ 08820 (US); YAO, Siu-long, West Windsor, NJ 08550 (US); LU, Brian Der-hua, Westfield, NJ 07090 (US); LADHA, Mohamed H., Parsippany, NJ 07054 (US)
(74) Representative: Buchan, Gavin MacNicol
(86) International application number: PCT/US2007/025398
(87) International publication number: WO 2008/076278

(56) References cited:
- WO-A-02/092599
- WO-A-2005/082415
- WO-A-2006/113483
- RIEDEMANN J ET AL: "IGF1R signalling and its inhibition" ENDOCRINE-RELATED CANCER 200612 GB, vol. 13, no. SUPPL. 1, 1 December 2006 (2006-12-01), pages S33-S43, XP002481460 ISSN: 1351-0088
- MITSIADES CONSTANTINE S ET AL: "Inhibition of the insulin-like growth factor receptor-1 tyrosine kinase activity as a therapeutic strategy for multiple myeloma, other hematologic malignancies, and solid tumors" CANCER CELL, vol. 5, no. 3, March 2004 (2004-03), pages 221-230, XP002481461 ISSN: 1535-6108
- GARCIA-ECHEVERRIA C: "Medicinal chemistry approaches to target the kinase activity of IGF-1R" IDRUGS 200606 GB, vol. 9, no. 6, June 2006 (2006-06), pages 415-419, XP009100637 ISSN: 1369-7056
- SCOTLANDI KATIA ET AL: "Antitumor activity of the insulin-like growth factor-I receptor kinase inhibitor NVP-AEW541 in musculoskeletal tumors" CANCER RESEARCH, vol. 65, no. 9, May 2005 (2005-05), pages 3868-3876,3861, XP002481462 ISSN: 0008-5472
- SARMA P K S ET AL: "Progress in the development of small molecule inhibitors of insulin-like growth factor-1 receptor kinase" EXPERT OPINION ON THERAPEUTIC PATENTS 200701 GB, vol. 17, no. 1, January 2007 (2007-01), pages 25-35, XP002481463 ISSN: 1354-3776
- DEEPALI SACHDEV ET AL: "Inhibitors of Insulin-like Growth Factor Signaling: A Therapeutic Approach for Breast Cancer" JOURNAL OF MAMMARY GLAND BIOLOGY AND NEOPLASIA, KLUWER ACADEMIC PUBLISHERS-PLENUM PUBLISHERS, NE, vol. 11, no. 1, 9 August 2006 (2006-08-09), pages 27-39, XP019400879 ISSN: 1573-7039
- WANG Y ET AL: "Inhibition of insulin-like growth factor-I receptor (IGF-IR) signaling and tumor cell growth by a fully human neutralizing anti-IGF-IR antibody" MOL CANCER THER,, vol. 4, no. 8, 1 August 2005 (2005-08-01), pages 1214-1221, XP002496476

### SEQUENCE LISTING

<110> Schering Corporation
<120> Methods of Treatment
<130> JB06578
<150> 60/874,589
   <151> 2006-12-13
<150> 60/870,937
   <151> 2006-12-20
<150> 60/946,011
   <151> 2007-06-25
<150> 60/979,274
   <151> 2007-10-11
<160> 108
<170> PatentIn version 3.3
<210> 1
   <211> 384
   <212> DNA
   <213> Artificial Sequence
<220>
   <223> Modified 19D12/15H12 Light Chain-C
<220>
   <221> CDS
   <222> (1) .. (384)
<400> 1
   atg tcg cca tca caa ctc att ggg ttt ctg ctg ctc tgg gtt cca gcc       48
   Met Ser Pro Ser Gln Leu Ile Gly Phe Leu Leu Leu Trp Val Pro Ala
   1               5                   10                  15
   tcc agg ggt gaa att gtg ctg act cag agc cca gac tct ctg tct gtg       96
   Ser Arg Gly Glu Ile Val Leu Thr Gln Ser Pro Asp Ser Leu Ser Val
               20                  25                  30
   act cca ggc gag aga gtc acc atc acc tgc cgg gcc agt cag agc att      144
   Thr Pro Gly Glu Arg Val Thr Ile Thr Cys Arg Ala Ser Gln Ser Ile
           35                  40                  45
   ggt agt agc tta cac tgg tac cag cag aaa cca ggt cag tct cca aag      192
   Gly Ser Ser Leu His Trp Tyr Gln Gln Lys Pro Gly Gln Ser Pro Lys
       50                  55                  60
   ctt ctc atc aag tat gca tcc cag tcc ctc tca ggg gtc ccc tcg agg      240
   Leu Leu Ile Lys Tyr Ala Ser Gln Ser Leu Ser Gly Val Pro Ser Arg
   65                  70                  75                  80
   ttc agt ggc agt gga tct ggg aca gat ttc acc ctc acc atc agt agc      288
   Phe Ser Gly Ser Gly Ser Gly Thr Asp Phe Thr Leu Thr Ile Ser Ser
                   85                  90                  95
   ctc gag gct gaa gat gct gca gcg tat tac tgt cat cag agt agt cgt      336
   Leu Glu Ala Glu Asp Ala Ala Ala Tyr Tyr Cys His Gln Ser Ser Arg
               100                 105                 110
   tta cct cac act ttc ggc caa ggg acc aag gtg gag atc aaa cgt acg      384
   Leu Pro His Thr Phe Gly Gln Gly Thr Lys Val Glu Ile Lys Arg Thr
           115                 120                 125
<210> 2
   <211> 128
   <212> PRT
   <213> Artificial Sequence
<220>
   <223> Synthetic Construct
<400> 2
   Met Ser Pro Ser Gln Leu Ile Gly Phe Leu Leu Leu Trp Val Pro Ala
   1               5                   10                  15
   Ser Arg Gly Glu Ile Val Leu Thr Gln Ser Pro Asp Ser Leu Ser Val
               20                  25                  30
   Thr Pro Gly Glu Arg Val Thr Ile Thr Cys Arg Ala Ser Gln Ser Ile
           35                  40                  45
   Gly Ser Ser Leu His Trp Tyr Gln Gln Lys Pro Gly Gln Ser Pro Lys
       50                  55                  60
   Leu Leu Ile Lys Tyr Ala Ser Gln Ser Leu Ser Gly Val Pro Ser Arg
   65                  70                  75                  80
   Phe Ser Gly Ser Gly Ser Gly Thr Asp Phe Thr Leu Thr Ile Ser Ser
                   85                  90                  95
   Leu Glu Ala Glu Asp Ala Ala Ala Tyr Tyr Cys His Gln Ser Ser Arg
               100                 105                 110
   Leu Pro His Thr Phe Gly Gln Gly Thr Lys Val Glu Ile Lys Arg Thr
           115                 120                 125
<210> 3
   <211> 384
   <212> DNA
   <213> Artificial Sequence
<220>
   <223> Modified 19D12/15H12 Light Chain-D
<220>
   <221> CDS
   <222> (1)..(384)
<400> 3
   atg tcg cca tca caa ctc att ggg ttt ctg ctg ctc tgg gtt cca gcc       48
   Met Ser Pro Ser Gln Leu Ile Gly Phe Leu Leu Leu Trp Val Pro Ala
   1               5                   10                  15
   tcc agg ggt gaa att gtg ctg act cag agc cca gac tct ctg tct gtg       96
   Ser Arg Gly Glu Ile Val Leu Thr Gln Ser Pro Asp Ser Leu Ser Val
               20                  25                  30
   act cca ggc gag aga gtc acc atc acc tgc cgg gcc agt cag agc att      144
   Thr Pro Gly Glu Arg Val Thr Ile Thr Cys Arg Ala Ser Gln Ser Ile
           35                  40                  45
   ggt agt agc tta cac tgg tac cag cag aaa cca ggt cag tct cca aag      192
   Gly Ser Ser Leu His Trp Tyr Gln Gln Lys Pro Gly Gln Ser Pro Lys
       50                  55                  60
   ctt ctc atc aag tat gca tcc cag tcc ctc tca ggg gtc ccc tcg agg      240
   Leu Leu Ile Lys Tyr Ala Ser Gln Ser Leu Ser Gly Val Pro Ser Arg
   65                  70                  75                  80
   ttc agt ggc agt gga tct ggg aca gat ttc acc ctc acc atc agt agc      288
   Phe Ser Gly Ser Gly Ser Gly Thr Asp Phe Thr Leu Thr Ile Ser Ser
                   85                  90                  95
   ctc gag gct gaa gat ttc gca gtg tat tac tgt cat cag agt agt cgt      336
   Leu Glu Ala Glu Asp Phe Ala Val Tyr Tyr Cys His Gln Ser Ser Arg
               100                 105                 110
   tta cct cac act ttc ggc caa ggg acc aag gtg gag atc aaa cgt acg      384
   Leu Pro His Thr Phe Gly Gln Gly Thr Lys Val Glu Ile Lys Arg Thr
           115                 120                 125
<210> 4
   <211> 128
   <212> PRT
   <213> Artificial Sequence
<220>
   <223> Synthetic Construct
<400> 4
   Met Ser Pro Ser Gln Leu Ile Gly Phe Leu Leu Leu Trp Val Pro Ala
   1               5                   10                  15
   Ser Arg Gly Glu Ile Val Leu Thr Gln Ser Pro Asp Ser Leu Ser Val
               20                  25                  30
   Thr Pro Gly Glu Arg Val Thr Ile Thr Cys Arg Ala Ser Gln Ser Ile
           35                  40                  45
   Gly Ser Ser Leu His Trp Tyr Gln Gln Lys Pro Gly Gln Ser Pro Lys
       50                  55                  60
   Leu Leu Ile Lys Tyr Ala Ser Gln Ser Leu Ser Gly Val Pro Ser Arg
   65                  70                  75                  80
   Phe Ser Gly Ser Gly Ser Gly Thr Asp Phe Thr Leu Thr Ile Ser Ser
                   85                  90                  95
   Leu Glu Ala Glu Asp Phe Ala Val Tyr Tyr Cys His Gln Ser Ser Arg
               100                 105                 110
   Leu Pro His Thr Phe Gly Gln Gly Thr Lys Val Glu Ile Lys Arg Thr
           115                 120                 125
<210> 5
   <211> 384
   <212> DNA
   <213> Artificial Sequence
<220>
   <223> Modified 19D12/15H12 Light Chain-E
<220>
   <221> CDS
   <222> (1)..(384)
<400> 5
   atg tcg cca tca caa ctc att ggg ttt ctg ctg ctc tgg gtt cca gcc      48
   Met Ser Pro Ser Gln Leu Ile Gly Phe Leu Leu Leu Trp Val Pro Ala
   1               5                   10                  15
   tcc agg ggt gaa att gtg ctg act cag agc cca ggt acc ctg tct gtg      96
   Ser Arg Gly Glu Ile Val Leu Thr Gln Ser Pro Gly Thr Leu Ser Val
               20                  25                  30
   tct cca ggc gag aga gcc acc ctc tcc tgc cgg gcc agt cag agc att     144
   Ser Pro Gly Glu Arg Ala Thr Leu Ser Cys Arg Ala Ser Gln Ser Ile
           35                  40                  45
   ggt agt agc tta cac tgg tac cag cag aaa cca ggt cag gct cca agg     192
   Gly Ser Ser Leu His Trp Tyr Gln Gln Lys Pro Gly Gln Ala Pro Arg
       50                  55                  60
   ctt ctc atc aag tat gca tcc cag tcc ctc tca ggg atc ccc gat agg     240
   Leu Leu Ile Lys Tyr Ala Ser Gln Ser Leu Ser Gly Ile Pro Asp Arg
   65                  70                  75                  80
   ttc agt ggc agt gga tct ggg aca gat ttc acc ctc acc atc agt aga      288
   Phe Ser Gly Ser Gly Ser Gly Thr Asp Phe Thr Leu Thr Ile Ser Arg
                   85                  90                  95
   ctg gag cct gaa gat gct gca gcg tat tac tgt cat cag agt agt cgt      336
   Leu Glu Pro Glu Asp Ala Ala Ala Tyr Tyr Cys His Gln Ser Ser Arg
               100                 105                 110
   tta cct cac act ttc ggc caa ggg acc aag gtg gag atc aaa cgt aca      384
   Leu Pro His Thr Phe Gly Gln Gly Thr Lys Val Glu Ile Lys Arg Thr
           115                 120                 125
<210> 6
   <211> 128
   <212> PRT
   <213> Artificial Sequence
<220>
   <223> Synthetic Construct
<400> 6
   Met Ser Pro Ser Gln Leu Ile Gly Phe Leu Leu Leu Trp Val Pro Ala
   1               5                   10                  15
   Ser Arg Gly Glu Ile Val Leu Thr Gln Ser Pro Gly Thr Leu Ser Val
               20                  25                  30
   Ser Pro Gly Glu Arg Ala Thr Leu Ser Cys Arg Ala Ser Gln Ser Ile
           35                  40                  45
   Gly Ser Ser Leu His Trp Tyr Gln Gln Lys Pro Gly Gln Ala Pro Arg
       50                  55                  60
   Leu Leu Ile Lys Tyr Ala Ser Gln Ser Leu Ser Gly Ile Pro Asp Arg
   65                  70                  75                  80
   Phe Ser Gly Ser Gly Ser Gly Thr Asp Phe Thr Leu Thr Ile Ser Arg
                   85                  90                  95
   Leu Glu Pro Glu Asp Ala Ala Ala Tyr Tyr Cys His Gln Ser Ser Arg
               100                 105                 110
   Leu Pro His Thr Phe Gly Gln Gly Thr Lys Val Glu Ile Lys Arg Thr
           115                 120                 125
<210> 7
   <211> 384
   <212> DNA
   <213> Artificial Sequence
<220>
   <223> 19D12/15H12 Light Chain-F
<220>
   <221> CDS
   <222> (1) .. (384)
<400> 7
   atg tcg cca tca caa ctc att ggg ttt ctg ctg ctc tgg gtt cca gcc       48
   Met Ser Pro Ser Gln Leu Ile Gly Phe Leu Leu Leu Trp Val Pro Ala
   1               5                   10                  15
   tcc agg ggt gaa att gtg ctg act cag agc cca ggt acc ctg tct gtg       96
   Ser Arg Gly Glu Ile Val Leu Thr Gln Ser Pro Gly Thr Leu Ser Val
               20                  25                  30
   tct cca ggc gag aga gcc acc ctc tcc tgc cgg gcc agt cag agc att      144
   Ser Pro Gly Glu Arg Ala Thr Leu Ser Cys Arg Ala Ser Gln Ser Ile
           35                  40                  45
   ggt agt agc tta cac tgg tac cag cag aaa cca ggt cag gct cca agg      192
   Gly Ser Ser Leu His Trp Tyr Gln Gln Lys Pro Gly Gln Ala Pro Arg
       50                  55                  60
   ctt ctc atc aag tat gca tcc cag tcc ctc tca ggg atc ccc gat agg      240
   Leu Leu Ile Lys Tyr Ala Ser Gln Ser Leu Ser Gly Ile Pro Asp Arg
   65                  70                  75                  80
   ttc agt ggc agt gga tct ggg aca gat ttc acc ctc acc atc agt aga      288
   Phe Ser Gly Ser Gly Ser Gly Thr Asp Phe Thr Leu Thr Ile Ser Arg
                   85                  90                  95
   ctg gag cct gaa gat ttc gca gtg tat tac tgt cat cag agt agt cgt      336
   Leu Glu Pro Glu Asp Phe Ala Val Tyr Tyr Cys His Gln Ser Ser Arg
               100                 105                 110
   tta cct cac act ttc ggc caa ggg acc aag gtg gag atc aaa cgt aca      384
   Leu Pro His Thr Phe Gly Gln Gly Thr Lys Val Glu Ile Lys Arg Thr
           115                 120                 125
<210> 8
   <211> 128
   <212> PRT
   <213> Artificial Sequence
<220>
   <223> Synthetic Construct
<400> 8
   Met Ser Pro Ser Gln Leu Ile Gly Phe Leu Leu Leu Trp Val Pro Ala
   1               5                   10                  15
   Ser Arg Gly Glu Ile Val Leu Thr Gln Ser Pro Gly Thr Leu Ser Val
   20 25 30
   Ser Pro Gly Glu Arg Ala Thr Leu Ser Cys Arg Ala Ser Gln Ser Ile
   35 40 45
   Gly Ser Ser Leu His Trp Tyr Gln Gln Lys Pro Gly Gln Ala Pro Arg
   50 55 60
   Leu Leu Ile Lys Tyr Ala Ser Gln Ser Leu Ser Gly Ile Pro Asp Arg
   65 70 75 80
   Phe Ser Gly Ser Gly Ser Gly Thr Asp Phe Thr Leu Thr Ile Ser Arg
   85 90 95
   Leu Glu Pro Glu Asp Phe Ala Val Tyr Tyr Cys His Gln Ser Ser Arg
   100 105 110
   Leu Pro His Thr Phe Gly Gln Gly Thr Lys Val Glu Ile Lys Arg Thr
   115 120 125
<210> 9
   <211> 411
   <212> DNA
   <213> Artificial Sequence
<220>
   <223> 19D12/15H12 heavy chain-A
<220>
   <221> CDS
   <222> (1)..(411)
<400> 9
   atg gag ttt ggg ctg agc tgg gtt ttc ctt gtt gct ata tta aaa ggt 48
   Met Glu Phe Gly Leu Ser Trp Val Phe Leu Val Ala Ile Leu Lys Gly
   1 5 10 15
   gtc cag tgt gag gtt cag ctg gtg cag tct ggg gga ggc ttg gta aag 96
   Val Gln Cys Glu Val Gln Leu Val Gln Ser Gly Gly Gly Leu Val Lys
   20 25 30
   cct ggg ggg tcc ctg aga ctc tcc tgt gca gcc tct gga ttc acc ttc 144
   Pro Gly Gly Ser Leu Arg Leu Ser Cys Ala Ala Ser Gly Phe Thr Phe
   35 40 45
   agt agc ttt gct atg cac tgg gtt cgc cag gct cca gga aaa ggt ctg 192
   Ser Ser Phe Ala Met His Trp Val Arg Gln Ala Pro Gly Lys Gly Leu
   50 55 60
   gag tgg ata tca gtt att gat act cgt ggt gcc aca tac tat gca gac 240
   Glu Trp Ile Ser Val Ile Asp Thr Arg Gly Ala Thr Tyr Tyr Ala Asp
   65                  70                  75                  80
   tcc gtg aag ggc cga ttc acc atc tcc aga gac aat gcc aag aac tcc      288
   Ser Val Lys Gly Arg Phe Thr Ile Ser Arg Asp Asn Ala Lys Asn Ser
                   85                  90                  95
   ttg tat ctt caa atg aac agc ctg aga gcc gag gac act gct gtg tat      336
   Leu Tyr Leu Gln Met Asn Ser Leu Arg Ala Glu Asp Thr Ala Val Tyr
               100                 105                 110
   tac tgt gca aga ctg ggg aac ttc tac tac ggt atg gac gtc tgg ggc      384
   Tyr Cys Ala Arg Leu Gly Asn Phe Tyr Tyr Gly Met Asp Val Trp Gly
           115                 120                 125
   caa ggg acc acg gtc acc gtc tcc tca                                  411
   Gln Gly Thr Thr Val Thr Val Ser Ser
       130                 135
<210> 10
   <211> 137
   <212> PRT
   <213> Artificial Sequence
<220>
   <223> Synthetic Construct
<400> 10
   Met Glu Phe Gly Leu Ser Trp Val Phe Leu Val Ala Ile Leu Lys Gly
   1               5                   10                  15
   Val Gln Cys Glu Val Gln Leu Val Gln Ser Gly Gly Gly Leu Val Lys
               20                  25                  30
   Pro Gly Gly Ser Leu Arg Leu Ser Cys Ala Ala Ser Gly Phe Thr Phe
           35                  40                  45
   Ser Ser Phe Ala Met His Trp Val Arg Gln Ala Pro Gly Lys Gly Leu
       50                  55                  60
   Glu Trp Ile Ser Val Ile Asp Thr Arg Gly Ala Thr Tyr Tyr Ala Asp
   65                  70                  75                  80
   Ser Val Lys Gly Arg Phe Thr Ile Ser Arg Asp Asn Ala Lys Asn Ser
                   85                  90                  95
   Leu Tyr Leu Gln Met Asn Ser Leu Arg Ala Glu Asp Thr Ala Val Tyr
               100                 105                 110
   Tyr Cys Ala Arg Leu Gly Asn Phe Tyr Tyr Gly Met Asp Val Trp Gly
           115                 120                 125
   Gln Gly Thr Thr Val Thr Val Ser Ser
       130                 135
<210> 11
   <211> 411
   <212> DNA
   <213> Artificial Sequence
<220>
   <223> Modified 19D12/15H12 heavy chain-B
<220>
   <221> CDS
   <222> (1) .. (411)
<400> 11
   atg gag ttt ggg ctg agc tgg gtt ttc ctt gtt gct ata tta aaa ggt       48
   Met Glu Phe Gly Leu Ser Trp Val Phe Leu Val Ala Ile Leu Lys Gly
   1               5                   10                  15
   gtc cag tgt gag gtt cag ctg gtg cag tct ggg gga ggc ttg gta cag       96
   Val Gln Cys Glu Val Gln Leu Val Gln Ser Gly Gly Gly Leu Val Gln
               20                  25                  30
   ccc ggg ggg tcc ctg aga ctc tcc tgt gca gcc tct gga ttc acc ttc      144
   Pro Gly Gly Ser Leu Arg Leu Ser Cys Ala Ala Ser Gly Phe Thr Phe
           35                  40                  45
   agt agc ttt gct atg cac tgg gtt cgc cag gct cca gga aaa ggt ctg      192
   Ser Ser Phe Ala Met His Trp Val Arg Gln Ala Pro Gly Lys Gly Leu
       50                  55                  60
   gag tgg ata tca gtt att gat act cgt ggt gcc aca tac tat gca gac      240
   Glu Trp Ile Ser Val Ile Asp Thr Arg Gly Ala Thr Tyr Tyr Ala Asp
   65                  70                  75                  80
   tcc gtg aag ggc cga ttc acc atc tcc aga gac aat gcc aag aac tcc      288
   Ser Val Lys Gly Arg Phe Thr Ile Ser Arg Asp Asn Ala Lys Asn Ser
                   85                  90                  95
   ttg tat ctt caa atg aac agc ctg aga gcc gag gac act gct gtg tat      336
   Leu Tyr Leu Gln Met Asn Ser Leu Arg Ala Glu Asp Thr Ala Val Tyr
               100                 105                 110
   tac tgt gca aga ctg ggg aac ttc tac tac ggt atg gac gtc tgg ggc      384
   Tyr Cys Ala Arg Leu Gly Asn Phe Tyr Tyr Gly Met Asp Val Trp Gly
           115                 120                 125
   caa ggg acc acg gtc acc gtc tcc tca                                  411
   Gln Gly Thr Thr Val Thr Val Ser Ser
       130                 135
<210> 12
   <211> 137
   <212> PRT
   <213> Artificial Sequence
<220>
   <223> Synthetic Construct
<400> 12
   Met Glu Phe Gly Leu Ser Trp Val Phe Leu Val Ala Ile Leu Lys Gly
   1 5 10 15
   Val Gln Cys Glu Val Gln Leu Val Gln Ser Gly Gly Gly Leu Val Gln
   20 25 30
   Pro Gly Gly Ser Leu Arg Leu Ser Cys Ala Ala Ser Gly Phe Thr Phe
   35 40 45
   Ser Ser Phe Ala Met His Trp Val Arg Gln Ala Pro Gly Lys Gly Leu
   50 55 60
   Glu Trp Ile Ser Val Ile Asp Thr Arg Gly Ala Thr Tyr Tyr Ala Asp
   65 70 75 80
   Ser Val Lys Gly Arg Phe Thr Ile Ser Arg Asp Asn Ala Lys Asn Ser
   85 90 95
   Leu Tyr Leu Gln Met Asn Ser Leu Arg Ala Glu Asp Thr Ala Val Tyr
   100 105 110
   Tyr Cys Ala Arg Leu Gly Asn Phe Tyr Tyr Gly Met Asp Val Trp Gly
   115 120 125
   Gln Gly Thr Thr Val Thr Val Ser Ser
   130 135
<210> 13
   <211> 174
   <212> PRT
   <213> Artificial Sequence
<220>
   <223> immunoglobulin heavy chain variable region
<400> 13
   Gly Arg Leu Gly Gln Ala Trp Arg Ser Leu Arg Leu Ser Cys Ala Ala
   1 5 10 15
   Ser Gly Phe Thr Phe Ser Asp Tyr Tyr Met Ser Trp Ile Arg Gln Ala
   20 25 30
   Pro Gly Lys Gly Leu Glu Trp Val Ser Tyr Ile Ser Ser Ser Gly Ser
           35                  40                  45
   Thr Arg Asp Tyr Ala Asp Ser Val Lys Gly Arg Phe Thr Ile Ser Arg
       50                  55                  60
   Asp Asn Ala Lys Asn Ser Leu Tyr Leu Gln Met Asn Ser Leu Arg Ala
   65                  70                  75                  80
   Glu Asp Thr Ala Val Tyr Tyr Cys Val Arg Asp Gly Val Glu Thr Thr
                   85                  90                  95
   Phe Tyr Tyr Tyr Tyr Tyr Gly Met Asp Val Trp Gly Gln Gly Thr Thr
               100                 105                 110
   Val Thr Val Ser Ser Ala Ser Thr Lys Gly Pro Ser Val Phe Pro Leu
           115                 120                 125
   Ala Pro Cys Ser Arg Ser Thr Ser Glu Ser Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ser Cys Ala
                   165                 170
<210> 14
   <211> 124
   <212> PRT
   <213> Artificial Sequence
<220>
   <223> immunoglobulin heavy chain variable region
<400> 14
   Val Gln Leu Leu Glu Ser Gly Gly Gly Leu Val Gln Pro Gly Gly Ser
   1               5                   10                  15
   Leu Arg Leu Ser Cys Thr Ala Ser Gly Phe Thr Phe Ser Ser Tyr Ala
               20                  25                  30
   Met Asn Trp Val Arg Gln Ala Pro Gly Lys Gly Leu Glu Trp Val Ser
           35                  40                  45
   Ala Ile Ser Gly Ser Gly Gly Thr Thr Phe Tyr Ala Asp Ser Val Lys
       50                  55                  60
   Gly Arg Phe Thr Ile Ser Arg Asp Asn Ser Arg Thr Thr Leu Tyr Leu
   65                  70                  75                  80
   Gln Met Asn Ser Leu Arg Ala Glu Asp Thr Ala Val Tyr Tyr Cys Ala
                   85                  90                  95
   Lys Asp Leu Gly Trp Ser Asp Ser Tyr Tyr Tyr Tyr Tyr Gly Met Asp
               100                 105                 110
   Val Trp Gly Gln Gly Thr Thr Val Thr Val Ser Ser
           115                 120
<210> 15
   <211> 112
   <212> PRT
   <213> Artificial Sequence
<220>
   <223> immunoglobulin heavy chain variable region
<400> 15
   Gly Pro Gly Leu Val Lys Pro Ser Glu Thr Leu Ser Leu Thr Cys Thr
   1               5                   10                  15
   Val Ser Gly Gly Ser Ile Ser Asn Tyr Tyr Trp Ser Trp Ile Arg Gln
               20                  25                  30
   Pro Ala Gly Lys Gly Leu Glu Trp Ile Gly Arg Ile Tyr Thr Ser Gly
           35                  40                  45
   Ser Pro Asn Tyr Asn Pro Ser Leu Lys Ser Arg Val Thr Met Ser Val
       50                  55                  60
   Asp Thr Ser Lys Asn Gln Phe Ser Leu Lys Leu Asn Ser Val Thr Ala
   65                  70                  75                  80
   Ala Asp Thr Ala Val Tyr Tyr Cys Ala Val Thr Ile Phe Gly Val Val
                   85                  90                  95
   Ile Ile Phe Asp Tyr Trp Gly Gln Gly Thr Leu Val Thr Val Ser Ser
               100                 105                 110
<210> 16
   <211> 125
   <212> PRT
   <213> Artificial Sequence
<220>
   <223> immunoglobulin heavy chain variable region
<400> 16
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ile Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asp Leu Gly Tyr Gly Asp Phe Tyr Tyr Tyr Tyr Tyr Gly Met
               100                 105                 110
   Asp Val Trp Gly Gln Gly Thr Thr Val Thr Val Ser Ser
           115                 120                 125
<210> 17
   <211> 113
   <212> PRT
   <213> Artificial Sequence
<220>
   <223> immunoglobulin heavy chain variable region
<400> 17
   Pro Gly Leu Val Lys Pro Ser Glu Thr Leu Ser Leu Thr Cys Thr Val
   1               5                   10                  15
   Ser Gly Gly Ser Ile Ser Ser Tyr Tyr Trp Ser Trp Ile Arg Gln Pro
               20                  25                  30
   Pro Gly Lys Gly Leu Glu Trp Ile Gly Tyr Ile Tyr Tyr Ser Gly Ser
   35 40 45
   Thr Asn Tyr Asn Pro Ser Leu Lys Ser Arg Val Thr Ile Ser Val Asp
   50 55 60
   Thr Ser Lys Asn Gln Phe Ser Leu Lys Leu Ser Ser Val Thr Ala Ala
   65 70 75 80
   Asp Thr Ala Val Tyr Tyr Cys Ala Arg Thr Tyr Ser Ser Ser Phe Tyr
   85 90 95
   Tyr Tyr Gly Met Asp Val Trp Gly Gln Gly Thr Thr Val Thr Val Ser
   100 105 110
   Ser
<210> 18
   <211> 122
   <212> PRT
   <213> Artificial Sequence
<220>
   <223> immunoglobulin heavy chain variable region
<400> 18
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Gly Ile Thr Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Asp Pro Gly Thr Thr Val Ile Met Ser Trp Phe Asp Pro Trp
   100 105 110
   Gly Gln Gly Thr Leu Val Thr Val Ser Ser
   115 120
<210> 19
   <211> 136
   <212> PRT
   <213> Artificial Sequence
<220>
   <223> immunoglobulin light chain variable region
<400> 19
   Ala Ser Val Gly Asp Arg Val Thr Phe Thr Cys Arg Ala Ser Gln Asp
   1               5                   10                  15
   Ile Arg Arg Asp Leu Gly Trp Tyr Gln Gln Lys Pro Gly Lys Ala Pro
               20                  25                  30
   Lys Arg Leu Ile Tyr Ala Ala Ser Arg Leu Gln Ser Gly Val Pro Ser
           35                  40                  45
   Arg Phe Ser Gly Ser Gly Ser Gly Thr Glu Phe Thr Leu Thr Ile Ser
       50                  55                  60
   Ser Leu Gln Pro Glu Asp Phe Ala Thr Tyr Tyr Cys Leu Gln His Asn
   65                  70                  75                  80
   Asn Tyr Pro Arg Thr Phe Gly Gln Gly Thr Glu Val Glu Ile Ile Arg
                   85                  90                  95
   Thr Val Ala Ala Pro Ser Val Phe Ile Phe Pro Pro Ser Asp Glu Gln
               100                 105                 110
   Leu Lys Ser Gly Thr Ala Ser Val Val Cys Leu Leu Asn Asn Phe Tyr
           115                 120                 125
   Pro Arg Glu Ala Lys Val Gln Trp
       130                 135
<210> 20
   <211> 107
   <212> PRT
   <213> Artificial Sequence
<220>
   <223> immunoglobulin light chain variable region
<400> 20
<210> 21
   <211> 100
   <212> PRT
   <213> Artificial Sequence
<220>
   <223> immunoglobulin light chain variable region
<400> 21
   Ser Ser Leu Ser Ala Ser Val Gly Asp Arg Val Thr Phe Thr Cys Arg
   1 5 10 15
   Ala Ser Gln Asp Ile Arg Arg Asp Leu Gly Trp Tyr Gln Gln Lys Pro
   20 25 30
   Gly Lys Ala Pro Lys Arg Leu Ile Tyr Ala Ala Ser Arg Leu Gln Ser
   35 40 45
   Gly Val Pro Ser Arg Phe Ser Gly Ser Gly Ser Gly Thr Glu Phe Thr
   50 55 60
   Leu Thr Ile Ser Ser Leu Gln Pro Glu Asp Phe Ala Thr Tyr Tyr Cys
   65 70 75 80
   Leu Gln His Asn Asn Tyr Pro Arg Thr Phe Gly Gln Gly Thr Glu Val
   85 90 95
   Glu Ile Ile Arg
   100
<210> 22
   <211> 107
   <212> PRT
   <213> Artificial Sequence
<220>
   <223> immunoglobulin light chain variable region
<400> 22
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Ser Asp
   20 25 30
   Leu Gly Trp Phe Gln Gln Lys Pro Gly Lys Ala Pro Lys Arg Leu Ile
   35 40 45
   Tyr Ala Ala Ser Lys Leu His Arg Gly Val Pro Ser Arg Phe Ser Gly
   50 55 60
   Ser Gly Ser Gly Thr Glu Phe Thr Leu Thr Ile Ser Arg Leu Gln Pro
   65 70 75 80
   Glu Asp Phe Ala Thr Tyr Tyr Cys Leu Gln His Asn Ser Tyr Pro Leu
   85 90 95
   Thr Phe Gly Gly Gly Thr Lys Val Glu Ile Lys
   100 105
<210> 23
   <211> 92
   <212> PRT
   <213> Artificial Sequence
<220>
   <223> immunoglobulin light chain variable region
<400> 23
   Gly Asp Arg Val Thr Ile Thr Cys Arg Ala Ser Gln Ser Ile Ser Thr
   1 5 10 15
   Phe Leu Asn Trp Tyr Gln Gln Lys Pro Gly Lys Ala Pro Lys Leu Leu
   20 25 30
   Ile His Val Ala Ser Ser Leu Gln Gly Gly Val Pro Ser Arg Phe Ser
   35 40 45
   Gly Ser Gly Ser Gly Thr Asp Phe Thr Leu Thr Ile Ser Ser Leu Gln
   50 55 60
   Pro Glu Asp Phe Ala Thr Tyr Tyr Cys Gln Gln Ser Tyr Asn Ala Pro
   65 70 75 80
   Leu Thr Phe Gly Gly Gly Thr Lys Val Glu Ile Lys
   85 90
<210> 24
   <211> 91
   <212> PRT
   <213> Artificial Sequence
<220>
   <223> immunoglobulin light chain variable region
<400> 24
   Arg Ala Thr Leu Ser Cys Arg Ala Ser Gln Ser Val Arg Gly Arg Tyr
   1 5 10 15
   Leu Ala Trp Tyr Gln Gln Lys Pro Gly Gln Ala Pro Arg Leu Leu Ile
   20 25 30
   Tyr Gly Ala Ser Ser Arg Ala Thr Gly Ile Pro Asp Arg Phe Ser Gly
   35 40 45
   Ser Gly Ser Gly Thr Asp Phe Thr Leu Thr Ile Ser Arg Leu Glu Pro
   50 55 60
   Glu Asp Phe Ala Val Phe Tyr Cys Gln Gln Tyr Gly Ser Ser Pro Arg
   65 70 75 80
   Thr Phe Gly Gln Gly Thr Lys Val Glu Ile Lys
   85 90
<210> 25
   <211> 236
   <212> PRT
   <213> Artificial Sequence
<220>
   <223> light chain immunoglobulin
<400> 25
   Met Asp Met Arg Val Pro Ala Gln Leu Leu Gly Leu Leu Leu Leu Trp
   1               5                   10                  15
   Phe Pro Gly Ala Arg Cys Asp Ile Gln Met Thr Gln Ser Pro Ser Ser
               20                  25                  30
   Leu Ser Ala Ser Val Gly Asp Arg Val Thr Ile Thr Cys Arg Ala Ser
           35                  40                  45
   Gln Gly Ile Arg Asn Asp Leu Gly Trp Tyr Gln Gln Lys Pro Gly Lys
       50                  55                  60
   Ala Pro Lys Arg Leu Ile Tyr Ala Ala Ser Ser Leu Gln Ser Gly Val
   65                  70                  75                  80
   Pro Ser Arg Phe Ser Gly Ser Gly Ser Gly Thr Glu Phe Thr Leu Thr
                   85                  90                  95
   Ile Ser Ser Leu Gln Pro Glu Asp Phe Ala Thr Tyr Tyr Cys Leu Gln
               100                 105                 110
   His Asn Ser Tyr Pro Trp Thr Phe Gly Gln Gly Thr Lys Val Glu Ile
           115                 120                 125
   Lys Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
               180                 185                 190
   Ser Thr Tyr Ser Leu Ser Ser Thr Leu Thr Leu Ser Lys Ala Asp Tyr
           195                 200                 205
   Glu Lys His Lys Val Tyr Ala Cys Glu Val Thr His Gln Gly Leu Ser
               210                 215                 220
   Ser Pro Val Thr Lys Ser Phe Asn Arg Gly Glu Cys
   225                 230                 235
<210> 26
   <211> 236
   <212> PRT
   <213> Artificial Sequence
<220>
   <223> light chain immunoglobulin
<400> 26
   Met Asp Met Arg Val Pro Ala Gln Leu Leu Gly Leu Leu Leu Leu Trp
   1               5                   10                  15
   Phe Pro Gly Ala Arg Cys Asp Ile Gln Met Thr Gln Ser Pro Ser Ser
               20                  25                  30
   Leu Ser Ala Ser Val Gly Asp Arg Val Thr Phe Thr Cys Arg Ala Ser
           35                  40                  45
   Gln Asp Ile Arg Arg Asp Leu Gly Trp Tyr Gln Gln Lys Pro Gly Lys
       50                  55                  60
   Ala Pro Lys Arg Leu Ile Tyr Ala Ala Ser Arg Leu Gln Ser Gly Val
   65                  70                  75                  80
   Pro Ser Arg Phe Ser Gly Ser Gly Ser Gly Thr Glu Phe Thr Leu Thr
                   85                  90                  95
   Ile Ser Ser Leu Gln Pro Glu Asp Phe Ala Thr Tyr Tyr Cys Leu Gln
               100                 105                 110
   His Asn Asn Tyr Pro Arg Thr Phe Gly Gln Gly Thr Glu Val Glu Ile
           115                 120                 125
   Ile Arg Thr Val Ala Ala Pro Ser Val Phe Ile Phe Pro Pro Ser Asp
       130                 135                 140
   Glu Gln Leu Lys Ser Gly Thr Ala Ser Val Val Cys Leu Leu Asn Asn
   145                 150                 155                 160
   Phe Tyr Pro Arg Glu Ala Lys Val Gln Trp Lys Val Asp Asn Ala Leu
                   165                 170                 175
   Gln Ser Gly Asn Ser Gln Glu Ser Val Thr Glu Gln Asp Ser Lys Asp
   180 185 190
   Ser Thr Tyr Ser Leu Ser Ser Thr Leu Thr Leu Ser Lys Ala Asp Tyr
   195 200 205
   Glu Lys His Lys Val Tyr Ala Cys Glu Val Thr His Gln Gly Leu Ser
   210 215 220
   Ser Pro Val Thr Lys Ser Phe Asn Arg Gly Glu Cys
   225 230 235
<210> 27
   <211> 236
   <212> PRT
   <213> Artificial Sequence
<220>
   <223> light chain immunoglobulin
<400> 27
   Met Asp Met Arg Val Pro Ala Gln Leu Leu Gly Leu Leu Leu Leu Trp
   1 5 10 15
   Phe Pro Gly Ala Arg Cys Asp Ile Gln Met Thr Gln Ser Pro Ser Ser
   20 25 30
   Leu Ser Ala Ser Val Gly Asp Arg Val Thr Ile Thr Cys Arg Ala Ser
   35 40 45
   Gln Gly Ile Arg Asn Asp Leu Gly Trp Tyr Gln Gln Lys Pro Gly Lys
   50 55 60
   Ala Pro Lys Arg Leu Ile Tyr Ala Ala Ser Ser Leu Gln Ser Gly Val
   65 70 75 80
   Pro Ser Arg Phe Ser Gly Ser Gly Ser Gly Thr Glu Phe Thr Leu Thr
   85 90 95
   Ile Ser Ser Leu Gln Pro Glu Asp Phe Ala Thr Tyr Tyr Cys Leu Gln
   100 105 110
   His Asn Ser Tyr Pro Tyr Thr Phe Gly Gln Gly Thr Lys Leu Glu Ile
   115 120 125
   Lys Arg Thr Val Ala Ala Pro Ser Val Phe Ile Phe Pro Pro Ser Asp
   130 135 140
   Glu Gln Leu Lys Ser Gly Thr Ala Ser Val Val Cys Leu Leu Asn Asn
   145 150 155 160
   Phe Tyr Pro Arg Glu Ala Lys Val Gln Trp Lys Val Asp Asn Ala Leu
   165 170 175
   Gln Ser Gly Asn Ser Gln Glu Ser Val Thr Glu Gln Asp Ser Lys Asp
   180 185 190
   Ser Thr Tyr Ser Leu Ser Ser Thr Leu Thr Leu Ser Lys Ala Asp Tyr
   195 200 205
   Glu Lys His Lys Val Tyr Ala Cys Glu Val Thr His Gln Gly Leu Ser
   210 215 220
   Ser Pro Val Thr Lys Ser Phe Asn Arg Gly Glu Cys
   225 230 235
<210> 28
   <211> 236
   <212> PRT
   <213> Artificial Sequence
<220>
   <223> light chain immunoglobulin
<400> 28
   Met Asp Met Arg Val Pro Ala Gln Leu Leu Gly Leu Leu Leu Leu Trp
   1 5 10 15
   Phe Pro Gly Ala Arg Cys Asp Ile Gln Met Thr Gln Phe Pro Ser Ser
   20 25 30
   Leu Ser Ala Ser Val Gly Asp Arg Val Thr Ile Thr Cys Arg Ala Ser
   35 40 45
   Gln Gly Ile Arg Asn Asp Leu Gly Trp Tyr Gln Gln Lys Pro Gly Lys
   50 55 60
   Ala Pro Lys Arg Leu Ile Tyr Ala Ala Ser Arg Leu His Arg Gly Val
   65 70 75 80
   Pro Ser Arg Phe Ser Gly Ser Gly Ser Gly Thr Glu Phe Thr Leu Thr
   85 90 95
   Ile Ser Ser Leu Gln Pro Glu Asp Phe Ala Thr Tyr Tyr Cys Leu Gln
   100 105 110
   His Asn Ser Tyr Pro Cys Ser Phe Gly Gln Gly Thr Lys Leu Glu Ile
   115 120 125
   Lys Arg Thr Val Ala Ala Pro Ser Val Phe Ile Phe Pro Pro Ser Asp
   130 135 140
   Glu Gln Leu Lys Ser Gly Thr Ala Ser Val Val Cys Leu Leu Asn Asn
   145 150 155 160
   Phe Tyr Pro Arg Glu Ala Lys Val Gln Trp Lys Val Asp Asn Ala Leu
   165 170 175
   Gln Ser Gly Asn Ser Gln Glu Ser Val Thr Glu Gln Asp Ser Lys Asp
   180 185 190
   Ser Thr Tyr Ser Leu Ser Ser Thr Leu Thr Leu Ser Lys Ala Asp Tyr
   195 200 205
   Glu Lys His Lys Val Tyr Ala Cys Glu Val Thr His Gln Gly Leu Ser
   210 215 220
   Ser Pro Val Thr Lys Ser Phe Asn Arg Gly Glu Cys
   225 230 235
<210> 29
   <211> 473
   <212> PRT
   <213> Artificial Sequence
<220>
   <223> heavy chain immunoglobulin
<400> 29
   Met Glu Phe Gly Leu Ser Trp Val Phe Leu Val Ala Ile Ile Lys Gly
   1 5 10 15
   Val Gln Cys Gln Val Gln Leu Val Glu Ser Gly Gly Gly Leu Val Lys
   20 25 30
   Pro Gly Gly Ser Leu Arg Leu Ser Cys Ala Ala Ser Gly Phe Thr Phe
   35 40 45
   Ser Asp Tyr Tyr Met Ser Trp Ile Arg Gln Ala Pro Gly Lys Gly Leu
   50 55 60
   Glu Trp Val Ser Tyr Ile Ser Ser Ser Gly Ser Thr Ile Tyr Tyr Ala
   65 70 75 80
   Asp Ser Val Lys Gly Arg Phe Thr Ile Ser Arg Asp Asn Ala Lys Asn
   85 90 95
   Ser Leu Tyr Leu Gln Met Asn Ser Leu Arg Ala Glu Asp Thr Ala Val
   100 105 110
   Tyr Tyr Cys Ala Arg Val Leu Arg Phe Leu Glu Trp Leu Leu Tyr Tyr
   115 120 125
   Tyr Tyr Tyr Tyr Gly Met Asp Val Trp Gly Gln Gly Thr Thr Val Thr
   130 135 140
   Val Ser Ser Ala Ser Thr Lys Gly Pro Ser Val Phe Pro Leu Ala Pro
   145 150 155 160
   Cys Ser Arg Ser Thr Ser Glu Ser Thr Ala Ala Leu Gly Cys Leu Val
   165 170 175
   Lys Asp Tyr Phe Pro Glu Pro Val Thr Val Ser Trp Asn Ser Gly Ala
   180 185 190
   Leu Thr Ser Gly Val His Thr Phe Pro Ala Val Leu Gln Ser Ser Gly
   195 200 205
   Leu Tyr Ser Leu Ser Ser Val Val Thr Val Pro Ser Ser Asn Phe Gly
   210 215 220
   Thr Gln Thr Tyr Thr Cys Asn Val Asp His Lys Pro Ser Asn Thr Lys
   225 230 235 240
   Val Asp Lys Thr Val Glu Arg Lys Cys Cys Val Glu Cys Pro Pro Cys
   245 250 255
   Pro Ala Pro Pro Val Ala Gly Pro Ser Val Phe Leu Phe Pro Pro Lys
   260 265 270
   Pro Lys Asp Thr Leu Met Ile Ser Arg Thr Pro Glu Val Thr Cys Val
   275 280 285
   Val Val Asp Val Ser His Glu Asp Pro Glu Val Gln Phe Asn Trp Tyr
   290 295 300
   Val Asp Gly Val Glu Val His Asn Ala Lys Thr Lys Pro Arg Glu Glu
   305 310 315 320
   Gln Phe Asn Ser Thr Phe Arg Val Val Ser Val Leu Thr Val Val His
   325 330 335
   Gln Asp Trp Leu Asn Gly Lys Glu Tyr Lys Cys Lys Val Ser Asn Lys
   340 345 350
   Gly Leu Pro Ala Pro Ile Glu Lys Thr Ile Ser Lys Thr Lys Gly Gln
   355 360 365
   Pro Arg Glu Pro Gln Val Tyr Thr Leu Pro Pro Ser Arg Glu Glu Met
   370 375 380
   Thr Lys Asn Gln Val Ser Leu Thr Cys Leu Val Lys Gly Phe Tyr Pro
   385 390 395 400
   Ser Asp Ile Ala Val Glu Trp Glu Ser Asn Gly Gln Pro Glu Asn Asn
   405 410 415
   Tyr Lys Thr Thr Pro Pro Met Leu Asp Ser Asp Gly Ser Phe Phe Leu
   420 425 430
   Tyr Ser Lys Leu Thr Val Asp Lys Ser Arg Trp Gln Gln Gly Asn Val
   435 440 445
   Phe Ser Cys Ser Val Met His Glu Ala Leu His Asn His Tyr Thr Gln
   450 455 460
   Lys Ser Leu Ser Leu Ser Pro Gly Lys
   465 470
<210> 30
   <211> 470
   <212> PRT
   <213> Artificial Sequence
<220>
   <223> heavy chain immunoglobulin
<400> 30
   Met Glu Phe Gly Leu Ser Trp Val Phe Leu Val Ala Ile Ile Lys Gly
   1               5                   10                  15
   Val Gln Cys Gln Ala Gln Leu Val Glu Ser Gly Gly Gly Leu Val Lys
               20                  25                  30
   Pro Gly Gly Ser Leu Arg Leu Ser Cys Ala Ala Ser Gly Phe Thr Phe
           35                  40                  45
   Ser Asp Tyr Tyr Met Ser Trp Ile Arg Gln Ala Pro Gly Lys Gly Leu
       50                  55                  60
   Glu Trp Val Ser Tyr Ile Ser Ser Ser Gly Ser Thr Arg Asp Tyr Ala
   65                  70                  75                  80
   Asp Ser Val Lys Gly Arg Phe Thr Ile Ser Arg Asp Asn Ala Lys Asn
                   85                  90                  95
   Ser Leu Tyr Leu Gln Met Asn Ser Leu Arg Ala Glu Asp Thr Ala Val
               100                 105                 110
   Tyr Tyr Cys Val Arg Asp Gly Val Glu Thr Thr Phe Tyr Tyr Tyr Tyr
           115                 120                 125
   Tyr Gly Met Asp Val Trp Gly Gln Gly Thr Thr Val Thr Val Ser Ser
       130                 135                 140
   Ala Ser Thr Lys Gly Pro Ser Val Phe Pro Leu Ala Pro Cys Ser Arg
   145                 150                 155                 160
   Ser Thr Ser Glu Ser Thr Ala Ala Leu Gly Cys Leu Val Lys Asp Tyr
                   165                 170                 175
   Phe Pro Glu Pro Val Thr Val Ser Trp Asn Ser Gly Ala Leu Thr Ser
               180                 185                 190
   Gly Val His Thr Phe Pro Ala Val Leu Gln Ser Ser Gly Leu Tyr Ser
           195                 200                 205
   Leu Ser Ser Val Val Thr Val Pro Ser Ser Asn Phe Gly Thr Gln Thr
       210                 215                 220
   Tyr Thr Cys Asn Val Asp His Lys Pro Ser Asn Thr Lys Val Asp Lys
   225                 230                 235                 240
   Thr Val Glu Arg Lys Cys Cys Val Glu Cys Pro Pro Cys Pro Ala Pro
   245 250 255
   Pro Val Ala Gly Pro Ser Val Phe Leu Phe Pro Pro Lys Pro Lys Asp
   260 265 270
   Thr Leu Met Ile Ser Arg Thr Pro Glu Val Thr Cys Val Val Val Asp
   275 280 285
   Val Ser His Glu Asp Pro Glu Val Gln Phe Asn Trp Tyr Val Asp Gly
   290 295 300
   Val Glu Val His Asn Ala Lys Thr Lys Pro Arg Glu Glu Gln Phe Asn
   305 310 315 320
   Ser Thr Phe Arg Val Val Ser Val Leu Thr Val Val His Gln Asp Trp
   325 330 335
   Leu Asn Gly Lys Glu Tyr Lys Cys Lys Val Ser Asn Lys Gly Leu Pro
   340 345 350
   Ala Pro Ile Glu Lys Thr Ile Ser Lys Thr Lys Gly Gln Pro Arg Glu
   355 360 365
   Pro Gln Val Tyr Thr Leu Pro Pro Ser Arg Glu Glu Met Thr Lys Asn
   370 375 380
   Gln Val Ser Leu Thr Cys Leu Val Lys Gly Phe Tyr Pro Ser Asp Ile
   385 390 395 400
   Ala Val Glu Trp Glu Ser Asn Gly Gln Pro Glu Asn Asn Tyr Lys Thr
   405 410 415
   Thr Pro Pro Met Leu Asp Ser Asp Gly Ser Phe Phe Leu Tyr Ser Lys
   420 425 430
   Leu Thr Val Asp Lys Ser Arg Trp Gln Gln Gly Asn Val Phe Ser Cys
   435 440 445
   Ser Val Met His Glu Ala Leu His Asn His Tyr Thr Gln Lys Ser Leu
   450 455 460
   Ser Leu Ser Pro Gly Lys
   465 470
<210> 31
   <211> 470
   <212> PRT
   <213> Artificial Sequence
<220>
   <223> heavy chain immunoglobulin
<400> 31
   Met Glu Phe Gly Leu Ser Trp Leu Phe Leu Val Ala Ile Leu Lys Gly
   1               5                   10                  15
   Val Gln Cys Glu Val Gln Leu Leu Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Ser Ser Tyr Ala Met Ser Trp Val Arg Gln Ala Pro Gly Lys Gly Leu
       50                  55                  60
   Glu Trp Val Ser Ala Ile Ser Gly Ser Gly Gly Ser Thr Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Lys Gly Tyr Ser Ser Gly Trp Tyr Tyr Tyr Tyr Tyr
           115                 120                 125
   Tyr Gly Met Asp Val Trp Gly Gln Gly Thr Thr Val Thr Val Ser Ser
       130                 135                 140
   Ala Ser Thr Lys Gly Pro Ser Val Phe Pro Leu Ala Pro Cys Ser Arg
   145                 150                 155                 160
   Ser Thr Ser Glu Ser Thr Ala Ala Leu Gly Cys Leu Val Lys Asp Tyr
                   165                 170                 175
   Phe Pro Glu Pro Val Thr Val Ser Trp Asn Ser Gly Ala Leu Thr Ser
               180                 185                 190
   Gly Val His Thr Phe Pro Ala Val Leu Gln Ser Ser Gly Leu Tyr Ser
           195                 200                 205
   Leu Ser Ser Val Val Thr Val Pro Ser Ser Asn Phe Gly Thr Gln Thr
       210                 215                 220
   Tyr Thr Cys Asn Val Asp His Lys Pro Ser Asn Thr Lys Val Asp Lys
   225                 230                 235                 240
   Thr Val Glu Arg Lys Cys Cys Val Glu Cys Pro Pro Cys Pro Ala Pro
                   245                 250                 255
   Pro Val Ala Gly Pro Ser Val Phe Leu Phe Pro Pro Lys Pro Lys Asp
               260                 265                 270
   Thr Leu Met Ile Ser Arg Thr Pro Glu Val Thr Cys Val Val Val Asp
           275                 280                 285
   Val Ser His Glu Asp Pro Glu Val Gln Phe Asn Trp Tyr Val Asp Gly
       290                 295                 300
   Val Glu Val His Asn Ala Lys Thr Lys Pro Arg Glu Glu Gln Phe Asn
   305                 310                 315                 320
   Ser Thr Phe Arg Val Val Ser Val Leu Thr Val Val His Gln Asp Trp
                   325                 330                 335
   Leu Asn Gly Lys Glu Tyr Lys Cys Lys Val Ser Asn Lys Gly Leu Pro
               340                 345                 350
   Ala Pro Ile Glu Lys Thr Ile Ser Lys Thr Lys Gly Gln Pro Arg Glu
           355                 360                 365
   Pro Gln Val Tyr Thr Leu Pro Pro Ser Arg Glu Glu Met Thr Lys Asn
       370                 375                 380
   Gln Val Ser Leu Thr Cys Leu Val Lys Gly Phe Tyr Pro Ser Asp Ile
   385                 390                 395                 400
   Ala Val Glu Trp Glu Ser Asn Gly Gln Pro Glu Asn Asn Tyr Lys Thr
                   405                 410                 415
   Thr Pro Pro Met Leu Asp Ser Asp Gly Ser Phe Phe Leu Tyr Ser Lys
               420                 425                 430
   Leu Thr Val Asp Lys Ser Arg Trp Gln Gln Gly Asn Val Phe Ser Cys
           435                 440                 445
   Ser Val Met His Glu Ala Leu His Asn His Tyr Thr Gln Lys Ser Leu
       450                 455                 460
   Ser Leu Ser Pro Gly Lys
   465                 470
<210> 32
   <211> 470
   <212> PRT
   <213> Artificial Sequence
<220>
   <223> heavy chain immunoglobulin
<400> 32
   Met Glu Phe Gly Leu Ser Trp Leu Phe Leu Val Ala Ile Leu Lys Gly
   1               5                   10                  15
   Val Gln Cys Glu Val Gln Leu Leu Glu Ser Gly Gly Gly Leu Val Gln
               20                  25                  30
   Pro Gly Gly Ser Leu Arg Leu Ser Cys Thr Ala Ser Gly Phe Thr Phe
           35                  40                  45
   Ser Ser Tyr Ala Met Asn Trp Val Arg Gln Ala Pro Gly Lys Gly Leu
       50                  55                  60
   Glu Trp Val Ser Ala Ile Ser Gly Ser Gly Gly Thr Thr Phe Tyr Ala
   65                  70                  75                  80
   Asp Ser Val Lys Gly Arg Phe Thr Ile Ser Arg Asp Asn Ser Arg Thr
                   85                  90                  95
   Thr Leu Tyr Leu Gln Met Asn Ser Leu Arg Ala Glu Asp Thr Ala Val
               100                 105                 110
   Tyr Tyr Cys Ala Lys Asp Leu Gly Trp Ser Asp Ser Tyr Tyr Tyr Tyr
           115                 120                 125
   Tyr Gly Met Asp Val Trp Gly Gln Gly Thr Thr Val Thr Val Ser Ser
       130                 135                 140
   Ala Ser Thr Lys Gly Pro Ser Val Phe Pro Leu Ala Pro Cys Ser Arg
   145                 150                 155                 160
   Ser Thr Ser Glu Ser Thr Ala Ala Leu Gly Cys Leu Val Lys Asp Tyr
                   165                 170                 175
   Phe Pro Glu Pro Val Thr Val Ser Trp Asn Ser Gly Ala Leu Thr Ser
               180                 185                 190
   Gly Val His Thr Phe Pro Ala Val Leu Gln Ser Ser Gly Leu Tyr Ser
           195                 200                 205
   Leu Ser Ser Val Val Thr Val Pro Ser Ser Asn Phe Gly Thr Gln Thr
       210                 215                 220
   Tyr Thr Cys Asn Val Asp His Lys Pro Ser Asn Thr Lys Val Asp Lys
   225                 230                 235                 240
   Thr Val Glu Arg Lys Cys Cys Val Glu Cys Pro Pro Cys Pro Ala Pro
                   245                 250                 255
   Pro Val Ala Gly Pro Ser Val Phe Leu Phe Pro Pro Lys Pro Lys Asp
               260                 265                 270
   Thr Leu Met Ile Ser Arg Thr Pro Glu Val Thr Cys Val Val Val Asp
           275                 280                 285
   Val Ser His Glu Asp Pro Glu Val Gln Phe Asn Trp Tyr Val Asp Gly
       290                 295                 300
   Val Glu Val His Asn Ala Lys Thr Lys Pro Arg Glu Glu Gln Phe Asn
   305                 310                 315                 320
   Ser Thr Phe Arg Val Val Ser Val Leu Thr Val Val His Gln Asp Trp
                   325                 330                 335
   Leu Asn Gly Lys Glu Tyr Lys Cys Lys Val Ser Asn Lys Gly Leu Pro
               340                 345                 350
   Ala Pro Ile Glu Lys Thr Ile Ser Lys Thr Lys Gly Gln Pro Arg Glu
           355                 360                 365
   Pro Gln Val Tyr Thr Leu Pro Pro Ser Arg Glu Glu Met Thr Lys Asn
       370                 375                 380
   Gln Val Ser Leu Thr Cys Leu Val Lys Gly Phe Tyr Pro Ser Asp Ile
   385 390 395 400
   Ala Val Glu Trp Glu Ser Asn Gly Gln Pro Glu Asn Asn Tyr Lys Thr
   405 410 415
   Thr Pro Pro Met Leu Asp Ser Asp Gly Ser Phe Phe Leu Tyr Ser Lys
   420 425 430
   Leu Thr Val Asp Lys Ser Arg Trp Gln Gln Gly Asn Val Phe Ser Cys
   435 440 445
   Ser Val Met His Glu Ala Leu His Asn His Tyr Thr Gln Lys Ser Leu
   450 455 460
   Ser Leu Ser Pro Gly Lys
   465 470
<210> 33
   <211> 470
   <212> PRT
   <213> Artificial Sequence
<220>
   <223> immunoglobulin heavy chain of 2.12.1 fx
<400> 33
   Met Glu Phe Gly Leu Ser Trp Val Phe Leu Val Ala Ile Ile Lys Gly
   1 5 10 15
   Val Gln Cys Gln Val Gln Leu Val Glu Ser Gly Gly Gly Leu Val Lys
   20 25 30
   Pro Gly Gly Ser Leu Arg Leu Ser Cys Ala Ala Ser Gly Phe Thr Phe
   35 40 45
   Ser Asp Tyr Tyr Met Ser Trp Ile Arg Gln Ala Pro Gly Lys Gly Leu
   50 55 60
   Glu Trp Val Ser Tyr Ile Ser Ser Ser Gly Ser Thr Arg Asp Tyr Ala
   65 70 75 80
   Asp Ser Val Lys Gly Arg Phe Thr Ile Ser Arg Asp Asn Ala Lys Asn
   85 90 95
   Ser Leu Tyr Leu Gln Met Asn Ser Leu Arg Ala Glu Asp Thr Ala Val
   100 105 110
   Tyr Tyr Cys Ala Arg Asp Gly Val Glu Thr Thr Phe Tyr Tyr Tyr Tyr
   115 120 125
   Tyr Gly Met Asp Val Trp Gly Gln Gly Thr Thr Val Thr Val Ser Ser
   130 135 140
   Ala Ser Thr Lys Gly Pro Ser Val Phe Pro Leu Ala Pro Cys Ser Arg
   145 150 155 160
   Ser Thr Ser Glu Ser Thr Ala Ala Leu Gly Cys Leu Val Lys Asp Tyr
   165 170 175
   Phe Pro Glu Pro Val Thr Val Ser Trp Asn Ser Gly Ala Leu Thr Ser
   180 185 190
   Gly Val His Thr Phe Pro Ala Val Leu Gln Ser Ser Gly Leu Tyr Ser
   195 200 205
   Leu Ser Ser Val Val Thr Val Pro Ser Ser Asn Phe Gly Thr Gln Thr
   210 215 220
   Tyr Thr Cys Asn Val Asp His Lys Pro Ser Asn Thr Lys Val Asp Lys
   225 230 235 240
   Thr Val Glu Arg Lys Cys Cys Val Glu Cys Pro Pro Cys Pro Ala Pro
   245 250 255
   Pro Val Ala Gly Pro Ser Val Phe Leu Phe Pro Pro Lys Pro Lys Asp
   260 265 270
   Thr Leu Met Ile Ser Arg Thr Pro Glu Val Thr Cys Val Val Val Asp
   275 280 285
   Val Ser His Glu Asp Pro Glu Val Gln Phe Asn Trp Tyr Val Asp Gly
   290 295 300
   Val Glu Val His Asn Ala Lys Thr Lys Pro Arg Glu Glu Gln Phe Asn
   305 310 315 320
   Ser Thr Phe Arg Val Val Ser Val Leu Thr Val Val His Gln Asp Trp
   325 330 335
   Leu Asn Gly Lys Glu Tyr Lys Cys Lys Val Ser Asn Lys Gly Leu Pro
   340 345 350
   Ala Pro Ile Glu Lys Thr Ile Ser Lys Thr Lys Gly Gln Pro Arg Glu
   355 360 365
   Pro Gln Val Tyr Thr Leu Pro Pro Ser Arg Glu Glu Met Thr Lys Asn
   370 375 380
   Gln Val Ser Leu Thr Cys Leu Val Lys Gly Phe Tyr Pro Ser Asp Ile
   385 390 395 400
   Ala Val Glu Trp Glu Ser Asn Gly Gln Pro Glu Asn Asn Tyr Lys Thr
   405 410 415
   Thr Pro Pro Met Leu Asp Ser Asp Gly Ser Phe Phe Leu Tyr Ser Lys
   420 425 430
   Leu Thr Val Asp Lys Ser Arg Trp Gln Gln Gly Asn Val Phe Ser Cys
   435 440 445
   Ser Val Met His Glu Ala Leu His Asn His Tyr Thr Gln Lys Ser Leu
   450 455 460
   Ser Leu Ser Pro Gly Lys
   465 470
<210> 34
   <211> 125
   <212> PRT
   <213> Artificial Sequence
<220>
   <223> mature immunoglobulin heavy chain variable region of 2.12.1 fx
<400> 34
   Gln Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Asp Tyr
   20 25 30
   Tyr Met Ser Trp Ile Arg Gln Ala Pro Gly Lys Gly Leu Glu Trp Val
   35 40 45
   Ser Tyr Ile Ser Ser Ser Gly Ser Thr Arg Asp Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Asp Gly Val Glu Thr Thr Phe Tyr Tyr Tyr Tyr Tyr Gly Met
   100 105 110
   Asp Val Trp Gly Gln Gly Thr Thr Val Thr Val Ser Ser
   115 120 125
<210> 35
   <211> 236
   <212> PRT
   <213> Artificial Sequence
<220>
   <223> immunoglobulin light chain of 2.12.1 fx
<400> 35
   Met Asp Met Arg Val Pro Ala Gln Leu Leu Gly Leu Leu Leu Leu Trp
   1 5 10 15
   Phe Pro Gly Ala Arg Cys Asp Ile Gln Met Thr Gln Ser Pro Ser Ser
   20 25 30
   Leu Ser Ala Ser Val Gly Asp Arg Val Thr Ile Thr Cys Arg Ala Ser
   35 40 45
   Gln Asp Ile Arg Arg Asp Leu Gly Trp Tyr Gln Gln Lys Pro Gly Lys
   50 55 60
   Ala Pro Lys Arg Leu Ile Tyr Ala Ala Ser Arg Leu Gln Ser Gly Val
   65 70 75 80
   Pro Ser Arg Phe Ser Gly Ser Gly Ser Gly Thr Glu Phe Thr Leu Thr
   85 90 95
   Ile Ser Ser Leu Gln Pro Glu Asp Phe Ala Thr Tyr Tyr Cys Leu Gln
   100 105 110
   His Asn Asn Tyr Pro Arg Thr Phe Gly Gln Gly Thr Lys Val Glu Ile
   115 120 125
   Lys Arg Thr Val Ala Ala Pro Ser Val Phe Ile Phe Pro Pro Ser Asp
   130 135 140
   Glu Gln Leu Lys Ser Gly Thr Ala Ser Val Val Cys Leu Leu Asn Asn
   145 150 155 160
   Phe Tyr Pro Arg Glu Ala Lys Val Gln Trp Lys Val Asp Asn Ala Leu
   165 170 175
   Gln Ser Gly Asn Ser Gln Glu Ser Val Thr Glu Gln Asp Ser Lys Asp
   180 185 190
   Ser Thr Tyr Ser Leu Ser Ser Thr Leu Thr Leu Ser Lys Ala Asp Tyr
   195 200 205
   Glu Lys His Lys Val Tyr Ala Cys Glu Val Thr His Gln Gly Leu Ser
   210 215 220
   Ser Pro Val Thr Lys Ser Phe Asn Arg Gly Glu Cys
   225 230 235
<210> 36
   <211> 108
   <212> PRT
   <213> Artificial Sequence
<220>
   <223> mature immunoglobulin light chain variable region of 2.12.1 fx
<400> 36
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Arg Arg Asp
   20 25 30
   Leu Gly Trp Tyr Gln Gln Lys Pro Gly Lys Ala Pro Lys Arg Leu Ile
   35 40 45
   Tyr Ala Ala Ser Arg Leu Gln Ser Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Glu Asp Phe Ala Thr Tyr Tyr Cys Leu Gln His Asn Asn Tyr Pro Arg
   85 90 95
   Thr Phe Gly Gln Gly Thr Lys Val Glu Ile Lys Arg
   100 105
<210> 37
   <211> 112
   <212> PRT
   <213> Artificial Sequence
<220>
   <223> humanized 7C10 immunoglobulin light chain variable region; version 1
<400> 37
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Gln Trp Tyr Leu Gln Lys Pro Gly Gln Ser
           35                  40                  45
   Pro Gln Leu Leu Ile Tyr Lys Val Ser Asn Arg Leu Tyr Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Trp Thr Phe Gly Gln Gly Thr Lys Val Glu Ile Lys
               100                 105                 110
<210> 38
   <211> 112
   <212> PRT
   <213> Artificial Sequence
<220>
   <223> humanized 7C10 immunoglobulin light chain variable region; version 2
<400> 38
<210> 39
   <211> 117
   <212> PRT
   <213> Artificial Sequence
<220>
   <223> humanized 7C10 immunoglobulin heavy chain variable region; version 1
<400> 39
   Gln Val Gln Leu Gln Glu Ser Gly Pro Gly Leu Val Lys Pro Ser Glu
   1 5 10 15
   Thr Leu Ser Leu Thr Cys Thr Val Ser Gly Tyr Ser Ile Thr Gly Gly
   20 25 30
   Tyr Leu Trp Asn Trp Ile Arg Gln Pro Pro Gly Lys Gly Leu Glu Trp
   35 40 45
   Met Gly Tyr Ile Ser Tyr Asp Gly Thr Asn Asn Tyr Lys Pro Ser Leu
   50 55 60
   Lys Asp Arg Ile Thr Ile Ser Arg Asp Thr Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Arg Tyr Gly Arg Val Phe Phe Asp Tyr Trp Gly Gln Gly Thr Leu
   100 105 110
   Val Thr Val Ser Ser
   115
<210> 40
   <211> 117
   <212> PRT
   <213> Artificial Sequence
<220>
   <223> humanized 7C10 immunoglobulin heavy chain variable region; version 2
<400> 40
<210> 41
   <211> 117
   <212> PRT
   <213> Artificial Sequence
<220>
   <223> humanized 7C10 immunoglobulin heavy chain variable region; version 3
<400> 41
   Gln Val Gln Leu Gln Glu Ser Gly Pro Gly Leu Val Lys Pro Ser Glu
   1 5 10 15
   Thr Leu Ser Leu Thr Cys Thr Val Ser Gly Tyr Ser Ile Ser Gly Gly
   20 25 30
   Tyr Leu Trp Asn Trp Ile Arg Gln Pro Pro Gly Lys Gly Leu Glu Trp
   35 40 45
   Ile Gly Tyr Ile Ser Tyr Asp Gly Thr Asn Asn Tyr Lys Pro Ser Leu
   50 55 60
   Lys Asp Arg Val Thr Ile Ser Val Asp Thr Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Arg Tyr Gly Arg Val Phe Phe Asp Tyr Trp Gly Gln Gly Thr Leu
   100 105 110
   Val Thr Val Ser Ser
   115
<210> 42
   <211> 130
   <212> PRT
   <213> Artificial Sequence
<220>
   <223> A12 immunoglobulin heavy chain variable region
<400> 42
   Glu Val Gln Leu Val Gln Ser Gly Ala Glu Val Lys Lys Pro Gly Ser
   1 5 10 15
   Ser Val Lys Val Ser Cys Lys Ala Ser Gly Gly Thr Phe Ser Ser Tyr
   20 25 30
   Ala Ile Ser Trp Val Arg Gln Ala Pro Gly Gln Gly Leu Glu Trp Met
   35 40 45
   Gly Gly Ile Ile Pro Ile Phe Gly Thr Ala Asn Tyr Ala Gln Lys Phe
   50 55 60
   Gln Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Ala Pro Leu Arg Phe Leu Glu Trp Ser Thr Gln Asp His Tyr
   100 105 110
   Tyr Tyr Tyr Tyr Met Asp Val Trp Gly Lys Gly Thr Thr Val Thr Val
   115 120 125
   Ser Ser
   130
<210> 43
   <211> 109
   <212> PRT
   <213> Artificial Sequence
<220>
   <223> A12 immunoglobulin light chain variable region
<400> 43
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Asn Ser Asp Asn Arg
   85 90 95
   Leu Ile Phe Gly Gly Gly Thr Lys Leu Thr Val Leu Ser
   100 105
<210> 44
   <211> 119
   <212> PRT
   <213> Artificial Sequence
<220>
   <223> 1A immunoglobulin heavy chain variable region
<220>
   <221> MISC_FEATURE
   <222> (1)..(119)
   <223> Possible mutations: R30, S30, N31, S31, Y94, H94, D104, E104.
<400> 44
   Glu Val Gln Leu Val Gln Ser Gly Gly Gly Leu Val His Pro Gly Gly
   1               5                   10                  15
   Ser Leu Arg Leu Ser Cys Ala Gly Ser Gly Phe Thr Phe Arg Asn Tyr
               20                  25                  30
   Ala Met Tyr Trp Val Arg Gln Ala Pro Gly Lys Gly Leu Glu Trp Val
           35                  40                  45
   Ser Ala Ile Gly Ser Gly Gly Gly Thr Tyr Tyr Ala Asp Ser Val Lys
       50                  55                  60
   Gly Arg Phe Thr Ile Ser Arg Asp Asn Ala Lys Asn Ser Leu Tyr Leu
   65                  70                  75                  80
   Gln Met Asn Ser Leu Arg Ala Glu Asp Met Ala Val Tyr Tyr Cys Ala
                   85                  90                  95
   Arg Ala Pro Asn Trp Gly Ser Asp Ala Phe Asp Ile Trp Gly Gln Gly
               100                 105                 110
   Thr Met Val Thr Val Ser Ser
           115
<210> 45
   <211> 107
   <212> PRT
   <213> Artificial Sequence
<220>
   <223> 1A immunoglobulin light chain variable region
<220>
   <221> MISC_FEATURE
   <222> (1).. (107)
   <223> possible mutations: P96, I96, P100, Q100, R103, K103, V104, L104, D105, E105
<400> 45
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Ser Trp
   20 25 30
   Leu Ala Trp Tyr Gln Gln Lys Pro Glu Lys Ala Pro Lys Ser Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Asn Ser Tyr Pro Pro
   85 90 95
   Thr Phe Gly Pro Gly Thr Lys Val Asp Ile Lys
   100 105
<210> 46
   <211> 251
   <212> PRT
   <213> Artificial Sequence
<220>
   <223> single chain fv 8A1
<400> 46
   Glu Val Gln Leu Val Gln Ser Gly Ala Glu Val Lys Lys Pro Gly Glu
   1 5 10 15
   Ser Leu Thr Ile Ser Cys Lys Gly Pro Gly Tyr Asn Phe Phe Asn Tyr
   20 25 30
   Trp Ile Gly Trp Val Arg Gln Met Pro Gly Lys Gly Leu Glu Trp Met
   35 40 45
   Gly Ile Ile Tyr Pro Thr Asp Ser Asp Thr Arg Tyr Ser Pro Ser Phe
   50 55 60
   Gln Gly Gln Val Thr Ile Ser Val Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Ser Ile Arg Tyr Cys Pro Gly Gly Arg Cys Tyr Ser Gly Tyr
               100                 105                 110
   Tyr Gly Met Asp Val Trp Gly Gln Gly Thr Met Val Thr Val Ser Ser
           115                 120                 125
   Gly Gly Gly Gly Ser Gly Gly Gly Gly Ser Gly Gly Gly Gly Ser Ser
       130                 135                 140
   Glu Leu Thr Gln Asp Pro Ala Val Ser Val Ala Leu Gly Gln Thr Val
   145                 150                 155                 160
   Arg Ile Thr Cys Gln Gly Asp Ser Leu Arg Ser Tyr Tyr Ala Ser Trp
                   165                 170                 175
   Tyr Gln Gln Lys Pro Gly Gln Ala Pro Val Leu Val Ile Tyr Gly Lys
               180                 185                 190
   Asn Asn Arg Pro Ser Gly Ile Pro Asp Arg Phe Ser Gly Ser Ser Ser
           195                 200                 205
   Gly Asn Thr Ala Ser Leu Thr Ile Thr Gly Ala Gln Ala Glu Asp Glu
       210                 215                 220
   Ala Asp Tyr Tyr Cys Asn Ser Arg Asp Ser Ser Gly Asn His Val Val
   225                 230                 235                 240
   Phe Gly Gly Gly Thr Lys Leu Thr Val Leu Gly
                   245                 250
<210> 47
   <211> 245
   <212> PRT
   <213> Artificial Sequence
<220>
   <223> single chain fv 9A2
<400> 47
   Gln Val Gln Leu Val Gln Ser Gly Ala Glu Val Arg Lys Pro Gly Ala
   1               5                   10                  15
   Ser Val Lys Val Ser Cys Lys Thr Ser Gly Tyr Thr Phe Arg Asn Tyr
               20                  25                  30
   Asp Ile Asn Trp Val Arg Gln Ala Pro Gly Gln Gly Leu Glu Trp Met
           35                  40                  45
   Gly Arg Ile Ser Gly His Tyr Gly Asn Thr Asp His Ala Gln Lys Phe
       50                  55                  60
   Gln Gly Arg Phe Thr Met Thr Lys Asp Thr Ser Thr Ser Thr Ala Tyr
   65                  70                  75                  80
   Met Glu Leu Arg Ser Leu Thr Phe Asp Asp Thr Ala Val Tyr Tyr Cys
                   85                  90                  95
   Ala Arg Ser Gln Trp Asn Val Asp Tyr Trp Gly Arg Gly Thr Leu Val
               100                 105                 110
   Thr Val Ser Ser Gly Gly Gly Gly Ser Gly Gly Gly Gly Ser Gly Gly
           115                 120                 125
   Gly Gly Ser Ala Leu Asn Phe Met Leu Thr Gln Pro His Ser Val Ser
       130                 135                 140
   Glu Ser Pro Gly Lys Thr Val Thr Ile Ser Cys Thr Arg Ser Ser Gly
   145                 150                 155                 160
   Ser Ile Ala Ser Asn Tyr Val Gln Trp Tyr Gln Gln Arg Pro Gly Ser
                   165                 170                 175
   Ser Pro Thr Thr Val Ile Phe Glu Asp Asn Arg Arg Pro Ser Gly Val
               180                 185                 190
   Pro Asp Arg Phe Ser Gly Ser Ile Asp Thr Ser Ser Asn Ser Ala Ser
           195                 200                 205
   Leu Thr Ile Ser Gly Leu Lys Thr Glu Asp Glu Ala Asp Tyr Tyr Cys
       210                 215                 220
   Gln Ser Phe Asp Ser Thr Asn Leu Val Val Phe Gly Gly Gly Thr Lys
   225                 230                 235                 240
   Val Thr Val Leu Gly
                   245
<210> 48
   <211> 245
   <212> PRT
   <213> Artificial Sequence
<220>
   <223> single chain fv 11A4
<400> 48
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Ser Pro Tyr Ser Ser Arg Trp Tyr Ser Phe Asp Pro Trp Gly
               100                 105                 110
   Gln Gly Thr Met Val Thr Val Ser Ser Gly Gly Gly Gly Ser Gly Gly
           115                 120                 125
   Gly Gly Ser Gly Gly Gly Gly Ser Ala Leu Ser Tyr Glu Leu Thr Gln
       130                 135                 140
   Pro Pro Ser Val Ser Val Ser Pro Gly Gln Thr Ala Thr Ile Thr Cys
   145                 150                 155                 160
   Ser Gly Asp Asp Leu Gly Asn Lys Tyr Val Ser Trp Tyr Gln Gln Lys
                   165                 170                 175
   Pro Gly Gln Ser Pro Val Leu Val Ile Tyr Gln Asp Thr Lys Arg Pro
               180                 185                 190
   Ser Gly Ile Pro Glu Arg Phe Ser Gly Ser Asn Ser Gly Asn Ile Ala
   195 200 205
   Thr Leu Thr Ile Ser Gly Thr Gln Ala Val Asp Glu Ala Asp Tyr Tyr
   210 215 220
   Cys Gln Val Trp Asp Thr Gly Thr Val Val Phe Gly Gly Gly Thr Lys
   225 230 235 240
   Leu Thr Val Leu Gly
   245
<210> 49
   <211> 251
   <212> PRT
   <213> Artificial Sequence
<220>
   <223> single chain fv 7A4
<400> 49
   Glu Val Gln Leu Val Gln Ser Gly Ala Glu Val Lys Lys Pro Gly Glu
   1 5 10 15
   Ser Leu Thr Ile Ser Cys Lys Gly Ser Gly Tyr Asn Phe Phe Asn Tyr
   20 25 30
   Trp Ile Gly Trp Val Arg Gln Met Pro Gly Lys Asp Leu Glu Trp Met
   35 40 45
   Gly Ile Ile Tyr Pro Thr Asp Ser Asp Thr Arg Tyr Ser Pro Ser Phe
   50 55 60
   Gln Gly Gln Val Thr Ile Ser Val Asp Lys Ser Ile Ser Thr Ala Tyr
   65 70 75 80
   Leu Gln Trp Ser Ser Leu Lys Ala Ser Asp Thr Ala Met Tyr Tyr Cys
   85 90 95
   Ala Arg Ser Ile Arg Tyr Cys Pro Gly Gly Arg Cys Tyr Ser Gly Tyr
   100 105 110
   Tyr Gly Met Asp Val Trp Gly Gln Gly Thr Met Val Thr Val Ser Ser
   115 120 125
   Gly Gly Gly Ser Ser Gly Gly Gly Gly Ser Gly Gly Gly Gly Ser Ser
   130 135 140
   Glu Leu Thr Gln Asp Pro Ala Val Ser Val Ala Leu Gly Gln Thr Val
   145 150 155 160
   Arg Ile Thr Cys Arg Gly Asp Ser Leu Arg Asn Tyr Tyr Ala Ser Trp
   165 170 175
   Tyr Gln Gln Lys Pro Gly Gln Ala Pro Val Leu Val Ile Tyr Gly Lys
   180 185 190
   Asn Asn Arg Pro Ser Gly Ile Pro Asp Arg Phe Ser Gly Ser Ser Ser
   195 200 205
   Gly Asn Thr Ala Ser Leu Thr Ile Thr Gly Ala Gln Ala Glu Asp Glu
   210 215 220
   Ala Asp Tyr Tyr Cys Asn Ser Arg Asp Ser Ser Gly Asn His Met Val
   225 230 235 240
   Phe Gly Gly Gly Thr Lys Leu Thr Val Leu Gly
   245 250
<210> 50
   <211> 249
   <212> PRT
   <213> Artificial Sequence
<220>
   <223> single chain fv 11A1
<400> 50
   Glu Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Asp Phe
   20 25 30
   Ala Met His Trp Val Arg Gln Ile Pro Gly Lys Gly Leu Glu Trp Leu
   35 40 45
   Ser Gly Leu Arg His Asp Gly Ser Thr Ala Tyr Tyr Ala Gly Ser Val
   50 55 60
   Lys Gly Arg Phe Thr Ile Ser Arg Asp Asn Ser Arg Asn Thr Val Tyr
   65 70 75 80
   Leu Gln Met Asn Ser Leu Arg Ala Glu Asp Thr Ala Thr Tyr Tyr Cys
   85 90 95
   Val Thr Gly Ser Gly Ser Ser Gly Pro His Ala Phe Pro Val Trp Gly
   100 105 110
   Lys Gly Thr Leu Val Thr Val Ser Ser Gly Gly Gly Gly Ser Gly Gly
   115 120 125
   Gly Gly Ser Gly Gly Gly Gly Ser Ala Leu Ser Tyr Val Leu Thr Gln
   130 135 140
   Pro Pro Ser Ala Ser Gly Thr Pro Gly Gln Arg Val Thr Ile Ser Cys
   145 150 155 160
   Ser Gly Ser Asn Ser Asn Ile Gly Thr Tyr Thr Val Asn Trp Phe Gln
   165 170 175
   Gln Leu Pro Gly Thr Ala Pro Lys Leu Leu Ile Tyr Ser Asn Asn Gln
   180 185 190
   Arg Pro Ser Gly Val Pro Asp Arg Phe Ser Gly Ser Lys Ser Gly Thr
   195 200 205
   Ser Ala Ser Leu Ala Ile Ser Gly Leu Gln Ser Glu Asp Glu Ala Asp
   210 215 220
   Tyr Tyr Cys Ala Ala Trp Asp Asp Ser Leu Asn Gly Pro Val Phe Gly
   225 230 235 240
   Gly Gly Thr Lys Val Thr Val Leu Gly
   245
<210> 51
   <211> 251
   <212> PRT
   <213> Artificial Sequence
<220>
   <223> single chain fv 7A6
<400> 51
   Glu Val Gln Leu Val Gln Ser Gly Ala Glu Val Lys Lys Pro Gly Glu
   1 5 10 15
   Ser Leu Thr Ile Ser Cys Lys Gly Ser Gly Tyr Asn Phe Phe Asn Tyr
   20 25 30
   Trp Ile Gly Trp Val Arg Gln Met Pro Gly Lys Gly Leu Glu Trp Met
   35 40 45
   Gly Ile Ile Tyr Pro Thr Asp Ser Asp Thr Arg Tyr Ser Pro Ser Phe
   50 55 60
   Gln Gly Gln Val Thr Ile Ser Val Asp Lys Ser Ile Ser Thr Ala Tyr
   65 70 75 80
   Leu Gln Trp Ser Ser Leu Lys Ala Ser Asp Thr Ala Met Tyr Tyr Cys
   85 90 95
   Ala Arg Ser Ile Arg Tyr Cys Pro Gly Gly Arg Cys Tyr Ser Gly Tyr
   100 105 110
   Tyr Gly Met Asp Val Trp Gly Gln Gly Thr Leu Val Thr Val Ser Ser
   115 120 125
   Gly Gly Gly Gly Ser Gly Gly Gly Gly Ser Gly Gly Gly Gly Ser Ser
   130 135 140
   Glu Leu Thr Gln Asp Pro Ala Val Ser Val Ala Leu Gly Gln Thr Val
   145 150 155 160
   Arg Ile Thr Cys Gln Gly Asp Ser Leu Arg Ser Tyr Tyr Thr Asn Trp
   165 170 175
   Phe Gln Gln Lys Pro Gly Gln Ala Pro Leu Leu Val Val Tyr Ala Lys
   180 185 190
   Asn Lys Arg Pro Ser Gly Ile Pro Asp Arg Phe Ser Gly Ser Ser Ser
   195 200 205
   Gly Asn Thr Ala Ser Leu Thr Ile Thr Gly Ala Gln Ala Glu Asp Glu
   210 215 220
   Ala Asp Tyr Tyr Cys Asn Ser Arg Asp Ser Ser Gly Asn His Val Val
   225 230 235 240
   Phe Gly Gly Gly Thr Lys Leu Thr Val Leu Gly
   245 250
<210> 52
   <211> 5
   <212> PRT
   <213> Artificial Sequence
<220>
   <223> CDR
<400> 52
   Ser Tyr Trp Met His
   1 5
<210> 53
   <211> 17
   <212> PRT
   <213> Artificial Sequece
<220>
   <223> CDR
<400> 53
   Glu Ile Asn Pro Ser Asn Gly Arg Thr Asn Tyr Asn Glu Lys Phe Lys
   1 5 10 15
   Arg
<210> 54
   <211> 15
   <212> PRT
   <213> Artificial Sequence
<220>
   <223> CDR
<400> 54
   Gly Arg Pro Asp Tyr Tyr Gly Ser Ser Lys Trp Tyr Phe Asp Val
   1 5 10 15
<210> 55
   <211> 16
   <212> PRT
   <213> Artificial Sequence
<220>
   <223> CDR
<400> 55
   Arg Ser Ser Gln Ser Ile Val His Ser Asn Val Asn Thr Tyr Leu Glu
   1 5 10 15
<210> 56
   <211> 7
   <212> PRT
   <213> Artificial Sequence
<220>
   <223> CDR
<400> 56
   Lys Val Ser Asn Arg Phe Ser
   1 5
<210> 57
   <211> 9
   <212> PRT
   <213> Artificial Sequence
<220>
   <223> CDR
<400> 57
   Phe Gln Gly Ser His Val Pro Pro Thr
   1 5
<210> 58
   <211> 123
   <212> PRT
   <213> Artificial Sequence
<220>
   <223> heavy chain immunoglobulin variable region
<400> 58
   Gln Val Gln Leu Val Gln Ser Gly Ala Glu Val Val Lys Pro Gly Ala
   1 5 10 15
   Ser Val Lys Leu Ser Cys Lys Ala Ser Gly Tyr Thr Phe Thr Ser Tyr
   20 25 30
   Trp Met His Trp Val Lys Gln Arg Pro Gly Gln Gly Leu Glu Trp Ile
   35 40 45
   Gly Glu Ile Asn Pro Ser Asn Gly Arg Thr Asn Tyr Asn Gln Lys Phe
   50 55 60
   Gln Gly Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Tyr Phe
   85 90 95
   Ala Arg Gly Arg Pro Asp Tyr Tyr Gly Ser Ser Lys Trp Tyr Phe Asp
   100 105 110
   Val Trp Gly Gln Gly Thr Thr Val Thr Val Ser
   115 120
<210> 59
   <211> 118
   <212> PRT
   <213> Artificial Sequence
<220>
   <223> heavy chain immunoglobulin variable region
<400> 59
   Gln Val Gln Phe Gln Gln Ser Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Leu Met His Trp Ile Lys Gln Arg Pro Gly Arg Gly Leu Glu Trp Ile
           35                  40                  45
   Gly Arg Ile Asp Pro Asn Asn Val Val Thr Lys Phe Asn Glu Lys Phe
       50                  55                  60
   Lys Ser Lys Ala Thr Leu Thr Val Asp Lys Pro Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Arg Tyr Ala Tyr Cys Arg Pro Met Asp Tyr Trp Gly Gln Gly Thr
               100                 105                 110
   Thr Val Thr Val Ser Ser
           115
<210> 60
   <211> 123
   <212> PRT
   <213> Artificial Sequence
<220>
   <223> heavy chain immunoglobulin variable region
<400> 60
   Gln Val Gln Leu Gln Gln Ser Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Gln Gly Leu Glu Trp Ile
           35                  40                  45
   Gly Glu Ile Asn Pro Ser Asn Gly Arg Thr Asn Tyr Asn Glu Lys Phe
       50                  55                  60
   Lys Arg Lys Ala Thr Leu Thr Val Asp Lys Ser Ser Ser Thr Ala Tyr
   65                  70                  75                  80
   Met Gln Leu Ser Ser Leu Thr Ser Glu Asp Ser Ala Val Tyr Tyr Phe
                   85                  90                  95
   Ala Arg Gly Arg Pro Asp Tyr Tyr Gly Ser Ser Lys Trp Tyr Phe Asp
               100                 105                 110
   Val Trp Gly Ala Gly Thr Thr Val Thr Val Ser
           115                 120
<210> 61
   <211> 120
   <212> PRT
   <213> Artificial Sequence
<220>
   <223> heavy chain immunoglobulin variable region
<400> 61
   Gln Val Gln Leu Gln Gln Ser Gly Ala Glu Leu Met Lys Pro Gly Ala
   1               5                   10                  15
   Ser Val Lys Ile Ser Cys Lys Ala Thr Gly Tyr Thr Phe Ser Ser Phe
               20                  25                  30
   Trp Ile Glu Trp Val Lys Gln Arg Pro Gly His Gly Leu Glu Trp Ile
           35                  40                  45
   Gly Glu Ile Leu Pro Gly Ser Gly Gly Thr His Tyr Asn Glu Lys Phe
   50 55 60
   Lys Gly Lys Ala Thr Phe Thr Ala Asp Lys Ser Ser Asn Thr Ala Tyr
   65 70 75 80
   Met Gln Leu Ser Ser Leu Thr Ser Glu Asp Ser Ala Val Tyr Tyr Cys
   85 90 95
   Ala Arg Gly His Ser Tyr Tyr Phe Tyr Asp Gly Asp Tyr Trp Gly Gln
   100 105 110
   Gly Thr Ser Val Thr Val Ser Ser
   115 120
<210> 62
   <211> 120
   <212> PRT
   <213> Artificial Sequence
<220>
   <223> heavy chain immunoglobulin variable region
<400> 62
   Gln Val Gln Leu Gln Gln Pro Gly Ser Val Leu Val Arg Pro Gly Ala
   1 5 10 15
   Ser Val Lys Leu Ser Cys Lys Ala Ser Gly Tyr Thr Phe Thr Ser Ser
   20 25 30
   Trp Ile His Trp Ala Lys Gln Arg Pro Gly Gln Gly Leu Glu Trp Ile
   35 40 45
   Gly Glu Ile His Pro Asn Ser Gly Asn Thr Asn Tyr Asn Glu Lys Phe
   50 55 60
   Lys Gly Lys Ala Thr Leu Thr Val Asp Thr Ser Ser Ser Thr Ala Tyr
   65 70 75 80
   Val Asp Leu Ser Ser Leu Thr Ser Glu Asp Ser Ala Val Tyr Tyr Cys
   85 90 95
   Ala Arg Trp Arg Tyr Gly Ser Pro Tyr Tyr Phe Asp Tyr Trp Gly Gln
   100 105 110
   Gly Thr Thr Leu Thr Val Ser Ser
   115 120
<210> 63
   <211> 120
   <212> PRT
   <213> Artificial Sequence
<220>
   <223> heavy chain immunoglobulin variable region
<400> 63
   Gln Val Gln Leu Gln Gln Pro Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Arg Gly Leu Glu Trp Ile
           35                  40                  45
   Gly Arg Ile Asp Pro Asn Ser Gly Gly Thr Lys Tyr Asn Glu Lys Phe
       50                  55                  60
   Lys Ser Lys Ala Thr Leu Thr Val Asp Lys Pro Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Asp Tyr Tyr Gly Ser Ser Tyr Phe Asp Tyr Trp Gly Gln
               100                 105                 110
   Gly Thr Thr Leu Thr Val Ser Ser
           115                 120
<210> 64
   <211> 123
   <212> PRT
   <213> Artificial Sequence
<220>
   <223> heavy chain immunoglobulin variable region
<400> 64
   Gln Val Gln Leu Val Gln Ser Gly Ala Glu Val Val Lys Pro Gly Ala
   1               5                   10                  15
   Ser Val Lys Leu Ser Cys Lys Ala Ser Gly Tyr Thr Phe Thr Ser Tyr
   20 25 30
   Trp Met His Trp Val Lys Gln Arg Pro Gly Gln Gly Leu Glu Trp Ile
   35 40 45
   Gly Glu Ile Asn Pro Ser Asn Gly Arg Thr Asn Tyr Asn Gln Lys Phe
   50 55 60
   Gln Gly Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Tyr Phe
   85 90 95
   Ala Arg Gly Arg Pro Asp Tyr Tyr Gly Ser Ser Lys Trp Tyr Phe Asp
   100 105 110
   Val Trp Gly Gln Gly Thr Thr Val Thr Val Ser
   115 120
<210> 65
   <211> 124
   <212> PRT
   <213> Artificial Sequence
<220>
   <223> heavy chain immunoglobulin variable region
<400> 65
   Gln Val Gln Leu Gln Gln Ser Gly Ala Glu Leu Val Lys Pro Gly Ala
   1 5 10 15
   Ser Val Lys Leu Ser Cys Lys Ala Ser Gly Tyr Thr Phe Thr Ser Tyr
   20 25 30
   Trp Met His Trp Val Lys Gln Arg Pro Gly Gln Gly Leu Glu Trp Ile
   35 40 45
   Gly Glu Ile Asn Pro Ser Asn Gly Arg Thr Asn Tyr Asn Glu Lys Phe
   50 55 60
   Lys Arg Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Tyr Phe
   85 90 95
   Ala Arg Gly Arg Pro Asp Tyr Tyr Gly Ser Ser Lys Trp Tyr Phe Asp
   100 105 110
   Val Trp Gly Ala Gly Thr Thr Val Thr Val Ser Ser
   115 120
<210> 66
   <211> 124
   <212> PRT
   <213> Artificial Sequence
<220>
   <223> heavy chain immunoglobulin variable region
<400> 66
   Gln Val Gln Leu Val Gln Ser Gly Ala Glu Val Val Lys Pro Gly Ala
   1 5 10 15
   Ser Val Lys Leu Ser Cys Lys Ala Ser Gly Tyr Thr Phe Thr Ser Tyr
   20 25 30
   Trp Met His Trp Val Lys Gln Arg Pro Gly Gln Gly Leu Glu Trp Ile
   35 40 45
   Gly Glu Ile Asn Pro Ser Asn Gly Arg Thr Asn Tyr Asn Gln Lys Phe
   50 55 60
   Gln Gly Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Tyr Phe
   85 90 95
   Ala Arg Gly Arg Pro Asp Tyr Tyr Gly Ser Ser Lys Trp Tyr Phe Asp
   100 105 110
   Val Trp Gly Gln Gly Thr Thr Val Thr Val Ser Ser
   115 120
<210> 67
   <211> 120
   <212> PRT
   <213> Artificial Sequence
<220>
   <223> heavy chain immunoglobulin variable region
<400> 67
   Gln Val Gln Leu Gln Gln Ser Gly Ala Glu Leu Val Lys Pro Gly Ala
   1 5 10 15
   Ser Val Lys Leu Ser Cys Lys Ala Ser Gly Tyr Thr Phe Thr Ser Tyr
   20 25 30
   Trp Met His Trp Val Lys Gln Arg Pro Gly Arg Gly Leu Glu Trp Ile
   35 40 45
   Gly Arg Ile Asp Pro Asn Ser Gly Gly Thr Lys Tyr Asn Glu Lys Phe
   50 55 60
   Lys Ser Lys Ala Thr Leu Thr Val Asp Lys Pro Ser Ser Thr Ala Tyr
   65 70 75 80
   Met Gln Leu Ser Ser Leu Thr Ser Glu Asp Ser Ala Val Tyr Tyr Cys
   85 90 95
   Ala Arg Tyr Asp Tyr Tyr Gly Ser Ser Tyr Phe Asp Tyr Trp Gly Gln
   100 105 110
   Gly Thr Thr Val Thr Val Ser Ser
   115 120
<210> 68
   <211> 117
   <212> PRT
   <213> Artificial Sequence
<220>
   <223> heavy chain immunoglobulin variable region
<400> 68
   Gln Ile Gln Leu Gln Gln Ser Gly Pro Glu Leu Val Arg Pro Gly Ala
   1 5 10 15
   Ser Val Lys Ile Ser Cys Lys Ala Ser Gly Tyr Thr Phe Thr Asp Tyr
   20 25 30
   Tyr Ile His Trp Val Lys Gln Arg Pro Gly Glu Gly Leu Glu Trp Ile
   35 40 45
   Gly Trp Ile Tyr Pro Gly Ser Gly Asn Thr Lys Tyr Asn Glu Lys Phe
   50 55 60
   Lys Gly Lys Ala Thr Leu Thr Val Asp Thr Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Arg Gly Gly Lys Phe Ala Met Asp Tyr Trp Gly Gln Gly Thr Ser
   100 105 110
   Val Thr Val Ser Ser
   115
<210> 69
   <211> 124
   <212> PRT
   <213> Artificial Sequence
<220>
   <223> heavy chain immunoglobulin variable region
<400> 69
   Gln Val Gln Leu Gln Gln Ser Gly Ala Glu Leu Val Lys Pro Gly Ala
   1 5 10 15
   Ser Val Lys Leu Ser Cys Lys Ala Ser Gly Tyr Thr Phe Thr Ser Tyr
   20 25 30
   Trp Met His Trp Val Lys Gln Arg Pro Gly Gln Gly Leu Glu Trp Ile
   35 40 45
   Gly Glu Ile Asn Pro Ser Asn Gly Arg Thr Asn Tyr Asn Glu Lys Phe
   50 55 60
   Lys Arg Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Tyr Phe
   85 90 95
   Ala Arg Gly Arg Pro Asp Tyr Tyr Gly Ser Ser Lys Trp Tyr Phe Asp
   100 105 110
   Val Trp Gly Ala Gly Thr Thr Val Thr Val Ser Ser
   115 120
<210> 70
   <211> 120
   <212> PRT
   <213> Artificial Sequence
<220>
   <223> heavy chain immunoglobulin variable region
<400> 70
   Gln Ile Gln Leu Gln Gln Ser Gly Pro Glu Leu Val Lys Pro Gly Ala
   1               5                   10                  15
   Ser Val Lys Ile Ser Cys Lys Ala Ser Gly Tyr Thr Phe Thr Asp Tyr
               20                  25                  30
   Tyr Ile Asn Trp Met Lys Gln Lys Pro Gly Gln Gly Leu Glu Trp Ile
           35                  40                  45
   Gly Trp Ile Asp Pro Gly Ser Gly Asn Thr Lys Tyr Asn Glu Lys Phe
       50                  55                  60
   Lys Gly Lys Ala Thr Leu Thr Val Asp Thr Ser Ser Ser Thr Ala Tyr
   65                  70                  75                  80
   Met Gln Leu Ser Ser Leu Thr Ser Glu Asp Thr Ala Val Tyr Phe Cys
                   85                  90                  95
   Ala Arg Glu Lys Thr Thr Tyr Tyr Tyr Ala Met Asp Tyr Trp Gly Gln
               100                 105                 110
   Gly Thr Ser Val Thr Val Ser Ala
           115                 120
<210> 71
   <211> 115
   <212> PRT
   <213> Artificial Sequence
<220>
   <223> heavy chain immunoglobulin variable region
<400> 71
   Val Gln Leu Gln Gln Ser Gly Ala Glu Leu Met Lys Pro Gly Ala Ser
   1               5                   10                  15
   Val Lys Ile Ser Cys Lys Ala Ser Gly Tyr Thr Phe Ser Asp Tyr Trp
               20                  25                  30
   Ile Glu Trp Val Lys Gln Arg Pro Gly His Gly Leu Glu Trp Ile Gly
   35 40 45
   Glu Ile Leu Pro Gly Ser Gly Ser Thr Asn Tyr His Glu Arg Phe Lys
   50 55 60
   Gly Lys Ala Thr Phe Thr Ala Asp Thr Ser Ser Ser Thr Ala Tyr Met
   65 70 75 80
   Gln Leu Asn Ser Leu Thr Ser Glu Asp Ser Gly Val Tyr Tyr Cys Leu
   85 90 95
   His Gly Asn Tyr Asp Phe Asp Gly Trp Gly Gln Gly Thr Thr Leu Thr
   100 105 110
   Val Ser Ser
   115
<210> 72
   <211> 120
   <212> PRT
   <213> Artificial Sequence
<220>
   <223> heavy chain immunoglobulin variable region
<400> 72
   Gln Val Gln Leu Leu Glu Ser Gly Ala Glu Leu Met Lys Pro Gly Ala
   1 5 10 15
   Ser Val Lys Ile Ser Cys Lys Ala Thr Gly Tyr Thr Phe Ser Ser Phe
   20 25 30
   Trp Ile Glu Trp Val Lys Gln Arg Pro Gly His Gly Leu Glu Trp Ile
   35 40 45
   Gly Glu Ile Leu Pro Gly Ser Gly Gly Thr His Tyr Asn Glu Lys Phe
   50 55 60
   Lys Gly Lys Ala Thr Phe Thr Ala Asp Lys Ser Ser Asn Thr Ala Tyr
   65 70 75 80
   Met Gln Leu Ser Ser Leu Thr Ser Glu Asp Ser Ala Val Tyr Tyr Cys
   85 90 95
   Ala Arg Gly His Ser Tyr Tyr Phe Tyr Asp Gly Asp Tyr Trp Gly Gln
   100 105 110
   Gly Thr Ser Val Thr Val Ser Ser
   115 120
<210> 73
   <211> 113
   <212> PRT
   <213> Artificial Sequence
<220>
   <223> light chain immunoglobulin variable region
<400> 73
   Asp Val Leu Met Thr Gln Ile Pro Val Ser Leu Pro Val Ser Leu Gly
   1 5 10 15
   Asp Gln Ala Ser Ile Ser Cys Arg Ser Ser Gln Ile Ile Val His Asn
   20 25 30
   Asn Gly Asn Thr Tyr Leu Glu Trp Tyr Leu Gln Lys Pro Gly Gln Ser
   35 40 45
   Pro Gln Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Phe Thr Phe Gly Ser Gly Thr Lys Leu Glu Ile Lys
   100 105 110
   Arg
<210> 74
   <211> 113
   <212> PRT
   <213> Artificial Sequence
<220>
   <223> light chain immunoglobulin variable region
<400> 74
   Asp Val Leu Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 75
   <211> 113
   <212> PRT
   <213> Artificial Sequence
<220>
   <223> light chain immunoglobulin variable region
<400> 75
   Asp Val Leu Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Arg Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 76
   <211> 113
   <212> PRT
   <213> Artificial Sequence
<220>
   <223> light chain immunoglobulin variable region
<400> 76
   Asp Val Leu Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 77
   <211> 113
   <212> PRT
   <213> Artificial Sequence
<220>
   <223> light chain immunoglobulin variable region
<400> 77
   Asp Val Leu Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 78
   <211> 113
   <212> PRT
   <213> Artificial Sequence
<220>
   <223> light chain immunoglobulin variable region
<220>
   <221> misc_feature
   <222> (28)..(28)
   <223> Xaa can be any naturally occurring amino acid
<220>
   <221> misc_feature
   <222> (101)..(101)
   <223> Xaa can be any naturally occurring amino acid
<400> 78
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Xaa Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Xaa Thr Phe Gly Gly Gly Thr Lys Leu Glu Ile Lys
   100 105 110
   Arg
<210> 79
   <211> 113
   <212> PRT
   <213> Artificial Sequence
<220>
   <223> light chain immunoglobulin variable region
<400> 79
   Asp Val Val Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 80
   <211> 113
   <212> PRT
   <213> Artificial Sequence
<220>
   <223> light chain immunoglobulin variable region
<400> 80
   Asp Val Val Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 81
   <211> 113
   <212> PRT
   <213> Artificial Sequence
<220>
   <223> light chain immunoglobulin variable region
<400> 81
   Asp Val Leu Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Arg Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 82
   <211> 113
   <212> PRT
   <213> Artificial Sequence
<220>
   <223> light chain immunoglobulin variable region
<400> 82
   Asp Val Leu Met Thr Gln Ile Pro Val Ser Leu Pro Val Ser Leu Gly
   1               5                   10                  15
   Asp Gln Ala Ser Ile Ser Cys Arg Ser Ser Gln Ile Ile Val His Asn
               20                  25                  30
   Asn Gly Asn Thr Tyr Leu Glu Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Phe Thr Phe Gly Ser Gly Thr Lys Leu Glu Ile Lys
   100 105 110
   Arg
<210> 83
   <211> 113
   <212> PRT
   <213> Artificial Sequence
<220>
   <223> light chain immunoglobulin variable region
<400> 83
   Asp Val Leu Met Thr Gln Thr Pro Leu Ser Leu Pro Val Ser Leu Gly
   1 5 10 15
   Asp Gln Ala Ser Ile Ser Cys Arg Phe Ser Gln Ser Ile Val His Ser
   20 25 30
   Asn Gly Asn Thr Tyr Leu Glu Trp Tyr Leu Gln Lys Ser Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Arg Thr Phe Gly Gly Gly Thr Lys Leu Glu Ile Lys
   100 105 110
   Arg
<210> 84
   <211> 113
   <212> PRT
   <213> Artificial Sequence
<220>
   <223> light chain immunoglobulin variable region
<400> 84
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 85
   <211> 113
   <212> PRT
   <213> Artificial Sequence
<220>
   <223> light chain immunoglobulin variable region
<400> 85
   Asp Val Val Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 86
   <211> 113
   <212> PRT
   <213> Artificial Sequence
<220>
   <223> light chain immunoglobulin variable region
<400> 86
   Glu Leu Val Met Thr Gln Thr Pro Leu Ser Leu Pro Val Ser Leu Gly
   1 5 10 15
   Asp Gln Ala Ser Ile Ser Cys Arg Ser Ser Gln Thr Ile Val His Ser
   20 25 30
   Asn Gly Asp Thr Tyr Leu Asp Trp Phe Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 87
   <211> 113
   <212> PRT
   <213> Artificial Sequence
<220>
   <223> light chain immunoglobulin variable region
<400> 87
   Asp Val Leu Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ala Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 88
   <211> 113
   <212> PRT
   <213> Artificial Sequence
<220>
   <223> light chain immunoglobulin variable region
<400> 88
   Asp Val Val Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Arg Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 89
   <211> 113
   <212> PRT
   <213> Artificial Sequence
<220>
   <223> light chain immunoglobulin variable region
<400> 89
   Asp Val Leu Met Thr Gln Thr Pro Val Ser Leu Ser Val Ser Leu Gly
   1 5 10 15
   Asp Gln Ala Ser Ile Ser Cys Arg Ser Ser Gln Ser Ile Val His Ser
   20 25 30
   Thr Gly Asn Thr Tyr Leu Glu Trp Tyr Leu Gln Lys Pro Gly Gln Ser
   35 40 45
   Pro Lys Leu Leu Ile Tyr Lys Ile Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Ala
   85 90 95
   Ser His Ala Pro Arg Thr Phe Gly Gly Gly Thr Lys Leu Glu Ile Lys
   100 105 110
   Arg
<210> 90
   <211> 113
   <212> PRT
   <213> Artificial Sequence
<220>
   <223> light chain immunoglobulin variable region
<400> 90
   Asp Val Leu Met Thr Gln Thr Pro Leu Ser Leu Pro Val Ser Leu Gly
   1 5 10 15
   Asp Gln Ala Ser Ile Ser Cys Lys Ser Ser Gln Ser Ile Val His Ser
   20 25 30
   Ser Gly Asn Thr Tyr Phe Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Ile Pro Phe Thr Phe Gly Ser Gly Thr Lys Leu Glu Ile Lys
   100 105 110
   Arg
<210> 91
   <211> 113
   <212> PRT
   <213> Artificial Sequence
<220>
   <223> light chain immunoglobulin variable region
<400> 91
   Asp Ile Glu Leu Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Tyr Thr Phe Gly Gly Gly Thr Lys Leu Glu Ile Lys
               100                 105                 110
   Arg
<210> 92
   <211> 113
   <212> PRT
   <213> Artificial Sequence
<220>
   <223> light chain immunoglobulin variable region
<400> 92
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 93
   <211> 113
   <212> PRT
   <213> Artificial Sequence
<220>
   <223> light chain immunoglobulin variable region
<400> 93
   Asp Val Val Met Thr Gln Thr Pro Leu Ser Leu Pro Val Ser Leu Gly
   1 5 10 15
   Asp Pro Ala Ser Ile Ser Cys Arg Ser Ser Gln Ser Ile Val His Ser
   20 25 30
   Asn Val Asn Thr Tyr Leu Glu Trp Tyr Leu Gln Lys Pro Gly Gln Ser
   35 40 45
   Pro Arg Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 94
   <211> 113
   <212> PRT
   <213> Artificial Sequence
<220>
   <223> light chain immunoglobulin variable region
<400> 94
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Arg Ile
   65                  70                  75                  80
   Ser Arg Val Glu Ala Glu Asp Leu Gly Ile Tyr Tyr Cys Phe Gln Gly
                   85                  90                  95
   Ser His Val Pro Pro Thr Phe Gly Gly Gly Thr Lys Leu Glu Ile Lys
               100                 105                 110
   Arg
<210> 95
   <211> 113
   <212> PRT
   <213> Artificial Sequence
<220>
   <223> light chain immunoglobulin variable region
<400> 95
   Asp Val Val Met Thr Gln Thr Pro Leu Ser Leu Pro Val Ser Leu Gly
   1               5                   10                  15
   Asp Pro Ala Ser Ile Ser Cys Arg Ser Ser Gln Ser Ile Val His Ser
               20                  25                  30
   Asn Val Asn Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ala Gly Thr Asp Phe Thr Leu Arg Ile
   65 70 75 80
   Ser Arg Val Glu Ala Glu Asp Leu Gly Ile Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 96
   <211> 113
   <212> PRT
   <213> Artificial Sequence
<220>
   <223> light chain immunoglobulin variable region
<400> 96
   Asp Val Leu Met Thr Gln Thr Pro Leu Ser Leu Pro Val Ser Leu Gly
   1 5 10 15
   Asp Gln Ala Ser Ile Ser Cys Arg Ser Asn Gln Thr Ile Leu Leu Ser
   20 25 30
   Asp Gly Asp Thr Tyr Leu Glu Trp Tyr Leu Gln Lys Pro Gly Gln Ser
   35 40 45
   Pro Lys Leu Leu Ile Tyr Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Pro Thr Phe Gly Gly Gly Thr Lys Leu Glu Ile Lys
   100 105 110
   Arg
<210> 97
   <211> 113
   <212> PRT
   <213> Artificial Sequence
<220>
   <223> light chain immunoglobulin variable region
<400> 97
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Thr Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Thr Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Thr His Ala Pro Tyr Thr Phe Gly Gly Gly Thr Lys Leu Glu Ile Lys
               100                 105                 110
   Arg
<210> 98
   <211> 113
   <212> PRT
   <213> Artificial Sequence
<220>
   <223> light chain immunoglobulin variable region
<400> 98
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Ser Ile Ser Ser Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Gln Ala Glu Asp Leu Gly Val Tyr Tyr Cys Phe Gln Gly
   85 90 95
   Ser His Val Pro Tyr Thr Phe Gly Gly Gly Thr Lys Leu Glu Ile Lys
   100 105 110
   Arg
<210> 99
   <211> 14
   <212> DNA
   <213> Artificial Sequence
<220>
   <223> primer
<400> 99
   ctccgcttcc tttc 14
<210> 100
   <211> 18
   <212> DNA
   <213> Artificial Sequence
<220>
   <223> anti-sense
<400> 100
   atctctccgc ttcctttc 18
<210> 101
   <211> 18
   <212> DNA
   <213> Artificial Sequence
<220>
   <223> anti-sense
<400> 101
   atctctccgc ttcctttc 18
<210> 102
   <211> 19
   <212> DNA
   <213> Artificial Sequence
<220>
   <223> primer
<400> 102
   aggagctcga ggcgttcag 19
<210> 103
   <211> 22
   <212> DNA
   <213> Artificial Sequence
<220>
   <223> primer
<400> 103
   gtcttgggtg ggtagagcaa tc 22
<210> 104
   <211> 21
   <212> DNA
   <213> Artificial Sequence
<220>
   <223> primer
<400> 104
   aggccaaacg tcaccgtccc c 21
<210> 105
   <211> 109
   <212> PRT
   <213> Artificial Sequence
<220>
   <223> A12 immunoglobulin light chain variable region
<400> 105
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Thr Trp Tyr Gln Gln Lys Pro Gly Gln Ala Pro Ile Leu Val Ile Tyr
   35 40 45
   Gly Glu Asn Lys Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Lys Ser Arg Asp Gly Ser Gly Gln His
   85 90 95
   Leu Val Phe Gly Gly Gly Thr Lys Leu Thr Val Leu Gly
   100 105
<210> 106
   <211> 22
   <212> DNA
   <213> Artificial Sequence
<220>
   <223> IGF1R/forward primer
<400> 106
   gaaagtgacg tcctgcattt ca 22
<210> 107
   <211> 19
   <212> DNA
   <213> Artificial Sequence
<220>
   <223> IGF1R/reverse Primer
<400> 107
   ccggtgccag gttatgatg 19
<210> 108
   <211> 23
   <212> DNA
   <213> Artificial Sequence
<220>
   <223> Probe Sequence
<400> 108
   caccaccacg tcgaagaatc gca 23

## Description

### Field of the Invention

The present invention relates to compositions for treating or preventing cancer.

### Background of the Invention

The insulin-like growth factors, also known as somatomedins, include insulin-like growth factor-I (IGF-I) and insulin-like growth factor-II (IGF-II) (Klapper, et al., (1983) Endocrinol. 112:2215 and Rinderknecht, et al., (1978) Febs.Lett. 89:283). These growth factors exert mitogenic activity on various cell types, including tumor cells (Macaulay, (1992) Br. J. Cancer 65:311), by binding to a common receptor named the insulin-like growth factor-1 receptor (IGF1R or IGFR1) (Sepp-Lorenzityo, (1998) Breast Cancer Research and Treatment 47:235). Interaction of IGFs with IGF1R activates the receptor by triggering autophosphorylation of the receptor on tyrosine residues (Butler, et a/., (1998) Comparative Biochemistry and Physiology 121:19). Once activated, IGF1R, in turn, phosphorylates intraceflular targets to activate cellular signaling pathways. This receptor activation is critical for stimulation of tumor cell growth and survival. Therefore, inhibition of IGF1R activity represents a valuable potential method to treat or prevent growth of human cancers and other proliferative diseases.

Accordingly, therapies that inhibit IGF1R are useful for the treatment or prevention of certain cancers. Anti-IGF1 R antibodies are useful therapies for treating or preventing the cancers. There are several anti-IGF1R antibodies that are known in the art (see *e.g.*, WO 03/100008; WO 2002/53596; WO 04/71529; WO 03/106621; US 2003/235582; WO 04/83248; WO 03/59951; WO 04/87756 or WO 2005/16970). Other small molecule IGF 1R inhibitors are also known in the art.

Although there are IGF1R inhibitors known in the art that may be used to treat or prevent some cancers, there remains a need in the art for therapeutic compositions and methods for treating or preventing other cancers such as head and neck cancer, squamous cell carcinoma, solitary plasmacytoma, multiple myeloma and renal cell cancer.

### Summary of the Invention

The present invention addresses this need, in part, by providing IGF1R inhibitors and combinations thereof that are effective at treating or preventing squamous cell carcinoma, or renal cell cancer.

Thus the present invention provides an isolated antibody or antigen-binding fragment thereof comprising CDR-H1, CDR-H2 and CDR-H3 from a heavy chain immunoglobulin comprising the amino acid sequence set forth in SEQ ID NO: 10; or CDR-L1, CDR-L2 and CDR-L3 from a light chain immunoglobulin comprising the amino acid sequence set forth in SEQ ID NO: 8; or both, for use in treating or preventing a medical condition, by administration to a mammalian subject, wherein the medical condition is squamous cell carninoma or renal cell cancer.

In an embodiment of the invention, the IGF1R inhibitor is selected from the group consisting of an isolated antibody or an antigen-binding fragment thereof wherein the antibody or antigen-binding fragment thereof comprises: (a) a light chain variable region comprising amino acids 20-128 of SEQ ID NO: 2 and a heavy chain variable region comprising amino acids 20-137 of SEQ ID NO: 10 or 12; (b) a light chain variable region comprising amino acids 20-128 of SEQ ID NO: 4 and a heavy chain variable region comprising amino acids 20-137 of SEQ ID NO: 10 or 12; (c) a light chain variable region comprising amino acids 20-128 of SEQ ID NO: 6 and a heavy chain variable region comprising amino acids 20-137 of SEQ ID NO: 10 or 12; (d) a light chain variable region comprising amino acids 20-128 of SEQ ID NO: 8 and a heavy chain variable region comprising amino acids 20-137 of SEQ ID NO: 10 or 12. In an embodiment of the invention, the IGF1R inhibitor is administered in association with one or more further anti-cancer chemotherapeutic agents or a pharmaceutical composition thereof. In an embodiment of the invention, the further anti-cancer chemotherapeutic agent is a member selected from the group consisting of teniposide 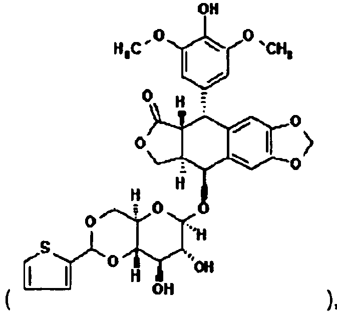 cisplatin 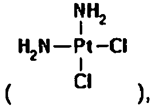 satraplatin, carboplatin 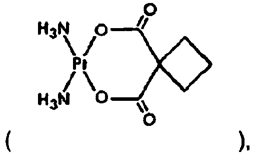 etoposide 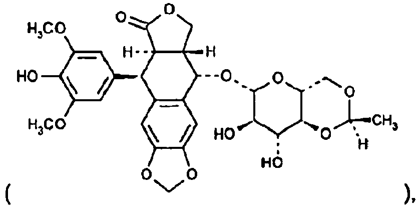 doxorubicin 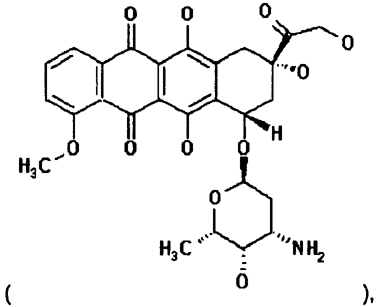 any liposomal formulation thereof such as Caelyx or Doxil®, cyclophosphamide 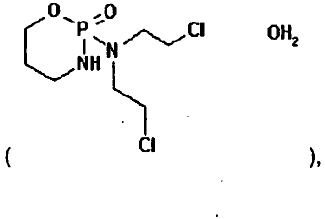 13- cis-retinoic acid 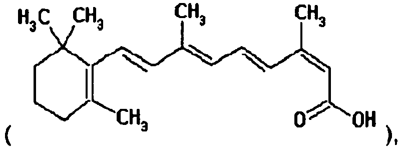 ifosfamide 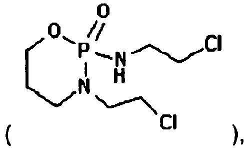 gemcitabine 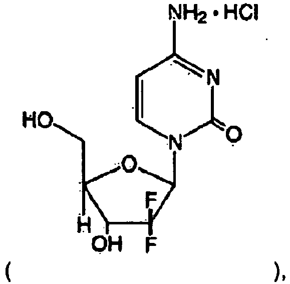 irinotecan ( 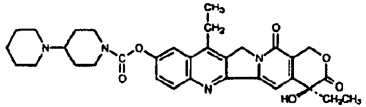 ), vincristine 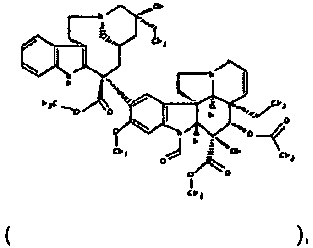 dactinomycin 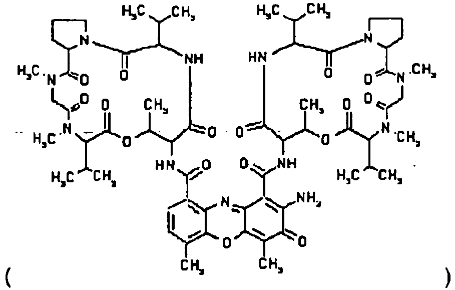 methotrexate 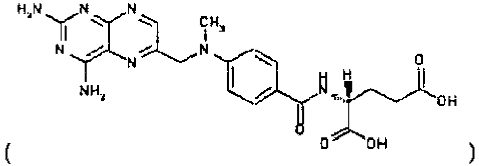 and any other chemotherapeutic agent set forth herein, for example, as set forth under the "Further Chemotherapeutics" section below. In an embodiment of the invention, the dosage of any anti-IGF1R antibody set forth herein is in the range of about 0.3-20 mg/kg of body weight or about 40-1200 mg/m². In an embodiment of the invention, the IGF1R inhibitor and the further anti-cancer therapeutic agent are administered simultaneously. In an embodiment of the invention, the IGF1R inhibitor and the further anti-cancer therapeutic agent are administered non-simultaneously. In an embodiment of the invention, the antibody comprises an IgG constant region. In an embodiment of the invention, the subject is a human (*e.g.*, a child). In an embodiment of the invention, the IGF1R inhibitor is administered in association with an anti-cancer therapeutic procedure.

### Brief Description of the Figures

**Figure 1****.** *IGF1R* mRNA expression in primary head and neck tumor samples as measued using Taqman. Each point represents the normalized expression level of IGF1R in a single tissue sample. The stage of head and neck cancer in the patient from which each sample was obtained is marked I, II, III or IV. Values corresponding to normal tissue samples are marked "normal"' and values corresponding to normal adjacent tissue, located adjacent to the tumor cells, but otherwise exhibiting normal characteristics, are marked "NAT".
**Figure 2**. Western blot analysis of the protein expression level of total *IGF1R*, *IGF-I* and *IGF-II* in various cell lines (top panel). Quantity of IGF1 and IGF2 secreted from various cell lines into the surrounding growth medium (ng IGF1 or IGF2 protein/10⁶ cells) (bottom panel).
**Figures 3(a)-3(c)****.** Inhibition of squamous cell carcinoma *in vitro* cell lines at various concentrations of anti-IGF1R antibody LCFIHCA. (a): cell line SCC 15; (b): cell line SCC 25; (c): cell line SCC 9.
**Figure 4**. Expression level of *IGF2* in primary stomach adenocarcinoma tumor tissue as compared to that of normal tissue.
**Figure 5****.** Expression levels of various protein in several gastic and ovarian cancer cell lines as measured by western blot analysis.
**Figure 6**. *In vitro* growth inhibition of squamous cell carcinoma cancer cells (cell line SNU-16) at various concentrations of anti-IGF1R antibody LCF/HCA.
**Figure 7**. Renal cell carcinoma tumor growth in a mouse xenograft model over time after exposure to anti-IGF1R antibody LCF/HCA or a control immunoglobulin.
**Figure 8**. *In vitro* growth inhibition of melanoma cell line A375-SM when exposed to various concentrations of anti-IGF1R antibody LCF/HCA.
**Figure 9**. *In vivo* growth inhibition of squamous cell carcinoma cell line SCC15 in mice when exposed to anti-IGF1R antibody LCF/HCA.
**Figure 10**. Evaluation of tumor volume over time in EW5 (Ewins sarcoma), OS-1 (osteosarcoma) and OS-9 (osteosarcoma) xenograft tumor models.

### Detailed Description of the Invention

The present invention comprises compositions for treating or preventing squamous cell carcinoma or renal cell cancer. The cancer is treated or prevented by administering an IGF1R inhibitor which is an anti-IGF1R antibody as defined above. The antibody can be associated with a further chemotherapeutic agent, such as an anti-cancer chemotherapeutic agent such as any of those set forth herein.

The terms IGF1R, IGFR1 and IGF-1R are synonymous and refer to insulin-like growth factor 1 receptor.

### IGF1R inhibitors

the terms "IGF1R inhibitor" or "IGF1R antagonist" or the like includes an antibody or antigen-binding fragment thereof as defined above that decreases the expression, ligand binding (*e.g.*, binding to IGF-1 and/or IGF-2), kinase activity (*e.g.*, autophosphorylation activity) or any other biological activity of IGF1R (*e.g.*, mediation of anchorage independent cellular growth) and the phospho-IRS-1 level that will elicit a biological or medical response of a tissue, system, subject or patient that is being sought by the administrator (such as a researcher, doctor or veterinarian) which includes any measurable alleviation of the signs, symptoms and/or clinical indicia of both non-cancerous medical disorders and of cancer (*e.g.*, tumor growth) and/or the prevention, slowing or halting of progression or metastasis of squamous cell carcinoma or renal cell cancer to any degree.

In one aspect the invention, an IGF1R inhibitor that is for administration to a patient according to the invention is any isolated antibody or antigen-binding fragment thereof that binds specifcally to insulin-like growth factor-1 receptor (*e.g.*, human IGF1R) (*e.g.*, monoclonal antibodies (*e.g.*, fully human monoclonal antibodies), polyclonal antibodies, bispecific antibodies, Fab antibody fragments, F(ab)₂ antibody fragments, Fv antibody fragments (*e.g.*, VH or VL), single chain Fv antibody fragments, dsFv antibody fragments, humanized antibodies, chimeric antibodies or anti-idiotypic antibodies) such as any of those disclosed in any of Burtrum et. al Cancer Research 63:8912-8921 (2003); in French Patent Applications FR2834990, FR2834991 and FR2834900 and in PCT Application Publication Nos. WO 03/100008; WO 03/59951; WO 2006/13472; WO 04/71529; WO 03/106621; WO 04/83248; WO 04/87756, WO 05/16970; and WO 02/53596.

In another embodiment of the invention, an IGF1R inhibitor that is for administration to a patient according to the invention is an isolated anti-insulin-like growth factor-1 receptor (IGF1R) antibody comprising a mature 19D12/15H12 Light Chain-C, D, E or F (LCC, LCD, LCE or LCF) and a mature 19D12/15H12 heavy chain-A or B (HCA or HCB). In an embodiment of the invention, the antibody comprises the mature LCF and the mature HCA (LCF/HCA). In an embodiment of the invention, an IGF1R inhibitor that is for administration to a patient according to the invention is an isolated antibody that specifically binds to IGF1R that comprises one or more complementarity determining regions (CDRs) of 19D12/15H12 Light Chain-C, D, E or F and/or 19D12/15H12 heavy chain-A or B (*e.g.*, all 3 light chain CDRs and all 3 heavy chain CDRs).

The amino acid and nucleotide sequences of the some antibody chains of the invention are shown below. Dotted, underscored type indicates the signal peptide. Solid underscored type indicates the CDRs. Plain type indicates the framework regions. Mature fragments lack the signal peptide.

The present invention includes embodiments wherein the antibody chains set forth herein are substituted conservatively with one or more mutations that not significantly affect antibody binding activity.

Plasmids comprising a CMV promoter operably linked to the 15H12/19D12 light chains and heavy chains have been deposited at the American Type Culture Collection (ATCC); 10801 University Boulevard; Manassas, Virginia 20110-2209 on May 21, 2003. The deposit name and the ATCC accession numbers for the plasmids are set forth below:
(i) CMV promoter-15H12/19D12 HCA (y4)-
   Deposit name: "15H12/19D12 HCA (γ4)";
   ATCC accession No.: PTA-5214;
(ii) CMV promoter-15H12/19D12 HCB (γ4)-
   Deposit name: "15H12/19D12 HCB (γ4)";
   ATCC accession No.: PTA-5215;
(iii) CMV promoter-15H12/19D12 HCA (γ1)-
   Deposit name: "15H12/19D12 HCA (γ1)";
   ATCC accession No.: PTA-5216;
(iv) CMV promoter-15H12/19D12 LCC (κ)-
   Deposit name: "15H12/19D12 LCC (κ)";
   ATCC accession No.: PTA-5217;
(v) CMV promoter-15H12/19D12 LCD (κ)-
   Deposit name: "15H12/19D12 LCD (κ)";
   ATCC accession No.: PTA-5218;
(vi) CMV promoter-15H12l19D12 LCE (κ)-
   Deposit name: "15H12/19D12 LCE (κ)";
   ATCC accession No.: PTA-5219; and
(vii) CMV promoter-15H12/19D12 LCF (κ)-
   Deposit name: "15H12/19D12 LCF (κ)";
   ATCC accession No.: PTA-5220;

The present invention includes compositions (*e.g.*, any disclosed herein) comprising anti-IGF1R antibodies and antigen-binding fragments thereof comprising any of the light and/or heavy immunoglobulin chains or mature fragments thereof located in any of the foregoing plasmids deposited at the ATCC.

In an embodiment of the invention, an antibody that binds "specifically" to human IGF1R binds with a Kd of about 10⁻⁸ M or 10⁻⁷ M or a smaller number; or, in an embodiment of the invention, with a Kd of about 1.28X10⁻¹⁰ M or a smaller number by Biacore measurement or with a Kd of about 2.05X10⁻¹² M or a lower number by KinExA measurement. In another embodiment, an antibody that binds "specifically" to human IGF1R binds exclusively to human IGF1R and to no other protein in any specific amount.

In an embodiment of the invention, an IGF1R inhibitor that is for administration to a patient according to the invention comprises any light chain immunoglobulin and/or a heavy chain immunoglobulin as set forth in Published International Application No. WO 2002/53596. For example, in an embodiment of the invention, the antibody comprises a light chain variable region comprising an amino acid sequence selected from the group consisting of SEQ ID NOs: 2, 6,10, 14, 18, 22, 47 and 51 as set forth in WO 2002/53596 and/or a heavy chain variable region comprising an amino acid sequence selected from the group consisting of SEQ ID NOs: 4, 8, 12, 16, 20, 24, 45 and 49 as set forth in WO 2002/53596. In an embodiment of the invention, the antibody comprises a heavy and/or light chain selected from that of antibody 2.12.1; 2.13.2; 2.14.3; 3.1.1; 4.9.2; and 4.17.3 in WO 2002/53596.

In an embodiment of the invention, an IGF1R inhibitor for administration to a patient according to the invention comprises any light chain immunoglobulin and/or a heavy chain immunoglobulin as set forth in Published International Application No. WO 2003/59951. For example, in an embodiment of the invention, the antibody comprises a light chain variable region comprising an amino acid sequence selected from the group consisting of SEQ ID NOs: 54, 61 and 65 as set forth in WO 2003/59951 and/or a heavy chain variable region comprising an amino acids sequence selected from the group consisting of SEQ ID NOs: 69, 75, 79 and 83 as set forth in WO 2003/59951.

In an embodiment of the invention, an IGF1R inhibitor for administration to a patient according to the invention comprises any light chain immunoglobulin and/or a heavy chain immunoglobulin as set forth in Published International Application No. WO 2004/83248. For example, in an embodiment of the invention, the antibody comprises a light chain variable region comprising an amino acid sequence selected from the group consisting of SEQ ID NOs: 109, 111, 113, 115, 117, 119, 121, 123, 125, 127, 129, 131, 133, 135, 137, 139, 141 and 143 as set forth in WO 2004/83248 and/or a heavy chain variable region comprising an amino acids sequence selected from the group consisting of SEQ ID NOs: 108, 110, 112, 114, 116, 118, 120, 122, 124, 126, 128, 130, 132, 134, 136, 138, 140 and 142 as set forth in WO 2004/83248. In an embodiment of the invention, the antibody comprises a light and/or heavy chain selected from that of PINT-6A1 ; PINT-7A2; PINT-7A4; PINT-7A5; PINT-7A6; PINT-8A1; PINT-9A2; PINT-11A1; PINT-11A2; PINT-11A3; PINT-11A4; PINT-11A5; PINT-11A7; PINT-12A1; PINT-12A2; PINT-1 2A3; PINT-12A4 and PINT-12A5 in WO 2004/83248.

In an embodiment of the invention, an IGF1R inhibitor for administration to a patient according to the invention comprises any light chain immunoglobulin and/or a heavy chain immunoglobulin as set forth in Published International Application No. WO 2003/106621. For example, in an embodiment of the invention, the antibody comprises a light chain variable region comprising an amino acid sequence selected from the group consisting of SEQ ID NOs: 8-12, 58-69, 82-86, 90, 94, 96, 98, as set forth in WO 2003/106621 and/or a heavy chain variable region comprising an amino acids sequence selected from the group consisting of SEQ ID NOs: 7. 13, 70-81, 87, 88, 92 as set forth in WO 2003/106621.

In an embodiment of the invention, an IGF1R inhibitor for administration to a patient according to the invention comprises any light chain immunoglobulin and/or a heavy chain immunoglobulin as set forth in Published International Application No. WO 2004/87756. For example, in an embodiment of the invention, the antibody comprises a light chain variable region comprising an amino acid sequence of SEQ ID NO: 2 as set forth in WO 2004/87756 and/or a heavy chain variable region comprising an amino acid sequence of SEQ ID NO: 1 as set forth in WO 2004/87756.

In an embodiment of the invention, an IGF1R inhibitor for administration to a patient according to the invention comprises any light chain immunoglobulin and/or a heavy chain immunoglobulin as set forth in Published International Application No. VVO 2005/16970. For example, in an embodiment of the invention, the antibody comprises a light chain variable region comprising an amino acid sequence of SEQ ID NO: 6 or 10 as set forth in WO 2005/16970 and/or a heavy chain variable region comprising an amino acid sequence of SEQ ID NO: 2 as set forth in WO 2005/16970.

In an embodiment of the invention, an anti-IGF1R antibody or antigen-binding fragment thereof of the invention comprises an immunoglobulin heavy chain variable region comprising an amino acid sequence selected from the group consisting of:

In an embodiment of the invention, an anti-IGF1R antibody or antigen-binding fragment thereof of the invention comprises an immunoglobulin light chain variable region comprising an amino acid sequence selected from the group consisting of:

In an embodiment of the invention, the anti-IGF1R antibody comprises a light chain immunoglobulin, or a mature fragment thereof (*i.e.*, lacking signal sequence), or variable region thereof, comprising the amino acid sequence of: or In an embodiment of the invention, the signal sequence is amino acids 1-22 of SEQ ID NOs: 25-28. In an embodiment of the invention, the mature variable region is underscored. In an embodiment of the invention, the CDRs are in bold/italicized font. In an embodiment of the invention, the anti-IGF1R antibody or antigen-binding fragment thereof of the invention comprises one or more CDRs (*e.g.*, 3 light chain CDRS) as set forth above.

In an embodiment of the invention, the anti-IGF1R antibody comprises a heavy chain immunoglobulin or a mature fragment thereof (*i.e.*, lacking signal sequence), or a variable region thereof, comprising the amino acid sequence of: or In an embodiment of the invention, the signal sequence is amino acids 1-19 of SEQ ID NOs: 29-32. In an embodiment of the invention, the mature variable region is underscored. In an embodiment of the invention, the anti-IGF1R antibody or antigen-binding fragment thereof of the invention comprises one or more CDRs (*e.g.*, 3 light chain CDRS) as set forth above.

In an embodiment of the invention, an anti-IGF1R antibody or antigen-binding fragment thereof of the invention comprises an immunoglobulin heavy chain or mature fragment or variable region of 2.12.1 fx (SEQ ID NO: 33) (in an embodiment of the invention, the leader sequence is underscored; in an embodiment of the invention, the CC7Rs are in bold/italicized font):

In an embodiment of the invention, the anti-IGF1R antibody or antigen-binding fragment thereof of the invention comprises amino acids 20-470 of 2.12.1 fx (SEQ ID NO: 33).

In an embodiment of the invention, an anti-IGF1R antibody or antigen-binding fragment thereof of the invention comprises mature immunoglobulin heavy chain variable region 2.12.1 fx (amino acids 20-144 or SEQ ID NO: 33; SEQ ID NO: 34):
q vqlvesgggl vkpggslrls caasgftfsd yymswirqap gkglewvsyi sssgstrdya dsvkgrftis rdnaknslyl qmnslraedt avyycardgv ettfyyyyyg mdvwgqgttv tvss

In an embodiment of the invention, an anti-1GF1R antibody or antigen-binding fragment thereof of the invention comprises an immunoglobulin light chain or mature fragment or variable region 2.12.1 fx (SEQ ID NO: 35) (in an embodiment of the invention, the leader sequence is underscored; in an embodiment of the invention, the CDRs are in bold/italicized font):

In an embodiment of the invention, an anti-IGF1R antibody or antigen-binding fragment thereof of the invention comprises amino acids 23-236 of 2.12.1 fx (SEQ ID NO: 35).

In an embodiment of the invention, an anti-IGF1R antibody or antigen-binding fragment thereof of the invention comprises mature immunoglobulin light chain variable region 2.12.1 fx (amino acids 23-130 of SEQ ID NO: 35; SEQ ID NO: 36):

In an embodiment of the invention, an anti-IGF1R antibody or antigen-binding fragment thereof comprises or consists of a light chain immunoglobulin chain comprising or consisting of amino acids 23-236 of 2.12.1 fx (SEQ ID NO: 35) and a heavy chain immunoglobulin chain comprising or consisting of amino acids 20-470 of 2.12.1 fx (SEQ ID NO: 33).

In an embodiment of the invention, the anti-IGF1 R antibody or antigen-binding fragment thereof comprises one or more 2.12.1 fx CDRs (*e.g.*, 3 light chain CDRs and/or 3 heavy chain CDRs) as set forth above.

In an embodiment of the invention, an anti-IGF1R antibody or antigen-binding fragment thereof of the invention or antigen-binding fragment thereof comprises a humanized 7C10 immunoglobulin light chain variable region; version 1 (SEQ ID NO: 37):

In an embodiment of the invention, an anti-IGF1R antibody or antigen-binding fragment thereof of the invention comprises humanized 7C10 immunoglobulin light chain variable region; version 2 (SEQ ID NO: 38):

In an embodiment of the invention, an anti-IGF1R antibody or antigen-binding fragment thereof of the invention comprises a humanized 7C10 immunoglobulin heavy chain variable region; version 1 (SEQ ID NO: 39):

In an embodiment of the invention, an anti-IGF1R antibody or antigen-binding fragment thereof of the invention comprises the humanized 7C10 immunoglobulin heavy chain variable region; version 2 (SEQ lD NO: 40):

In an embodiment of the invention, an anti-IGF1R antibody or antigen-binding fragment thereof of the invention comprises the humanized 7C10 immunoglobulin heavy chain variable region; version 3 (SEQ ID NO: 41):

In an embodiment of the invention, an anti-IGF1R antibody or antigen-binding fragment thereof of the invention comprises A12 immunoglobulin heavy chain variable region (SEQ ID NO: 42):

In an embodiment of the invention, an anti-IGF1R antibody or antigen-binding fragment thereof of the invention comprises A12 immunoglobulin light chain variable region (SEQ ID NO: 43): or (SEQ ID NO: 105):

In an embodiment of the invention, an anti-IGF1 R antibody or antigen-binding fragment thereof of the invention comprises 1A immunoglobulin heavy chain variable region (SEQ ID NO: 44): ;optionally including one or more of the following mutations: R30, S30, N31, S31, Y94, H94, D104, E104.

In an embodiment of the invention, an anti-IGF1R antibody or antigen-binding fragment thereof of the invention comprises 1A immunoglobulin light chain variable region (SEQ ID NO: 45): ;optionally including one or more of the following mutations: P96, 196, P100, Q100, R103, K103, V104, L104, D105, E105

In an embodiment of the invention, an anti-IGF1R antibody or antigen-binding fragment thereof of the invention comprises single chain antibody (fv) 8A1 (SEQ ID NO: 46):

In an embodiment of the invention, an anti-IGF1R antibody or antigen-binding fragment thereof of the invention comprises single chain antibody (fv) 9A2 (SEQ ID NO: 47):

In an embodiment of the invention, an anti-IGF1R antibody or antigen-binding fragment thereof of the invention comprises single chain antibody (fv) 11 A4 (SEQ ID NO: 48):

In an embodiment of the invention, an anti-IGF1R antibody or antigen-binding fragment thereof of the invention comprises single chain antibody (fv) 7A4 (SEQ ID NO: 49):

In an embodiment of the invention, an anti-IGF1R antibody or antigen-binding fragment thereof of the invention comprises single chain antibody (fv) 11A1 (SEQ ID NO: 50):

In an embodiment of the invention, an anti-IGF1R antibody or antigen-binding fragment thereof of the invention comprises single chain antibody (fv) 7A6 (SEQ ID NO: 51)

In an embodiment of the invention, an anti-IGF1 R antibody or an antigen-binding fragment thereof (*e.g.*, a heavy chain or light chain immunoglobulin) of the invention comprises one or more complementarity determing regions (CDR) selected from the group consisting of:
sywmh (SEQ ID NO: 52);
einpsngrtnynekfkr (SEQ ID NO: 53);
grpdyygsskwyfdv (SEQ ID NO: 54);
rssqsivhsnvntyle (SEQ ID NO: 55);
kvsnrfs (SEQ ID NO: 56); and
fqgshvppt (SEQ ID NO: 57).

In an embodiment of the invention, an anti-IGF1 R antibody or an antigen-binding fragment thereof of the invention comprises a heavy chain immunoglobulin variable region selected from the group consisting of : and/or a light chain immunoglobulin variable region selected from the group consisting of: and

Disclosed are methods wherein a patient is administered an anti-insulin-like growth factor receptor-1 (IGF1R) antibody wherein the variable region of the antibody is linked to any immunoglobulin constant region. In an embodiment of the invention, the light chain variable region is linked to a κ chain constant region. In an embodiment of the invention, the heavy chain variable region is linked to a γ1, γ2, γ3 or γ4 chain constant region. Any of the immunoglobulin variable regions set forth herein, in embodiments of the invention, can be linked to any of the foregoing constant regions.

Furthermore, the scope of the present invention comprises any antibody or antibody fragment comprising one or more CDRs (3 light chain CDRs and/or 3 heavy chain CDRs) and/or framework regions of any of the light chain immunoglobulin or heavy chain immunoglobulins set forth herein as identified by any of the methods set forth in Chothia et al., J. Mol. Biol. 186:651-663 (1985); Novotny and Haber, Proc. Natl. Acad. Sci. USA 82:4592-4596 (1985) or Kabat, E. A. et al., Sequences of Proteins of Immunological Interest, National Institutes of Health, Bethesda, Md., (1987)).

In an embodiment of the invention, the term "monoclonal antibody," as used herein, refers to an antibody obtained from a population of substantially homogeneous antibodies, *i.e.*, the individual antibodies comprising the population are identical except for possible naturally occurring mutations that may be present in minor amounts. As mentioned above, the monoclonal antibodies to be used in accordance with the present invention may be made by the hybridoma method first described by Kohler, et al., (1975) Nature 256: 495.

In an embodiment of the invention, a bispecific or bifunctional antibody is an artificial hybrid antibody having two different heavy/light chain pairs and two different binding sites. Bispecific antibodies can be produced by a variety of methods including fusion of hybridomas or linking of Fab' fragments. See, *e.g.*, Songsivilai, et al., (1990) Clin. Exp. Immunol. 79: 315-321, Kostelny, et al., (1992) J Immunol. 148:1547- 1553. In addition, bispecific antibodies may be formed as "diabodies" (Holliger, et a/., (1993) PNAS USA 90:6444-6448) or as "Janusins" (Traunecker, et al., (1991) EMBO J. 10:3655-3659 and Traunecker, et al., (1992) Int. J. Cancer Suppl. 7:51-52).

In an embodiment of the invention, the term "fully human antibody" refers to an antibody which comprises human immunoglobulin protein sequences only. A fully human antibody may contain non-human, *e.g.*, murine, carbohydrate chains if produced in a mouse, in a mouse cell or in a hybridoma derived from a mouse cell. Similarly, "mouse antibody" refers to an antibody which comprises mouse immunoglobulin protein sequences only.

The present invention includes "chimeric antibodies"- an antibody which comprises a variable region of the present invention fused or chimerized with an antibody region (*e.g.*, constant region) from another, human or non-human species (*e.g.*, mouse, horse, rabbit, dog, cow, chicken). These antibodies may be used to modulate the expression or activity of IGF1R in the non-human species.

"Single-chain Fv" or "sFv" antibody fragments have the V_{H} and V_{L} domains of an antibody, wherein these domains are present in a single polypeptide chain. Generally, the sFv polypeptide further comprises a polypeptide linker between the V_{H} and V_{L} domains which enables the sFv to form the desired structure for antigen binding. Techniques described for the production of single chain antibodies (U.S. Patent Nos. 5,476,786; 5,132,405 and 4,946,778) can be adapted to produce anti-IGF1R-specific single chain antibodies. For a review of sFv see Pluckthun in The Pharmacology of Monoclonal Antibodies, vol. 113, Rosenburg and Moore eds. Springer-Verlag, N.Y., pp. 269-315 (1994).

In an embodiment of the invention, "disulfide stabilized Fv fragments" and "dsFv" refer to immunoglobulins comprising a variable heavy chain (V_{H}) and a variable light chain (V_{L}) which are linked by a disulfide bridge.

Antigen-binding fragments of antibodies within the scope of the present invention also include F(ab)₂ fragments which may be produced by enzymatic cleavage of an IgG by, for example, pepsin. Fab fragments may be produced by, for example, reduction of F(ab)₂ with dithiothreitol or mercaptoethylamine. A Fab fragment is a V_{L}-C_{L} chain appended to a V_{H}-C_{H1} chain by a disulfide bridge. A F(ab)₂ fragment is two Fab fragments which, in turn, are appended by two disulfide bridges. The Fab portion of an F(ab)₂ molecule includes a portion of the F_{c} region between which disulfide bridges are located.

An F_{V} fragment is a V_{L} or V_{H} region.

Depending on the amino acid sequences of the constant domain of their heavy chains, immunoglobulins can be assigned to different classes. There are at least five major classes of immunoglobulins: IgA, IgD, IgE, IgG and IgM, and several of these may be further divided into subclasses (isotypes), *e.g.* IgG-1, IgG-2, IgG-3 and IgG-4; IgA-1 and IgA-2. As discussed herein, any such antibody or antigen-binding fragment thereof is within the scope of the present invention.

The anti-IGF1 R antibodies and fragments of the invention may also be conjugated to a chemical moiety. The chemical moiety may be, *inter alia*, a polymer, a radionuclide or a cytotoxic factor. In an embodiment of the invention, the chemical moiety is a polymer which increases the half-life of the antibody molecule in the body of a subject. Suitable polymers include, but are not limited to, polyethylene glycol (PEG) (*e.g.*, PEG with a molecular weight of 2kDa, 5 kDa, 10 kDa, 12kDa, 20 kDa, 30kDa or 40kDa), dextran and monomethoxypolyethylene glycol (mPEG). Lee, et al., (1999) (Bioconj. Chem. 10:973-981) discloses PEG conjugated single-chain antibodies. Wen, et al., (2001) (Bioconj. Chem. 12:545-553) disclose conjugating antibodies with PEG which is attached to a radiometal chelator (diethylenetriaminpentaacetic acid (DTPA)).

The antibodies and antibody fragments of the invention may also be conjugated with labels such as ⁹⁹Tc,⁹⁰Y, ¹¹¹In, ³²P, ¹⁴C, ¹²⁵I, ³H, ¹³¹I, ¹¹C, ¹⁵O, ¹³N, ¹⁸F, ³⁵S, ⁵¹Cr, ⁵⁷To, ²²⁶Ra ⁶⁰Co, ⁵⁹Fe, ⁵⁷Se, ¹⁵²Eu, ⁶⁷Cu, ²¹⁷Ci, ²¹¹At, ²¹²Pb, ⁴⁷Sc, ¹⁰⁹Pd, ²³⁴Th, and ⁴⁰K, ¹⁵⁷Gd, ⁵⁵Mn, ⁵²Tr and ⁵⁶Fe.

The antibodies and antibody fragments of the invention may also be conjugated with fluorescent or chemilluminescent labels, including fluorophores such as rare earth chelates, fluorescein and its derivatives, rhodamine and its derivatives, isothiocyanate, phycoerythrin, phycocyanin, allophycocyanin, o-phthaladehyde, fluorescamine, ¹⁵²Eu, dansyl, umbelliferone, luciferin, luminal label, isoluminal label, an aromatic acridinium ester label, an imidazole label, an acridimium salt label, an oxalate ester label, an aequorin label, 2,3-dihydrophthalazinediones, biotin/avidin, spin labels and stable free radicals.

The antibodies and antibody fragments may also be conjugated to a cytotoxic factor such as diptheria toxin, *Pseudomonas aeruginosa* exotoxin A chain , ricin A chain, abrin A chain, modeccin A chain, alpha-sarcin, *Aleurites fordii* proteins and compounds (*e.g.*, fatty acids), dianthin proteins, *Phytoiacca* americana proteins PAPI, PAPII, and PAP-S, *momordica* charantia inhibitor, curcin, crotin, saponaria *officinalis* inhibitor, mitogellin, restrictocin, phenomycin, and enomycin.

Any method known in the art for conjugating the antibody molecules and fragments of the invention to the various moieties may be employed, including those methods described by Hunter, et al., (1962) Nature 144:945; David, et al., (1974) Biochemistry 13:1014; Pain, et al., (1981) J. Immunol. Meth. 40:219; and Nygren, J., (1982) Histochem. and Cytochem. 30:407. Methods for conjugating antibodies are conventional and very well known in the art.

### Generation of Antibodies

Any suitable method can be used to elicit an antibody with the desired biologic properties to inhibit IGF1R. It is desirable to prepare monoclonal antibodies (mAbs) from various mammalian hosts, such as mice, rodents, primates, humans, etc. Description of techniques for preparing such monoclonal antibodies may be found in, *e.g.*, Stites, et al. (eds.) BASIC AND CLINICAL IMMUNOLOGY (4th ed.) Lange Medical Publications, Los Altos, CA, and references cited therein; Harlow and Lane (1988) ANTIBODIES: A LABORATORY MANUAL CSH Press; Goding (1986) MONOCLONAL ANTIBODIES: PRINCIPLES AND PRACTICE (2d ed.) Academic Press, New York, NY. Thus, monoclonal antibodies may be obtained by a variety of techniques familiar to researchers skilled in the art. Typically, spleen cells from an animal immunized with a desired antigen are immortalized, commonly by fusion with a myeloma cell. See Kohler and Milstein (1976) Eur. J. Immunol. 6:511-519. Alternative methods of immortalization include transformation with Epstein Barr Virus, oncogenes, or retroviruses, or other methods known in the art. See, *e.g.*, Doyle, et al. (eds. 1994 and periodic supplements) CELL AND TISSUE CULTURE: LABORATORY PROCEDURES, John Wiley and Sons, New York, NY. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according, *e.g.*, to the general protocol outlined by Huse, et al. (1989) Science 246:1275-1281.

Other suitable techniques involve selection of libraries of antibodies in phage or similar vectors. See, *e.g.*, Huse et al., Science 246:1275-1281 (1989); and Ward et al., Nature 341:544-546 (1989). The polypeptides and antibodies of the present invention may be used with or without modification, including chimeric or humanized antibodies. Frequently, the polypeptides and antibodies will be labeled by joining, either covalently or non-covalently, a substance which provides for a detectable signal. A wide variety of labels and conjugation techniques are known and are reported extensively in both the scientific and patent literature. As discussed above, suitable labels include radionuclides, enzymes, substrates, cofactors, inhibitors, fluorescent moieties, chemiluminescent moieties, magnetic particles, and the like. Patents teaching the use of such labels include U.S. Patent Nos. 3,817,837; 3,850,752; 3,939,350; 3,996,345; 4,277,437; 4,275,149; and 4,366,241. Also, recombinant immunoglobulins may be produced, see Cabilly U.S. Patent No. 4,816,567; and Queen et al. (1989) Proc. Nat'l Acad. Sci. USA 86:10029-10033; or made in transgenic mice, see Mendez et al. (1997) Nature Genetics 15:146-156. Further methods for producing chimeric, humanized and human antibodies are well known in the art. See, *e.g.*, U.S. Pat. No. 5,530,101, issued to Queen et al, U.S. Pat. No. 5,225,539, issued to Winter et al, U. S. Pat. Nos. 4,816,397 issued to Boss et al.

Mammalian cell lines available as hosts for expression of antibodies of the invention are well known in the art and include many immortalized cell lines available from the American Type Culture Collection (ATCC). These include, inter alia, Chinese hamster ovary (CHO) cells, NSO, SP2 cells, HeLa cells, baby hamster kidney (BHK) cells, monkey kidney cells (COS), human hepatocellular carcinoma cells (*e.g.*, Hep G2), A549 cells, 3T3 cells, HEK-293 cells and a number of other cell lines. Mammalian host cells include human, mouse, rat, dog, monkey, pig, goat, bovine, horse and hamster cells. Cell lines of particular preference are selected through determining which cell lines have high expression levels. Other cell lines that may be used are insect cell lines, such as Sf9 cells, amphibian cells, bacterial cells, plant cells and fungal cells. When recombinant expression vectors encoding the heavy chain or antigen-binding portion thereof, the light chain and/or antigen-binding portion thereof are introduced into mammalian host cells, the antibodies are produced by culturing the host cells for a period of time sufficient to allow for expression of the antibody in the host cells or, more preferably, secretion of the antibody into the culture medium in which the host cells are grown.

Antibodies can be recovered from the culture medium using standard protein purification methods. Further, expression of antibodies of the invention (or other moieties therefrom) from production cell lines can be enhanced using a number of known techniques. For example, the glutamine synthetase gene expression system (the GS system) is a common approach for enhancing expression under certain conditions. The GS system is discussed in whole or part in connection with European Patent Nos. 0 216 846, 0 256 055, and 0 323 997 and European Patent Application No. 89303964.4.

It is likely that antibodies expressed by different cell lines or in transgenic animals will have different glycosylation from each other. However, all antibodies encoded by the nucleic acid molecules provided herein, or comprising the amino acid sequences provided herein are part of the instant invention, regardless of the glycosylation of the antibodies.

A convenient plasmid system useful for producing an anti-IGF1 R antibody or antigen-binding fragment thereof is set forth in published U.S. application no. US2005/0176099 (see also WO2005/47512).

### Further Chemotherapeutics

The present invention provides combinations for treating or preventing any of the medical disorders set forth herein by administering a therapeutically acceptable dosage or amount of an anti-IGF1R antibody or antigen-binding fragment thereof of the invention, *e.g.*, as set forth herein, in association with a further chemotherapeutic agent (*e.g.*, an anti-cancer chemotherapeutic agent, and/or an anti-emetic chemotherapeutic agent). For example, further chemotherapeutic agents include erlotinib, dasatanib, nilotinib, decatanib, panitumumab, amrubicin, oregovomab, Lep-etu ("liposome entrapped paclitaxel-Easy to Use"), nolatrexed, azd2171, batabulin, ofatumumab, zanolimumab, edotecarin, tetrandrine, rubitecan, tesmilifene, oblimersen, ticilimumab, ipilimumab, gossypol, Bio 111, 131-I-TM-601, ALT-110, BIO 140, CC 8490, cilengitide, gimatecan, IL13-PE38QQR, INO 1001, IPdR, KRX-0402, lucanthone, LY 317615, neuradiab, vitespan, Rta 744, Sdx 102, talampanel, atrasentan, Xr 311, everolimus, trabectedin, abraxane, TLK 286, AV-299, DN-101, pazopanib, GSK690693, RTA 744, ON 0910.Na, AZD 6244 (ARRY-142886), AMN-107, TKI-258, GSK461364, AZD 1152, enzastaurin, vandetanib, ARQ-197, MK-0457, MLN8054, PHA-739358, R-763 or AT-9263.

Abraxane is an injectable suspension of paclitaxel protein-bound particles comprising an albumin-bound form of paclitaxel with a mean particle size of approximately 130 nanometers. Abraxane is supplied as a white to yellow, sterile, lyophilized powder for reconstitution with 20 mL of 0.9% Sodium Chloride Injection, USP prior to intravenous infusion. Each single-use vial contains 100 mg of paclitaxel and approximately 900 mg of human albumin. Each milliliter (mL) of reconstituted suspension contains 5 mg paclitaxel. Abraxane is free of solvents and is free of cremophor (polyoxyethylated castor oil).

In an embodiment of the invention, an antibody or antigen-binding fragment thereof of the invention is provided in association with romidepsin (FK-228; 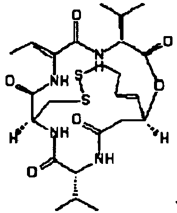 ADS-100380, 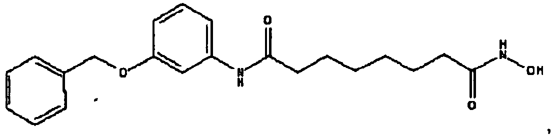 CG-781 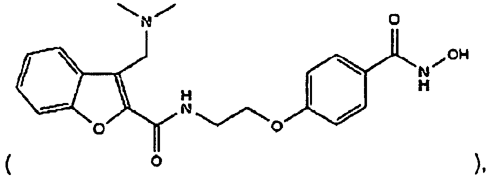 CG-1521 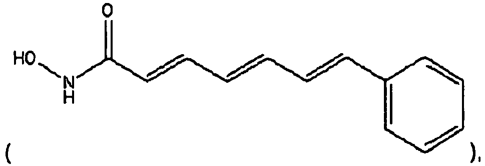 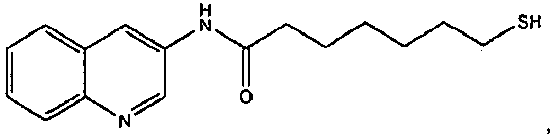 SB-556629 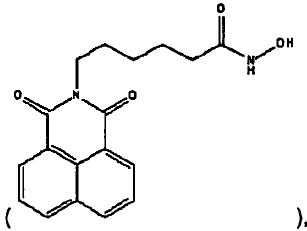 chlamydocin 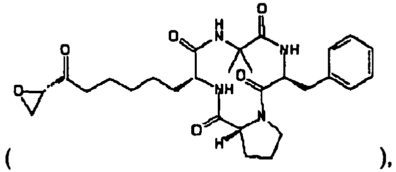 JNJ-16241199 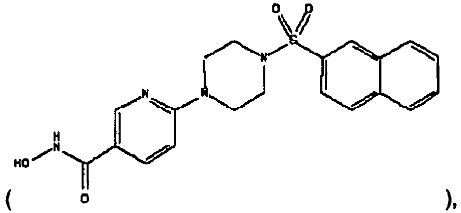 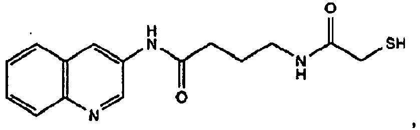 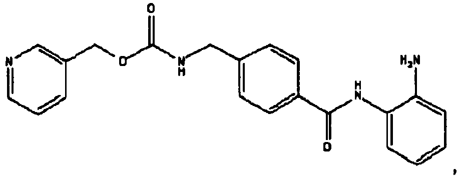 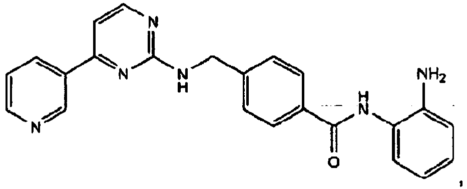 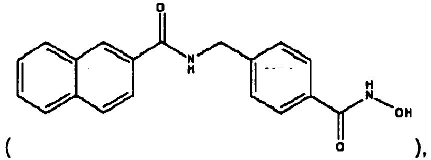 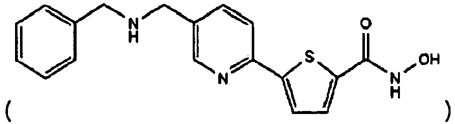 or vorinostat (SAHA;

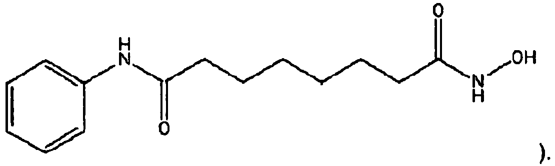

In an embodiment of the invention, an antibody or antigen-binding fragment thereof of the invention is provided in association with etoposide (VP-16;

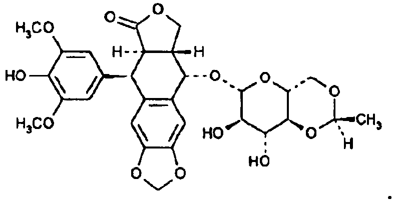

In an embodiment of the invention, an antibody or antigen-binding fragment thereof of the invention is provided in association with gemcitabine

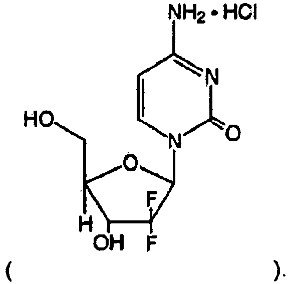

In an embodiment of the invention, an antibody or antigen-binding fragment thereof of the invention is provided in association with any compound disclosed in published U.S. patent application no. U.S. 2004/0209878A1 (*e.g.*, comprising a core structure represented by 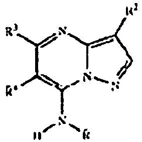 ) or doxorubicin ( 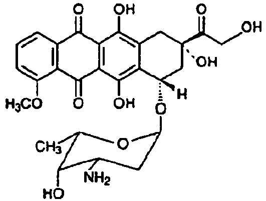 ) including Caelyx or Doxil® (doxorubicin HCl liposome injection; Ortho Biotech Products L.P; Raritan, NJ). Doxil® comprises doxorubicin in STEALTH® liposome carriers which are composed of N-(carbonyl-methoxypolyethylene glycol 2000)-1,2-distearoyl-sn-glycero-3-phosphoethanolamine sodium salt (MPEG-DSPE); fully hydrogenated soy phosphatidylcholine (HSPC), and cholesterol.

In an embodiment of the invention, an antibody or antigen-binding fragment thereof of the invention is provided in association with 5'-deoxy-5-fluorouridine ( 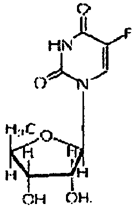 ).

In an embodiment of the invention, an antibody or antigen-binding fragment thereof of the invention is provided in association with vincristine ( 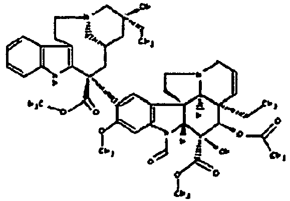 )

In an embodiment of the invention, an antibody or antigen-binding fragment thereof of the invention is provided in association with temozolomide 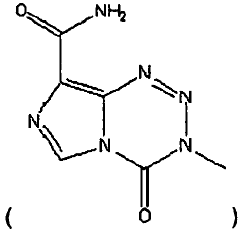 any CDK inhibitor such as ZK-304709, Seliciclib (R-roscovitine) 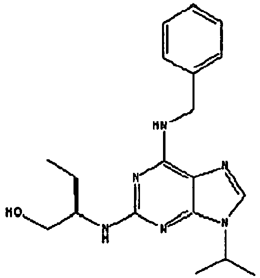 );any MEK inhibitor such as PD0325901 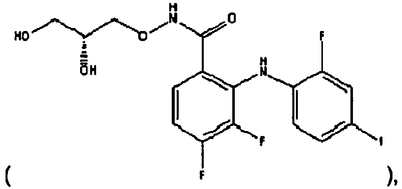 AZD-6244 ; capecitabine (5'-deoxy-5-fluoro-N-[(pentyloxy) carbonyl]-cytidine); or L-Glutamic acid, N-[4-[2-(2-amino-4,7-dihydro-4-oxo-1H -pyrrolo[2,3-d]pyrimidin-5-yl)ethyl]benzoyl]-disodium salt, heptahydrate ( 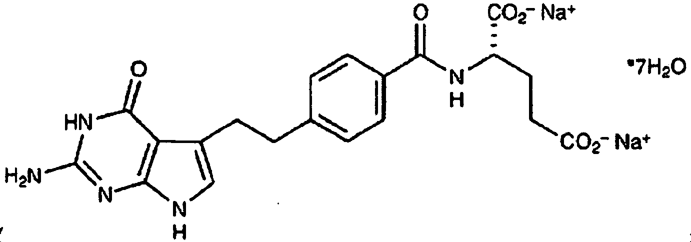 Pemetrexed disodium heptahydrate).

In an embodiment of the invention, an antibody or antigen-binding fragment thereof of the invention is provided in association with camptothecin ( 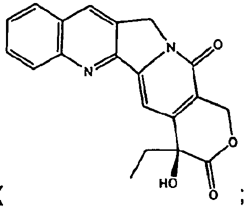 Stork et al., J. Am. Chem. Soc. 93(16): 4074-4075 (1971 ); Beisler et al., J. Med. Chem. 14(11): 1116-1117 (1962)), irinotecan ( 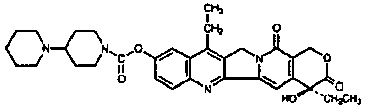 ; sold as Camptosar®; Pharmacia & Upjohn Co.; Kalamazoo, MI); a combination of irinotecan, 5-fluorouracil and leucovorin; or PEG-labeled irinotecan.

In an embodiment of the invention, an antibody or antigen-binding fragment thereof of the invention is provided in association with the FOLFOX regimen (oxaliplatin 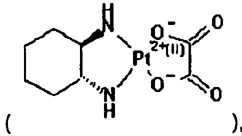 together with infusional fluorouracil 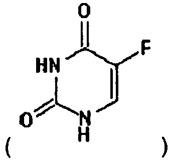 and folinic acid 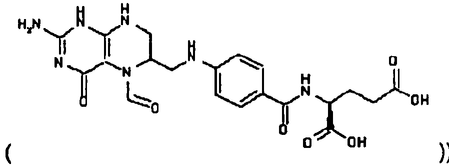 ) (Chaouche et al., Am. J. Clin. Oncol. 23(3):288-289 (2000); de Gramont et al., J. Clin. Oncol. 18(16):2938-2947 (2000)).

In an embodiment of the invention, an antibody or antigen-binding fragment thereof of the invention is provided in association with an antiestrogen such as 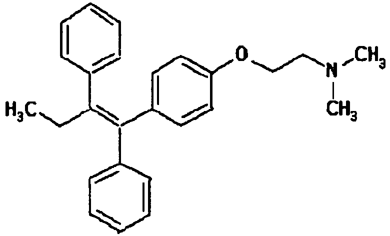 (tamoxifen; sold as Nolvadex® by AstraZeneca Pharmaceuticals LP; Wilmington, DE) or 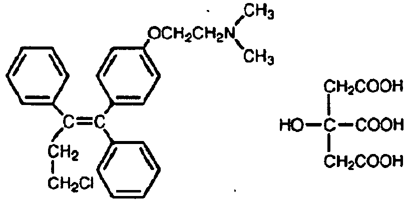 (toremifene citrate; sold as Fareston® by Shire US, Inc.; Florence, KY).

In an embodiment of the invention, an antibody or antigen-binding fragment thereof of the invention is provided in association with an aromatase inhibitor such as 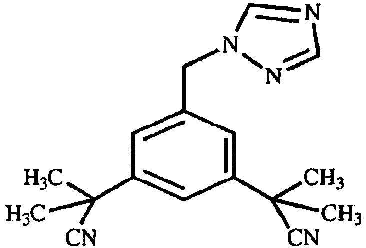 (anastrazole; sold as Arimidex® by AstraZeneca Pharmaceuticals LP; Wilmington, DE), 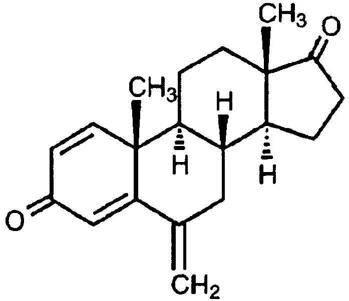 (exemestane; sold as Aromasin® by Pharmacia Corporation; Kalamazoo, MI) or 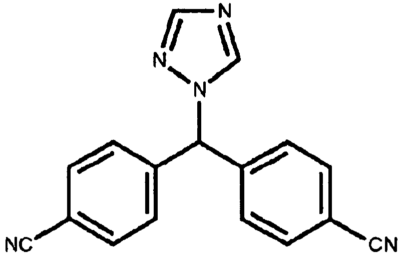 (letrozole; sold as Femara® by Novartis Pharmaceuticals Corporation; East Hanover, NJ).

In an embodiment of the invention, an antibody or antigen-binding fragment thereof of the invention is provided in association with an estrogen such as DES(diethylstilbestrol), 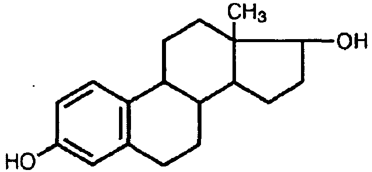 (estradiol; sold as Estrol® by Warner Chilcott, Inc.; Rockaway, NJ) or conjugated estrogens (sold as Premarin® by Wyeth Pharmaceuticals Inc. ; Philadelphia, PA).

In an embodiment of the invention, an antibody or antigen-binding fragment thereof of the invention is provided in association with anti-angiogenesis agents including bevacizumab (Avastin™; Genentech; San Francisco, CA), the anti-VEGFR-2 antibody IMC-1C11, other VEGFR inhibitors such as: CHIR-258 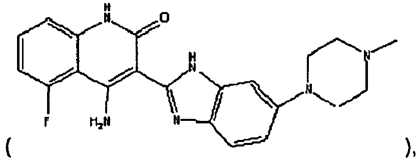 any of the inhibitors set forth in WO2004/13145 (*e.g.*, comprising the core structural formula: 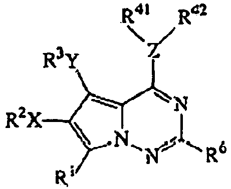 ), WO2004/09542 (*e.g.*,comprising the core structural formula: 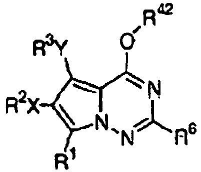 ), WO00/71129 (*e.g.*, comprising the core structural formula: 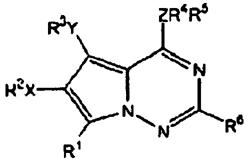 ),WO2004/09601 (*e.g.*, comprising the core structural formula: 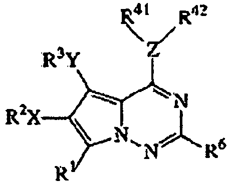 ), WO2004/01059 (*e.g.*, comprising the core structural formula: 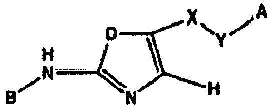 ) WO01/29025 (*e.g.*, comprising the core structural formula: 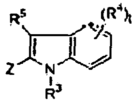 ), WO02/32861 (*e.g.*, comprising the core structural formula: 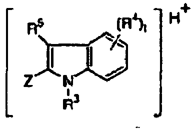 A ) or set forth in WO03/88900 (*e.g.*, comprising the core structural formula 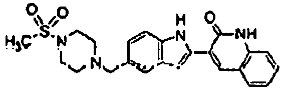 ); 3-[5-(methylsulfonylpiperadinemethyl)-indolyl]-quinolone; Vatalanib ( 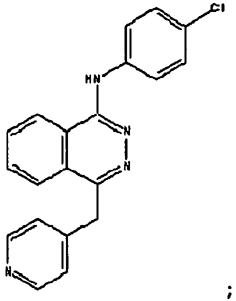 PTK/ZK; CPG-79787; ZK-222584), AG-013736 ( 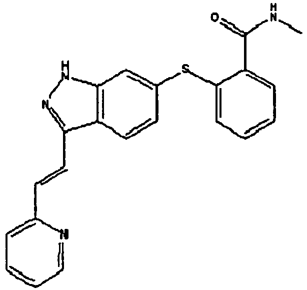 ); and the VEGF trap (AVE-0005), a soluble decoy receptor comprising portions of VEGF receptors 1 and 2.

In an embodiment of the invention, an antibody or antigen-binding fragment thereof of the invention is provided in association with a LHRH (Lutenizing hormone-releasing hormone) agonist such as the acetate salt of [D-Ser(Bu t) 6 ,Azgly 10] (pyro-Glu-His-Trp-Ser-Tyr-D-Ser(Bu t)-Leu-Arg-Pro-Azgly-NH₂acetate[C₅₉H₈₄N₁₈O₁₄·(C₂H₄O₂)ₓ where x = 1 to 2.4]; 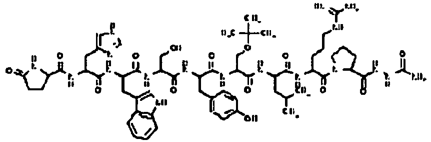 (goserelin acetate; sold as Zoladex® by AstraZeneca UK Limited; Macclesfield, England), 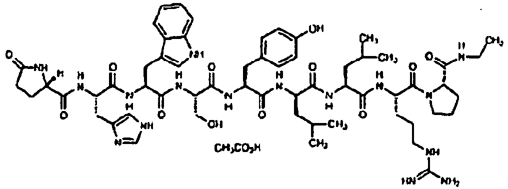 (leuprolide acetate; sold as Eligard® by Sanofi-Synthelabo Inc.; New York, NY) or 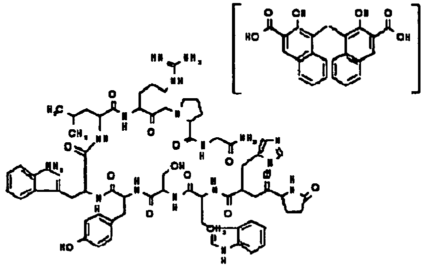 (triptorelin pamoate; sold as Trelstar® by Pharmacia Company, Kalamazoo, MI).

In an embodiment of the invention, an antibody or antigen-binding fragment thereof of the invention is provided in association with sunitinib or sunitinib malate

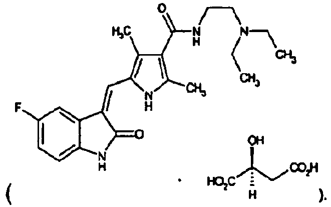

In an embodiment of the invention, an antibody or antigen-binding fragment thereof of the invention is provided in association with a progestational agent such as 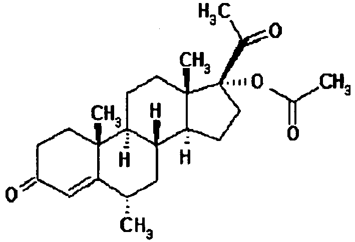 (medroxyprogesterone acetate; sold as Provera® by Pharmacia & Upjohn Co.; Kalamazoo, MI), 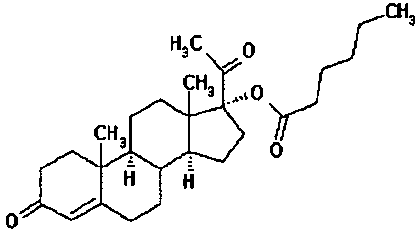 (hydroxyprogesterone caproate; 17-((1-Oxohexyl)oxy)pregn-4-ene-3,20-dione; ), megestrol acetate or progestins.

In an embodiment of the invention, an antibody or antigen-binding fragment thereof of the invention is provided in association with selective estrogen receptor modulator (SERM) such as 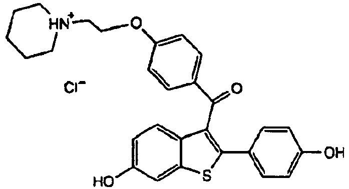 (raloxifene; sold as Evista® by Eli Lilly and Company; Indianapolis, IN).

In an embodiment of the invention, an antibody or antigen-binding fragment thereof of the invention is provided in association with an anti-androgen including, but not limited to: 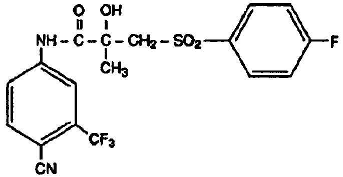 (bicalutamide; sold at CASODEX ® by AstraZeneca Pharmaceuticals LP; Wilmington, DE); 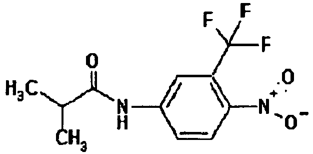 (flutamide; 2-methyl-N-[4-nitro-3 (trifluoromethyl) phenyl] propanamide; sold as Eulexin® by Schering Corporation; Kenilworth, NJ); 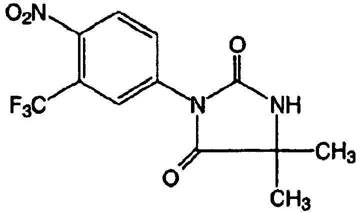 (nilutamide; sold as Nilandron® by Aventis Pharmaceuticals Inc.; Kansas City, MO) and 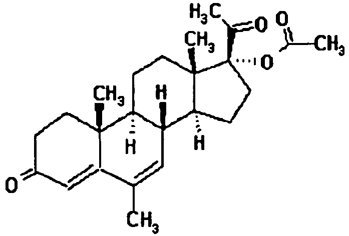 (Megestrol acetate; sold as Megace® by Bristol-Myers Squibb).

In an embodiment of the invention, an antibody or antigen-binding fragment thereof of the invention is provided in association with one or more inhibitors which antagonize the action of the EGF Receptor or HER2, including, but not limited to, CP-724714 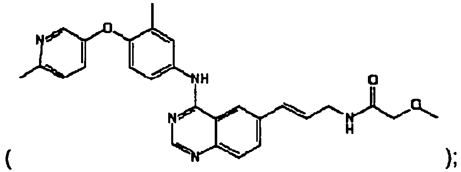 ; TAK-165 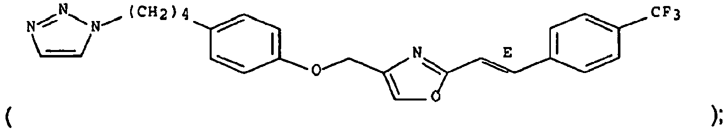 HKI-272 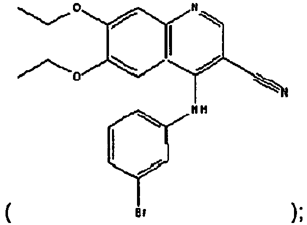 OSI-774 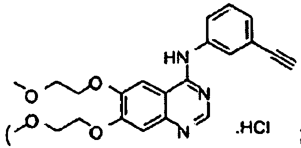 erlotinib, Hidalgo et al., J. Clin. Oncol. 19(13): 3267-3279 (2001)), Lapatanib ( 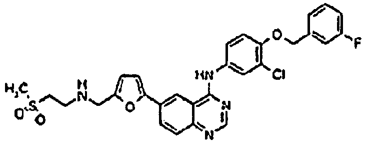 ; GW2016; Rusnak et al., Molecular Cancer Therapeutics 1:85-94 (2001); N-{3-Chloro-4-[(3-fluorobenzyl)oxy]phenyl}-6-[5-({[2-(methylsulfonyl)ethyl]amino}methyl)-2-furyl]-4-quinazolinamine; PCT Application No. WO99/35146), Canertinib (CI-1033; 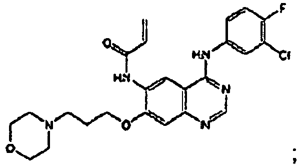 Erlichman et al., Cancer Res. 61(2):739-48 (2001 ); Smaill et al., J. Med. Chem. 43(7):1380-97 (2000)), ABX-EGF antibody (Abgenix, Inc.; Freemont, CA; Yang et al., Cancer Res. 59(6):1236-43 (1999); Yang et al., Crit Rev Oncol Hematol. 38(1):17-23 (2001)), erbitux (U.S. Patent No. 6,217,866; IMC-C225, cetuximab; Imclone; New York, NY), EKB-569 ( 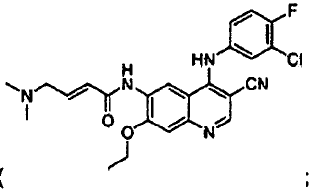 Wissner et al., J. Med. Chem. 46(1): 49-63 (2003 )), PKI-166 ( 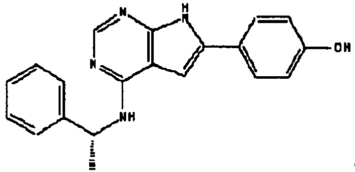 ;CGP-75166), GW-572016, any anti-EGFR antibody and any anti-HER2 antibody.

In an embodiment of the invention, an antibody or antigen-binding fragment thereof of the invention is provided in association with: 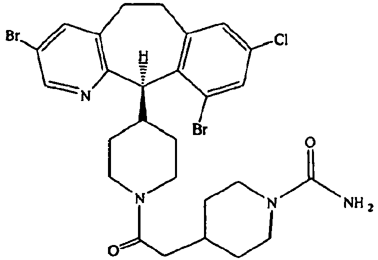 (Ionafarnib; Sarasar™; Schering-Plough; Kenilworth, NJ). In another embodiment, one of the following FPT inhibitors is provided in association with an antibody or antigen-binding fragment thereof of the invention: 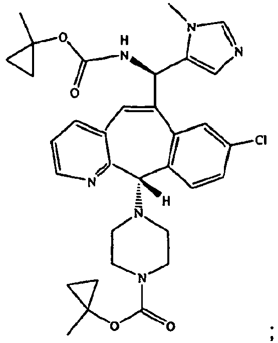 or

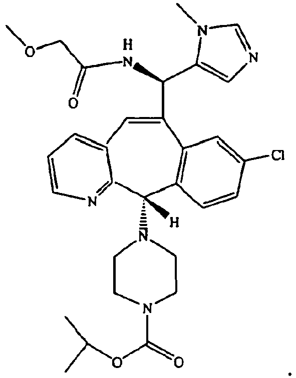

Other FPT inhibitors, that can be provided in association with an antibody or antigen-binding fragment thereof of the invention, include BMS-214662 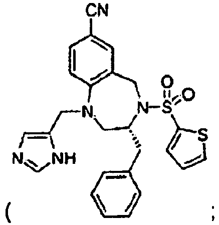 Hunt et al., J. Med. Chem. 43(20):3587-95 (2000 ); Dancey et al., Curr. Pharm. Des. 8:2259-2267 (2002); (R)-7-cyano-2,3,4,5-tetrahydro-1-(1H-imidazol-4-ylmethyl)-3-(phenylmethyl)-4-(2-thienylsulfonyl)-1H-1,4-benzodiazepine)) and R155777 (tipifarnib; Garner et al., Drug Metab. Dispos. 30(7):823-30 (2002); Dancey et al., Curr. Pharm. Des. 8:2259-2267 (2002); (B)-6-[amino(4-chlorophenyl)(1-methyl-1H-imidazol-5-yl)-methyl]-4-(3-chlorophenyl)-1-methyl-2(1H)-quinolinone]; 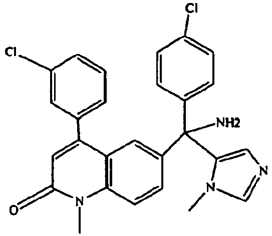 sold as Zarnestra™; Johnson & Johnson; New Brunswick, NJ).

In an embodiment of the invention, an antibody or antigen-binding fragment thereof of the invention is provided in association with 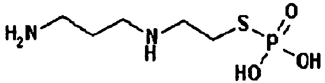 (Amifostine); 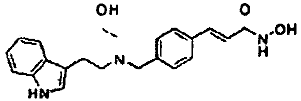 (NVP-LAQ824; Atadja et al., Cancer Research 64: 689-695 (2004)), 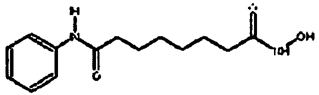 (suberoyl analide hydroxamic acid), 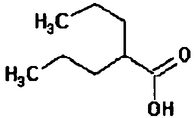 (Valproic acid; Michaelis et al., Mol. Pharmacol. 65:520-527 (2004)), 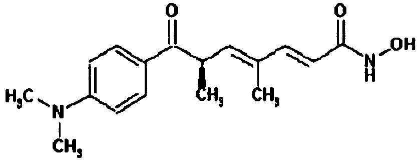 (trichostatin A), 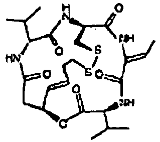 (FK-228; Furumai et al., Cancer Research 62: 4916-4921 (2002)), 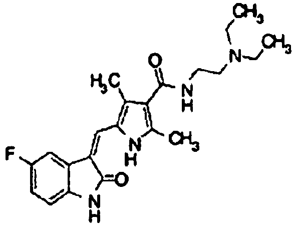 (SU11248; Mendel et al., Clin. Cancer Res. 9(1):327-37 (2003)), 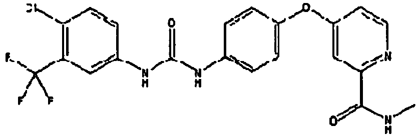 (BAY43-9006; sorafenib), 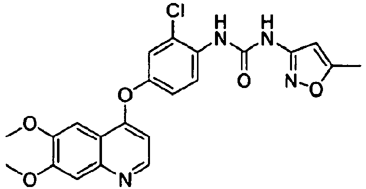 (KRN951), 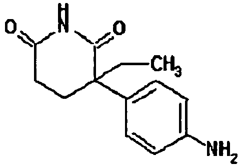 (Aminoglutethimide); 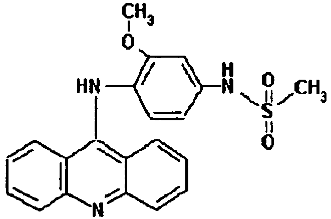 (Amsacrine); 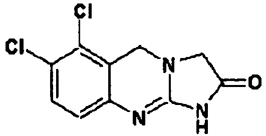 (Anagrelide); 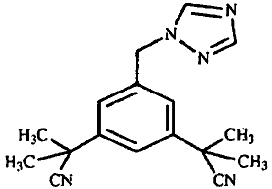 (Anastrozole; sold as Arimidex by AstraZeneca Pharmaceuticals LP; Wilmington, DE); Asparaginase; Bacillus Calmette-Guerin (BCG) vaccine (Garrido et al., Cytobios. 90(360):47-65 (1997)); 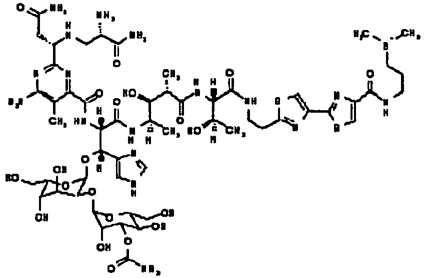 (Bleomycin); 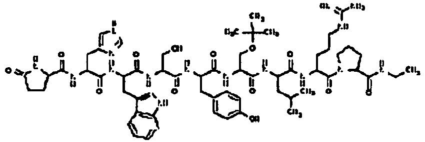 (Buserelin); 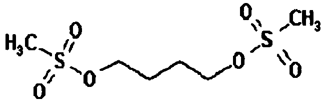 (Busulfan; 1,4-butanediol, dimethanesulfonate; sold as Busulfex® by ESP Pharma, Inc.; Edison, New Jersey); 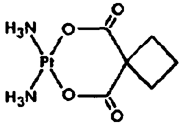 (Carboplatin; sold as Paraplatin® by Bristol-Myers Squibb; Princeton, NJ); 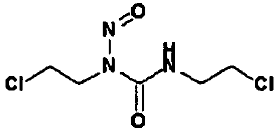 (Carmustine); 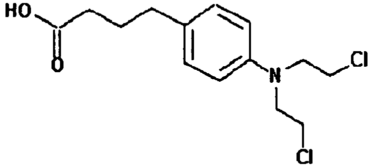 (Chlorambucil); 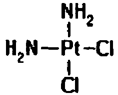 (Cisplatin); 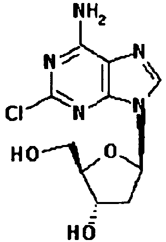 (Cladribine); 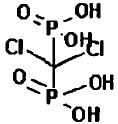 (Clodronate); 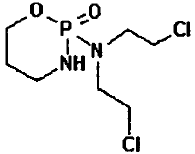 OH₂ (Cyclophosphamide); 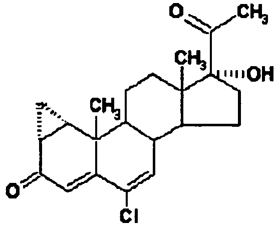 (Cyproterone); 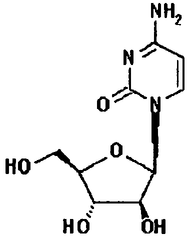 (Cytarabine); 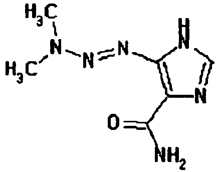 (Dacarbazine); 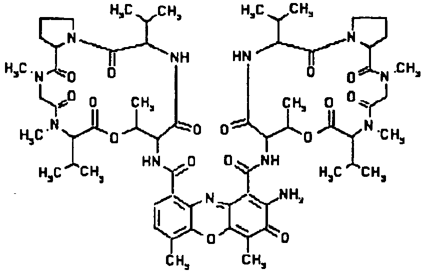 (Dactinomycin); 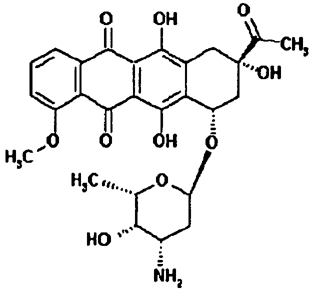 (Daunorubicin); 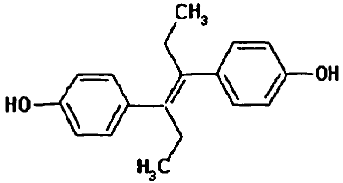 (Diethylstilbestrol); 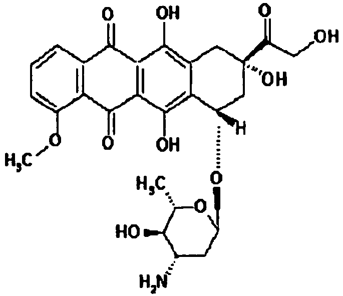 (Epirubicin); 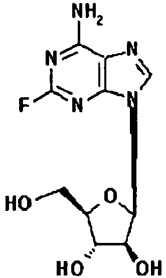 (Fludarabine); 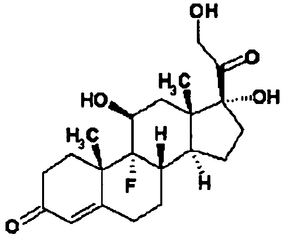 (Fludrocortisone); 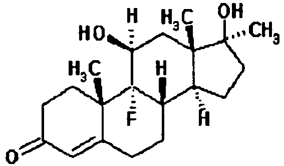 (Fluoxymesterone); 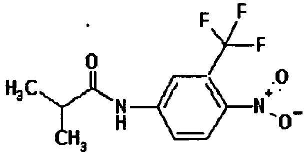 (Flutamide); 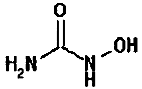 (Hydroxyurea); 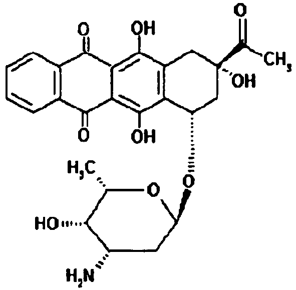 (Idarubicin); 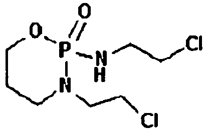 (Ifosfamide); 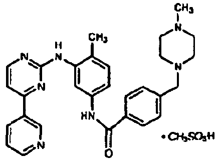 (Imatinib; sold as Gleevec® by Novartis Pharmaceuticals Corporation; East Hanover, NJ); 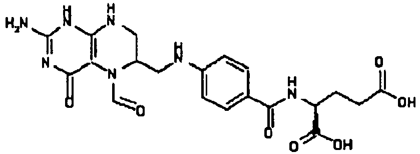 (Leucovorin); 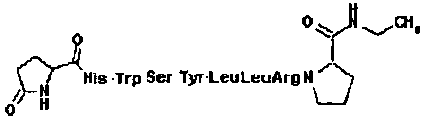 (Leuprolide); 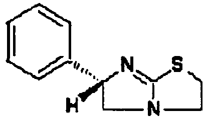 (Levamisole); 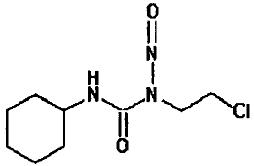 (Lomustine); 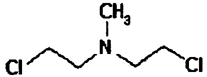 (Mechlorethamine); 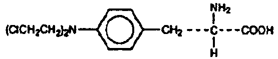 (Melphalan; sold as Alkeran® by Celgene Corporation; Warren, NJ); 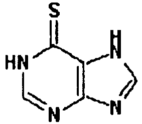 (Mercaptopurine); 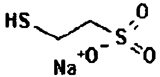 (Mesna); 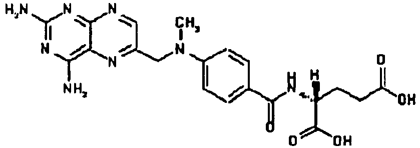 (Methotrexate); 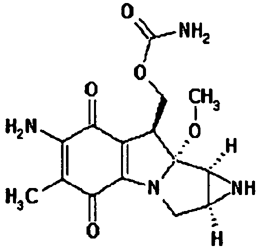 (Mitomycin); 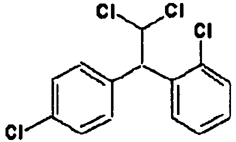 (Mitotane); 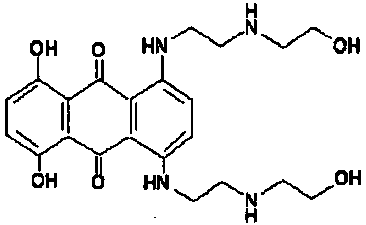 (Mitoxantrone); 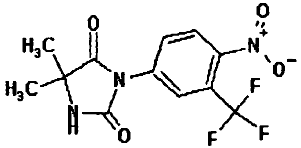 (Nilutamide); octreotide ( 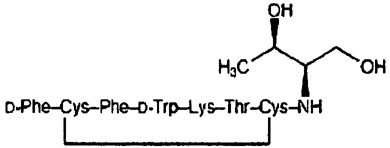 ; Katz et al., Clin Pharm. 8(4):255-73 (1989); sold as Sandostatin LAR® Depot; Novartis Pharm. Corp; E. Hanover, NJ); edotreotide (yttrium-90 labeled or unlabeled); oxaliplatin ( 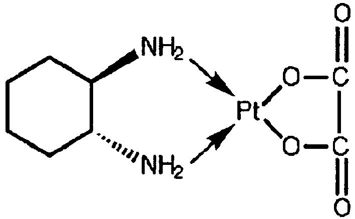 ; sold as Eloxatin™ by Sanofi-Synthelabo Inc.; New York, NY); 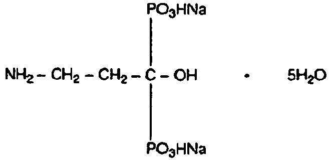 (Pamidronate; sold as Aredia® by Novartis Pharmaceuticals Corporation; East Hanover, NJ); 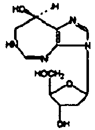 (Pentostatin; sold as Nipent® by Supergen; Dublin, CA); 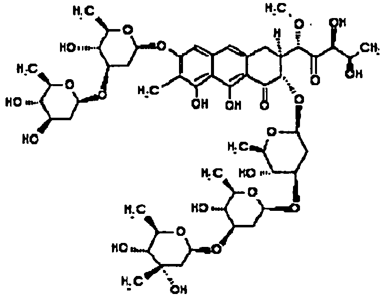 (Plicamycin); 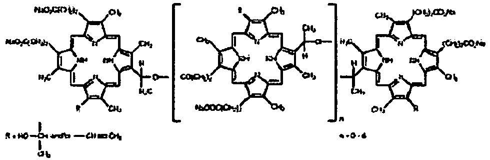 (Porfimer; sold as Photofrin® by Axcan Scandipharm Inc.; Birmingham, AL); 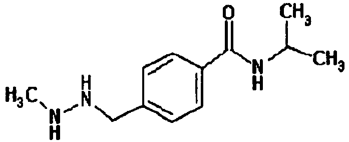 (Procarbazine); 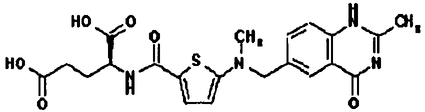 (Raltitrexed); Rituximab (sold as Rituxan® by Genentech, Inc.; South San Francisco, CA); 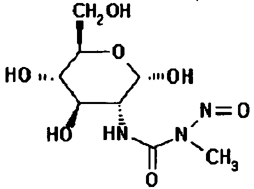 (Streptozocin); 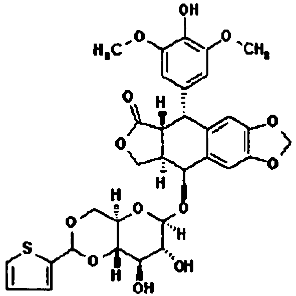 (Teniposide); 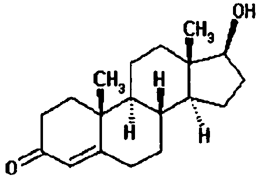 (Testosterone); 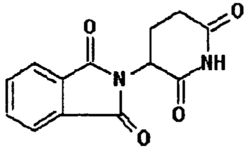 (Thalidomide); 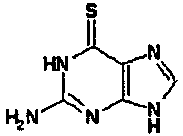 (Thioguanine); 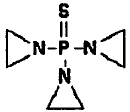 (Thiotepa); 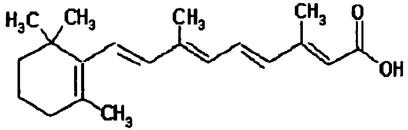 (Tretinoin); 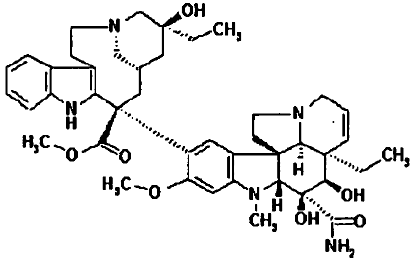 (Vindesine) or 13-cis-retinoic acid

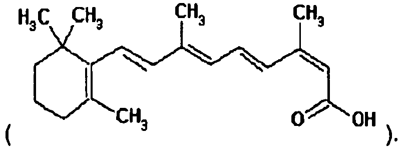

In an embodiment of the invention, an antibody or antigen-binding fragment thereof of the invention is provided in association with one or more of any of: phenylalanine mustard, uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin, diftitox, gefitinib, bortezimib, paclitaxel, docetaxel, epithilone B, BMS-247550 (see *e.g.*, Lee et al., Clin. Cancer Res. 7:1429-1437 (2001)), BMS-310705, droloxifene (3-hydroxytamoxifen), 4-hydroxytamoxifen, pipendoxifene, ERA-923, arzoxifene, fulvestrant, acolbifene, lasofoxifene (CP-336156), idoxifene, TSE-424, HMR-3339, ZK186619, topotecan, PTK787/ZK 222584 (Thomas et al., Semin Oncol. 30(3 Suppl 6):32-8 (2003)), the humanized anti-VEGF antibody Bevacizumab, VX-745 (Haddad, Curr Opin. Investig. Drugs 2(8):1070-6 (2001)), PD 184352 (Sebolt-Leopold, et al. Nature Med. 5: 810-816 (1999)), any mTOR inhibitor, rapamycin 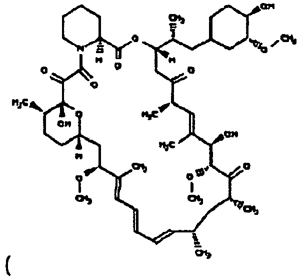 ; sirolimus), 40-O-(2-hydroxyethyl)-rapamycin, CCI-779 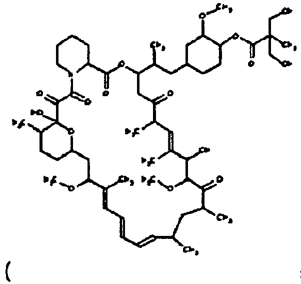 temsirolimus; Sehgal et al., Med. Res. Rev., 14:1-22 (1994); Elit, Curr. Opin. Investig. Drugs 3(8):1249-53 (2002)), AP-23573 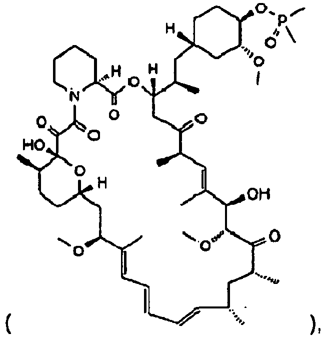 RAD001 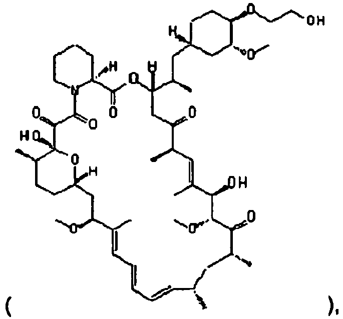 ABT-578 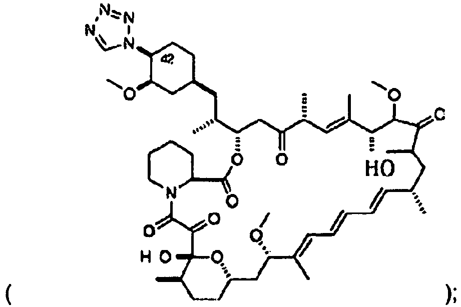 BC-210 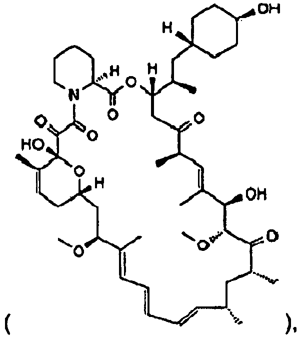 LY294002, LY292223, LY292696, LY293684, LY293646 (Vlahos et al., J. Biol. Chem. 269(7): 5241-5248 (1994)), wortmannin, BAY-43-9006, (Wilhelm et al., Curr. Pharm. Des. 8:2255-2257 (2002)), ZM336372, L-779,450, any Raf inhibitor disclosed in Lowinger et al., Curr. Pharm Des. 8:2269-2278 (2002); flavopiridol (L86-8275/HMR 1275; Senderowicz, Oncogene 19(56): 6600-6606 (2000)) or UCN-01 (7-hydroxy staurosporine; Senderowicz, Oncogene 19(56): 6600-6606 (2000)).

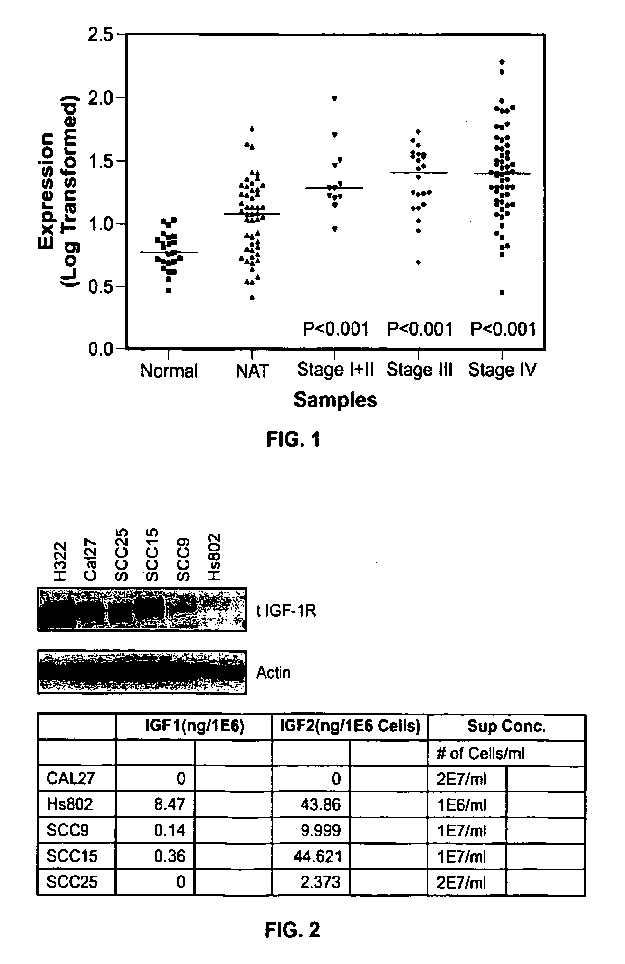

In an embodiment of the invention, an antibody or antigen-binding fragment thereof of the invention is provided in association with one or more of any of the compounds set forth in U.S. Patent 5,656,655, which discloses styryl substituted heteroaryl EGFR inhibitors; in U.S. Patent 5,646,153 which discloses bis mono and/or bicyclic aryl heteroaryl carbocyclic and heterocarbocyclic EGFR and PDGFR inhibitors; in U.S. Patent 5,679,683 which discloses tricyclic pyrimidine compounds that inhibit the EGFR; in U.S. Patent 5,616,582 which discloses quinazoline derivatives that have receptor tyrosine kinase inhibitory activity;in Fry et al., Science 265 1093-1095 (1994) which discloses a compound having a structure that inhibits EGFR (see Figure 1 of Fry et al.); in U.S. Patent 5,196,446 which discloses heteroarylethenediyl or heteroarylethenediylaryl compounds that inhibit EGFR; in Panek, et al., Journal of Pharmacology and Experimental Therapeutics 283: 1433-1444 (1997) which disclose a compound identified as PD166285 that inhibits the EGFR, PDGFR, and FGFR families of receptors-PD166285 is identified as 6- (2,6- dichlorophenyl)-2-(4-(2-diethylaminoethoxy)phenylarnino)-8-methyl-8H-pyrido(2,3- d)pyrimidin-7-one.

In an embodiment of the invention, an antibody or antigen-binding fragment thereof of the invention is provided in association with one or more of any of: pegylated or unpegylated interferon alfa-2a, pegylated or unpegylated interferon alfa-2b, pegylated or unpegylated interferon alfa-2c, pegylated or unpegylated interferon alfa n-1, pegylated or unpegylated interferon alfa n-3 and pegylated, unpegylated consensus interferon or albumin-interferon-alpha.

The term "interferon alpha" as used herein means the family of highly homologous species-specific proteins that inhibit cellular proliferation and modulate immune response. Typical suitable interferon-alphas include, but are not limited to, recombinant interferon alpha-2b, recombinant interferon alpha-2a, recombinant interferon alpha-2c, alpha 2 interferon, interferon alpha-n1 (INS), a purified blend of natural alpha interferons, a consensus alpha interferon such as those described in U.S. Pat. Nos. 4, 897,471 and 4,695,623 (especially Examples 7, 8 or 9 thereof), or interferon alpha-n3, a mixture of natural alpha interferons.

Interferon alfa-2a is sold as ROFERON-A® by Hoffmann-La Roche (Nutley, N.J).

Interferon alfa-2b is sold as INTRON-A® by Schering Corporation (Kenilworth, NJ). The manufacture of interferon alpha 2b is described, for example, in U.S. Pat. No. 4,530,901.

Interferon alfa-n3 is a mixture of natural interferons sold as ALFERON N INJECTION® by Hemispherx Biopharma, Inc. (Philadelphia, PA).

Interferon alfa-n1 (INS) is a mixture of natural interferons sold as WELLFERON® by Glaxo-Smith-Kline (Research Triangle Park, NC).

Consensus interferon is sold as INFERGEN® by Intermune, Inc. (Brisbane, CA).

Interferon alfa-2c is sold as BEROFOR® by Boehringer Ingelheim Pharmaceutical, Inc. (Ridgefield, CT).

A purified blend of natural interferons is sold as SUMIFERON® by Sumitomo; Tokyo, Japan.

The term "pegylated interferon alpha" as used herein means polyethylene glycol modified conjugates of interferon alpha, preferably interferon alpha-2a and alpha-2b. The preferred polyethylene-glycol-interferon alpha-2b conjugate is PEG 12000-interferon alpha-2b. The phrases "12,000 molecular weight polyethylene glycol conjugated interferon alpha" and "PEG 12000-IFN alpha" as used herein include conjugates such as are prepared according to the methods of International Application No. WO 95/13090 and EP1039922and containing urethane linkages between the interferon alpha-2a or -2b amino groups and polyethylene glycol having an average molecular weight of 12000. The pegylated inteferon alpha, PEG 12000-IFN-alpha-2b is available from Schering-Plough Research Institute, Kenilworth, N.J.

The preferred PEG 12000-interferon alpha-2b can be prepared by attaching a PEG polymer to the histidine residue in the interferon alpha-2b molecule. A single PEG 12000 molecule can be conjugated to free amino groups on an IFN alpha-2b molecule via a urethane linkage. This conjugate is characterized by the molecular weight of PEG 12000 attached. The PEG 12000-IFN alpha-2b conjugate can be formulated as a lyophilized powder for injection.

Pegylated interferon alfa-2b is sold as PEG-INTRON® by Schering Corporation (Kenilworth, NJ).

Pegylated interferon-alfa-2a is sold as PEGASYS® by Hoffmann-La Roche (Nutley, N.J).

Other interferon alpha conjugates can be prepared by coupling an interferon alpha to a water-soluble polymer. A non-limiting list of such polymers includes other polyalkylene oxide homopolymers such as polypropylene glycols, polyoxyethylenated polyols, copolymers thereof and block copolymers thereof. As an alternative to polyalkylene oxide-based polymers, effectively non-antigenic materials such as dextran, polyvinylpyrrolidones, polyacrylamides, polyvinyl alcohols, carbohydrate- based polymers and the like can be used. Such interferon alpha-polymer conjugates are described, for example, in U.S. Pat. No. 4,766,106, U.S. Pat. No. 4,917, 888, European Patent Application No. 0 236 987 or 0 593 868 or International Publication No. WO 95/13090. The preferred PEG12000-IFN alfa 2b can be prepared by attaching in a PEG polymer to a histidine residue in the interferon alfa-2b molecule.

Pharmaceutical compositions of pegylated interferon alpha suitable for parenteral administration can be formulated with a suitable buffer, *e.g.*, Tris-HCl, acetate or phosphate such as dibasic sodium phosphate/monobasic sodium phosphate buffer, and pharmaceutically acceptable excipients (*e.g.*, sucrose), carriers (e.g. human plasma albumin), toxicity agents (*e.g.*, NaCl), preservatives (*e.g.*, thimerosol, cresol or benzyl alcohol), and surfactants (*e.g.*, tween or polysorbates) in sterile water for injection. The pegylated interferon alpha can be stored as lyophilized powder under refrigeration at 2°-8°C. The reconstituted aqueous solutions are stable when stored between 2° and 8°C and used within 24 hours of reconstitution. See for example U.S. Pat. Nos, 4,492,537; 5,762,923 and 5, 766,582. The reconstituted aqueous solutions may also be stored in prefilled, multi-dose syringes such as those useful for delivery of drugs such as insulin. Typical, suitable syringes include systems comprising a prefilled vial attached to a pen-type syringe such as the NOVOLET® Novo Pen available from Novo Nordisk or the REDIPEN®, available from Schering Corporation, Kenilworth, NJ. Other syringe systems include a pen-type syringe comprising a glass cartridge containing a diluent and lyophilized pegylated interferon alpha powder in a separate compartment.

The scope of the present invention also includes combinations for the treatment or prevention of a medical condition or disease as set forth herein by administering a composition comprising an anti-IGF1 R antibody or antigen-binding fragment thereof of the invention in association with one or more other anti-cancer chemotherapeutic agents (*e.g.*, as described herein) and/or in association with one or more antiemetics including, but not limited to, casopitant (GlaxoSmithKline), Netupitant (MGI-Helsinn) and other NK-1 receptor antagonists, palonosetron (sold as Aloxi by MGI Pharma), aprepitant (sold as Emend by Merck and Co.; Rahway, NJ), diphenhydramine (sold as Benadryl® by Pfizer; New York, NY), hydroxyzine (sold as Atarax® by Pfizer; New York, NY), metoclopramide (sold as Regland® by AH Robins Co,; Richmond, VA), lorazepam (sold as Ativan® by Wyeth; Madison, NJ), alprazolam (sold as Xanax® by Pfizer; New York, NY), haloperidol (sold as Haldol® by Ortho-McNeil; Raritan, NJ), droperidol (Inapsine®), dronabinol (sold as Marinol® by Solvay Pharmaceuticals, Inc.; Marietta, GA), dexamethasone (sold as Decadron® by Merck and Co.; Rahway, NJ), methylprednisolone (sold as Medrol® by Pfizer; New York, NY), prochlorperazine (sold as Compazine® by Glaxosmithkline; Research Triangle Park, NC), granisetron (sold as Kytril® by Hoffmann-La Roche Inc.; Nutley, NJ), ondansetron (sold as Zofran® by by Glaxosmithkline; Research Triangle Park, NC), dolasetron (sold as Anzemet® by Sanofi-Aventis; New York, NY), tropisetron (sold as Navoban® by Novartis; East Hanover, NJ).

Compositions comprising an antiemetic are useful for preventing or treating nausea; a common side effect of anti-cancer chemotherapy. Accordingly, the present invention includes combinations for treating or preventing cancer in a subject by administering an antibody or antigen-binding fragment thereof of the invention optionally in association with one or more other chemotherapeutic agents (*e.g.*, as described herein) and/or optionally in association with one or more antiemetics.

Other side effects of cancer treatment include red and white blood cell deficiency. Accordingly, the present invention includes compositions comprising an anti-IGF1 R antibody or antigen-binding fragment thereof optionally in association with an agent which treats or prevents such a deficiency, such as, *e.g.*, pegfilgrastim, erythropoietin, epoetin alfa or darbepoetin alfa.

Another side-effect of anti-cancer therapy is mucositis. Accordingly, the present invention provides compositions for treating or preventing a medical condition or disease as set forth herein by administering an anti-IGF1 R antibody or antigen-binding fragment thereof in association with an anti-mucositis treatment. For example, mucositis treatments include swishing and gargling the anesthetic gel viscous Xylocaine® (lidocaine) 2%; or mouth washes including allopurinol, benzydamine hydrochloride, corticosteroids and/or chamomile.

The present invention further comprises a combination for treating or preventing any stage or type of any medical condition set forth herein by administering an antibody or antigen-binding fragment thereof of the invention in association with a therapeutic procedure such as surgical tumorectomy or anti-cancer radiation treatment; optionally in association with a further chemotherapeutic agent and/or antiemetic, for example, as set forth above.

The term "in association with" indicates that the components of a composition of the invention (*e.g*., anti-IGF1R antibody or antigen-binding fragment thereof along with docetaxel) can be formulated into a single composition for simultaneous delivery or formulated separately into two or more compositions (*e.g*., a kit). Furthermore, each component can be administered to a subject at a different time than when the other component is administered; for example, each administration may be given non-simultaneously (*e.g*., separately or sequentially) at several intervals over a given period of time. Moreover, the separate components may be administered to a subject by the same or by a different route (*e.g*., wherein an anti-IGF1 R antibody is administered parenterally and gefitinib is administered orally).

### Therapeutic Methods and Administration

The present invention discloses methods for using an IGF1R inhibitor, which is optionally in association with a further chemotherapeutic agent, or a pharmaceutically formulation thereof, for treating or preventing squamous cell carcinoma or renal cell cancer.

Pharmaceutical compositions comprising an IGF1R inhibitor in association with a further chemotherapeutic agent (*e.g.*, as set forth above) and a pharmaceutical acceptable carrier are also within the scope of the present invention (*e.g*., in a single composition or separately in a kit). The pharmaceutical compositions may be prepared by any methods well known in the art of pharmacy; see, *e.g*., Gilman, et al., (eds.) (1990), The Pharmacological Bases of Therapeutics, 8th Ed., Pergamon Press; A. Gennaro (ed.), Remington's Pharmaceutical Sciences, 18th Edition, (1990), Mack Publishing Co., Easton, Pennsylvania.; Avis, et al., (eds.) (1993) Pharmaceutical Dosage Forms: Parenteral Medications Dekker, New York; Lieberman, et al., (eds.) (1990) Pharmaceutical Dosage Forms: Tablets Dekker, New York; and Lieberman, et al., (eds.) (1990), Pharmaceutical Dosage Forms: Disperse Systems Dekker, New York. In an embodiment of the invention, the antibody is administered to a subject as part of a pharmaceutical composition comprising an antibody, such as LCF/HCA (*e.g.,* 1, 5, 10, 15 20 or 25 mg/ml), sodium acetate trihydrate (*e.g.,* 1.8, 2.3 or 3.1 mg/ml), glacial acetic acid (*e.g.,* 0.5, 0.18 or 2.2 mg/ml); sucrose (*e.g.,* 50 or 70 mg/ml); and water at a pH of about 5.5.

The term "squamous cell carcinoma" (SCC) includes any cancer involving the squamous cells of the skin. The term includes any subtype of squamous cell carcinoma including, for example, keratoacanthoma, carcinoma cuniculatum, invasive SCC, *in situ* SCC, or metastatic SCC.

The term "renal cell carcinoma" and the like is also called cancer of the kidney or renal adenocarcinoma and includes embodiments wherein cancer cells are found in any tissues of the kidney. The term includes, for example, any subtype of renal cell carcinoma including, for example, clear cell carcinomas (mixed with granular cells or not), chromophilic cancers, rhabdoid tumors of the kidney, chromophobic cancers, oncocytic cancers, collecting duct cancers, transitional cell carcinomas and sarcomatoid tumors.

The term "subject" or "patient" includes any organism, preferably a mammal (*e.g.,* primate, dog, horse, rat, mouse, cat, rabbit) and most preferably a human. In an embodiment of the invention, a "subject" or "patient" is an adult human (*e.g.,* 18 years or older) or a human child (*e.g.,* under 18 years of age, for example, less than 1, 1, 2, 3, 4, 5, 6, 7,8, 9 or 10 years of age).

A pharmaceutical composition containing an IGF1R inhibitor, which is optionally in association with a further chemotherapeutic agent, can be prepared using conventional pharmaceutically acceptable excipients and additives and conventional techniques. Such pharmaceutically acceptable excipients and additives include non-toxic compatible fillers, binders, disintegrants, buffers, preservatives, anti-oxidants, lubricants, flavorings, thickeners, coloring agents, emulsifiers and the like. All routes of administration are contemplated including, but not limited to, parenteral (*e.g.,* subcutaneous, intravenous, intraperitoneal, intramuscular, topical, intra-peritoneal, inhalation, intra-cranial) and non-parenteral (*e.g.,* oral, transdermal, intranasal, intraocular, sublingual, rectal and topical).

Injectables can be prepared in conventional forms, either as liquid solutions or suspensions, solid forms suitable for solution or suspension in liquid prior to injection, or as emulsions. The injectables, solutions and emulsions can also contain one or more excipients. Excipients include, for example, water, saline, dextrose, glycerol or ethanol. In addition, if desired, the pharmaceutical compositions to be administered may also contain minor amounts of non-toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins.

In an embodiment of the invention, pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles include Sodium Chloride Injection, Ringers Injection, Isotonic Dextrose Injection, Sterile Water Injection, Dextrose and Lactated Ringers Injection. Nonaqueous parenteral vehicles include fixed oils of vegetable origin, cottonseed oil, corn oil, sesame oil and peanut oil. Antimicrobial agents in bacteriostatic or fungistatic concentrations must be added to parenteral preparations packaged in multiple-dose containers which include phenols or cresols, mercurials, benzyl alcohol, chlorobutanol, methyl and propyl p-hydroxybenzoic acid esters, thimerosal, benzalkonium chloride and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcelluose, hydroxypropyl methylcellulose and polyvinylpyrrolidone. Emulsifying agents include Polysorbate 80 (TWEEN- 80). A sequestering or chelating agent of metal ions includes EDTA. Pharmaceutical carriers also include ethyl alcohol, polyethylene glycol and propylene glycol for water miscible vehicles; and sodium hydroxide, hydrochloric acid, citric acid or lactic acid for pH adjustment.

In an embodiment of the invention, preparations for parenteral administration can include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

Implantation of a slow-release or sustained-release system, such that a constant level of dosage is maintained is also contemplated herein. Briefly, an active agent (*e.g.,* IGF1R inhibitor, which is optionally in association with a further chemotherapeutic agent) is dispersed in a solid inner matrix, *e.g.,* polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, *e.g.,* polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylenelvinyloxyethanol copolymer, that is insoluble in body fluids. The compound diffuses through the outer polymeric membrane in a release rate controlling step. The percentage of active compound contained in such parenteral compositions is highly dependent on the specific nature thereof, as well as the activity of the IGF1 R inhibitor, which is optionally in association with a further chemotherapeutic agent, and the needs of the subject.

The concentration of the IGF1R inhibitor, which is optionally in association with a further chemotherapeutic agent, can be adjusted so that an injection provides an effective amount to produce the desired pharmacological effect. As discussed below, the exact dose depends on the age, weight and condition of the patient or animal as is known in the art.

In an embodiment of the invention, unit-dose parenteral preparations are packaged in an ampoule, a vial or a syringe with a needle. All preparations for parenteral administration must be sterile, as is known and practiced in the art.

In an embodiment of the invention, an IGF1R inhibitor, which is optionally in association with a further chemotherapeutic agent, is formulated into a lyophilized powder, which can be reconstituted for administration as solutions, emulsions and other mixtures. The powder may also be reconstituted and formulated as a solid or gel.

In an embodiment of the invention, the sterile, lyophilized powder is prepared by dissolving an IGF1R inhibitor (*e.g.,* an anti-IGF1R antibody), which is optionally in association with a further chemotherapeutic agent, or a pharmaceutically acceptable derivative thereof, in a suitable solvent. The solvent may contain an excipient which improves the stability or other pharmacological components of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at, in one embodiment, about neutral pH. Subsequent sterile filtration of the solution followed by lyophilization under standard conditions known to those of skill in the art provides a desirable formulation. In one embodiment, the resulting solution will be apportioned into vials for lyophilization. Each vial can contain a single dosage or multiple dosages of the IGF1 R inhibitor optionally in association with the further chemotherapeutic agent. Overfilling vials with a small amount above that needed for a dose or set of doses (*e.g.,* about 10%) is acceptable so as to facilitate accurate sample withdrawal and accurate dosing. The lyophilized powder can be stored under appropriate conditions, such as at about 4 °C to room temperature.

Reconstitution of a lyophilized powder with water for injection provides a formulation for use in parenteral administration. In an embodiment of the invention, for reconstitution, the lyophilized powder is added to sterile water or other suitable carrier. The precise amount depends upon the selected therapy being given. Such amount can be empirically determined.

Administration by inhalation can be provided by using, *e.g.,* an aerosol containing sorbitan trioleate or oleic acid, for example, together with trichlorofluoromethane, dichlorofluoromethane, dichlorotetrafluoroethane or any other biologically compatible propellant gas; it is also possible to use a system containing an IGF1R inhibitor, which is optionally in association with a further chemotherapeutic agent, by itself or associated with an excipient, in powder form.

In an embodiment of the invention, IGF1R inhibitor, which is optionally in association with a further chemotherapeutic agent, is formulated into a solid dosage form for oral administration, in one embodiment, into a capsule or tablet. Tablets, pills, capsules, troches and the like can contain one or more of the following ingredients, or compounds of a similar nature: a binder; a lubricant; a diluent; a glidant; a disintegrating agent; a coloring agent; a sweetening agent; a flavoring agent; a wetting agent; an emetic coating; and a film coating. Examples of binders include microcrystalline cellulose, gum tragacanth, glucose solution, acacia mucilage, gelatin solution, molasses, polvinylpyrrolidine, povidone, crospovidones, sucrose and starch paste. Lubricants include talc, starch, magnesium or calcium stearate, lycopodium and stearic acid. Diluents include, for example, lactose, sucrose, starch, kaolin, salt, mannitol and dicalcium phosphate. Glidants include, but are not limited to, colloidal silicon dioxide. Disintegrating agents include crosscarmellose sodium, sodium starch glycolate, alginic acid, corn starch, potato starch, bentonite, methylcellulose, agar and carboxymethylcellulose. Coloring agents include, for example, any of the approved certified water soluble FD and C dyes, mixtures thereof; and water insoluble FD and C dyes suspended on alumina hydrate. Sweetening agents include sucrose, lactose, mannitol and artificial sweetening agents such as saccharin, and any number of spray dried flavors. Flavoring agents include natural flavors extracted from plants such as fruits and synthetic blends of compounds which produce a pleasant sensation, such as, but not limited to peppermint and methyl salicylate. Wetting agents include propylene glycol monostearate, sorbitan monooleate, dieihylene glycol monolaurate and polyoxyethylene laural ether. Emetic-coatings include fatty acids, fats, waxes, shellac, ammoniated shellac and cellulose acetate phthalates. Film coatings include hydroxyethylcellulose, sodium carboxymethylcellulose, polyethylene glycol 4000 and cellulose acetate phthalate.

Any of the agents set forth herein can be formulated into a sustained release formulation including liposomal formulations such as unilamellar vesicular (ULV) and multilamellar vesicular (MLV) liposomes and DepoFoam™ particles (Kim et al., Biochim. Biophys. Acta (1983) 728(3):339-348; Kim, Methods Neurosci. (1994)21: 118-131; Kim et al., Anesthesiology (1996) 85(2): 331-338; Katre et al., J. Pharm. Sci. (1998) 87(11) : 1341-1346). A feature of the DepoFoam system is that, inside each DepoFoam particle, discontinuous internal aqueous chambers, bounded by a continuous, non-concentric network of lipid membranes render a higher aqueous volume-to-lipid ratio and much larger particle diameters compared with MLV.

### Dosage and Administration

Methods disclosed herein include administration of an IGF1R inhibitor, which is optionally in association with a further chemotherapeutic agent, or a pharmaceutical composition thereof. Typically, the administration and dosage of such agents is, when possible, done according to the schedule listed in the product information sheet of the approved agents, in the Physicians' Desk Reference 2003 (Physicians' Desk Reference, 57th Ed); Medical Economics Company; ISBN: 1563634457; 57th edition (November 2002), as well as therapeutic protocols well known in the art.

The term "therapeutically effective amount" or "therapeutically effective dosage" means that amount or dosage of a composition of the invention (e.g., IGF1R inhibitor, such as an anti-IGF1R antibody) that will elicit a biological or medical response of a tissue, system, subject or host that is being sought by the administrator (such as a researcher, doctor or veterinarian) which includes any measurable alleviation of the signs, symptoms and/or clinical indicia of cancer (including *e.g.,* inhibition of any IGF1R activity such as IGF-I or IGF-II binding or kinase activity), namely squamous cell carcinoma or renal cell cancer. For example a "therapeutically effective dosage" of any anti-IGF1R antibody; for example, an antibody or antigen-binding fragment thereof comprising (a) a light chain variable region comprising amino acids 20-128 of SEQ ID NO: 2 and a heavy chain variable region comprising amino acids 20-137 of SEQ ID NO: 10 or 12; (b) a light chain variable region comprising amino acids 20-128 of SEQ ID NO: 4 and a heavy chain variable region comprising amino acids 20-137 of SEQ ID NO: 10 or 12; (c) a light chain variable region comprising amino acids 20-128 of SEQ ID NO: 6 and a heavy chain variable region comprising amino acids 20-137 of SEQ ID NO: 10 or 12; or (d) a light chain variable region comprising amino acids 20-128 of SEQ ID NO: 8 and a heavy chain variable region comprising amino acids 20-137 of SEQ ID NO: 10 or 12; or any other anti-IGF1 R antibody mentioned herein is between about 0.3 and about 20 mg/kg of body weight (*e.g*., about 0.5 mg/kg body weight, about 1 mg/kg of body weight, about 2 mg/kg of body weight, about 3 mg/kg of body weight, about 4 mg/kg of body weight, about 5 mg/kg of body weight, about 6 mg/kg of body weight, about 7 mg/kg of body weight, about 8 mg/kg of body weight, about 9 mg/kg of body weight, about 10 mg/kg of body weight, about 11 mg/kg of body weight, about 12 mg/kg of body weight, about 13 mg/kg of body weight, about 14 mg/kg of body weight, about 15 mg/kg of body weight, about 16 mg/kg of body weight, about 17 mg/kg of body weight, about 18 mg/kg of body weight, about 19 mg/kg of body weight, about 20 mg/kg of body weight), twice per week, once per week, once every 2 weeks, once every 3 weeks or once a month.

Dosage regimens may be adjusted to provide the optimum desired response (*e.g*., a therapeutic response). For example, a single dose may be administered or several divided doses may be administered over time or the dose may be proportionally reduced or increased as indicated by exigencies of the therapeutic situation. For example, dosage may be determined or adjusted, by a practitioner of ordinary skill in the art (*e.g.,* physician or veterinarian) according to the patient's age, weight, height, past medical history, present medications and the potential for cross-reaction, allergies, sensitivities and adverse side-effects. It is especially advantageous to formulate parenteral compositions in dosage unit form for ease of administration and uniformity of dosage.

A practitioner (*e.g.,* physician or veterinarian) having ordinary skill in the art can readily determine and prescribe the effective amount of the pharmaceutical composition required. For example, the physician or veterinarian could start doses of the antibody or antigen-binding fragment of the invention employed in the pharmaceutical composition at levels lower than that required in order to achieve the desired therapeutic effect and gradually increase the dosage until the desired effect is achieved. The effectiveness of a given dose or treatment regimen of an antibody or combination of the invention can be determined , for example, by determining whether a tumor being treated in the subject shrinks or ceases to grow. The size of tumor can be easily determined, for example, by X-ray, magnetic resonance imaging (MRI), visually in a surgical procedure or manually by palpation. Tumor size and proliferation can also be measured by use of a thymidine PET scan (see *e.g.,* Wells et al., Clin. Oncol. 8: 7-14 (1996)). Generally, the thymidine PET scan includes the injection of a radioactive tracer, such as [2-¹¹C]-thymidine, followed by a PET scan of the patient's body (Vander Borght et a/., Gastroenterology 101: 794-799, 1991; Vander Borght et al., J. Radiat. Appl. Instrum. Part A, 42: 103-104 (1991)). Other tracers that can be used include [¹⁸F]-FDG (18-fluorodeoxyglucose), [¹²⁴I]IUdR (5-[124I]iodo-2'-deoxyuridine), [⁷⁶Br]BrdUrd (Bromodeoxyuridine), [¹⁸F]FLT (3'-deoxy-3'fluorothymidine] or [¹¹C]FMAU (2'-fluoro-5-methyl-1-ß-D-arabinofuranosyfuracil).

For example, squamous cell carcinoma progress can be monitored, by the physician or veterinarian by a variety of methods, and the dosing regimen can be altered accordingly. Methods by which to monitor squamous cell carcinoma include, for example, by patient interview or by physical examination (*e.g.,* visual inspection and documentation of any lesion's size, shape or other visual qualities).

For example, renal cell carcinoma progress can be monitored, by the physician or veterinarian by a variety of methods, and the dosing regimen can be altered accordingly. Methods by which to monitor renal cell carcinoma include, for example, palpation of the abdomen to detect a mass, complete blood count (CBC), urine test to detect red blood cells, assay of serum calcium levels to detect elevation, assay of Serum glutamate pyruvate transaminase (SGPT) and alkaline phosphatase to detect elevation, urine cytology assay, liver function tests, an ultrasound examination of the abdomen and kidney, kidney X-ray, intravenous pyelogram (IVP) or renal arteriography.

Compositions of the invention include an IGF1R inhibitor optionally "in association" with one or more chemotherapeutic agents. The term "in association" indicates that the components of the combinations of the invention can be formulated into a single composition for simultaneous delivery or formulated separately into two or more compositions (*e.g.,* a kit). Furthermore, each component of a combination of the invention can be administered to a subject at a different time than when the other component is administered; for example, each administration may be given non-simultaneously (*e.g.,* separately or sequentially) at several intervals over a given period of time. Moreover, the separate components may be administered to a subject by the same or by a different route (*e.g.,* orally, intravenously, subcutaneously).

### Examples

### Example 1: IGF1R and IGF-II are expressed in head and neck cancer cells.

In this example, head and neck cancer cells were assayed to determine the level of expression of *IGF1R* or *IGF-2.* Primary tumor tissue samples (from stage 2, 3 or 4 cancers), normal adjacent tissue samples and normal tissue samples were analyzed for the level of *IGF1R* RNA expression. Furthermore, the level of *IGF1R, IGF-1* and *IGF-2*, in various cell lines, were analyzed.

RNA was made from tumor samples and cDNAs were synthesized therefrom. Expression of *IGF1R* was analyzed using a 20 ng cDNA sample in a Fluorogenic 5'-nuclease PCR assay with specific probes and primers using the ABI Prism 7700 Sequence detection System 9 (Applied Biosystems; Foster City, CA). CT numbers were normalized against ubiqitin or actin mRNA expression in all samples.

The primers and probes used were as follows:
IGF1R/forward primer:GAAAGTGACGTCCTGCATTTCA (SEQ ID NO: 106)
IGF1R/reverse Primer: CCGGTGCCAGGTTATGATG (SEQ ID NO: 107)
Probe Sequence: CACCACCACGTCGAAGAATCGCA (SEQ ID NO: 108)
H322 is a non-small cell lung cancer cell line; CAL27, SCC25, SCC15, SCC9 and HS802 are squamous cell carcinoma cell lines.

The data generated in these methods is set forth in Figures 1 and 2. Figure 1 demonstrates that *IGF1R* was expressed, in primary head and neck tumor samples, at a higher level than that of normal tissue. Moreover, normal adjacent tissue (NAT) exhibited a greater level of *IGF1R* expression than that of normal tissue. The level of *IGF1R* expression increased slightly as the stage of head and neck tumor increased (from I to IV). Each point represents the normalized level of *IGF1R* in a single tissue sample.

Figure 2 also demonstrates that IGF1R, IGF-I and IGF-II were expressed in various lung and squamous cell carcinoma cell lines. The top panel is a western blot analysis of several cell lines demonstrating expression levels of total IGF1R (tIGF-1R) on the cell. Actin expression is also shown as an internal control. The bottom panel indicates the level of IGF1 and IGF2 secreted secreted from the cells assayed (ng secreted protein per 10⁶ cells).

### Example 2: Anti-IGF1R (LCF(κ)/HCA(γ1)) inhibits in vitro proliferation of squamous cell carcinoma cell lines

This example demonstrates that the anti-IGF1R antibody LCF/HCA inhibits proliferation of various squamous cell carcinoma (SCC) cell lines.

Cell proliferation was assayed using a Cell-Titer Glo assay (Promega Corp.; Madison, WI). A cell-titer glo assay assay generates a "glow" luminescent signal in the presence of ATP from viable cells, which can be detected with a plate reader luminometer or CCD imaging device.

In this assay, squamous cell carcinoma cells, SCC9, SCC25 and SCC15, were trypsinized, counted and resuspended at 25,000 cells/ml in 10% HI-FBS RPMI containing NEAA, L-Glu, MEM Vitamins and PS. 100ml of cell suspension (2500 cells) were added to each well BD Falcon 96 well black, clear bottom TC treated plate. Cells were allowed to attach and spread overnight at 37°C. Then, 10% RPMI medium was replaced with 100m12% RPMI, containing the LCF/HCA antibody at the appropriate concentration, the following day. The anti-IGF1 R antibody concentrations used in each experiment were 100 nM, 20 nM, 5 nM, 0.8 nM, 0.16 nM, 0.032 nM, 0.0064 nM, 0.00128 nM, and 0.000256 nM. All treatments were prepared in 2% RPMI at 20X concentration and serial diluted. Every test point was prepared in triplicate on separate assay plates. As mentioned above, cell proliferation was measured using the CellTiter-Glo Luminescent Cell Viability Assay (Promega) at 96 hours post treatment. Luminescence was detected on a Wallac 420 Plate Reader with stacker.

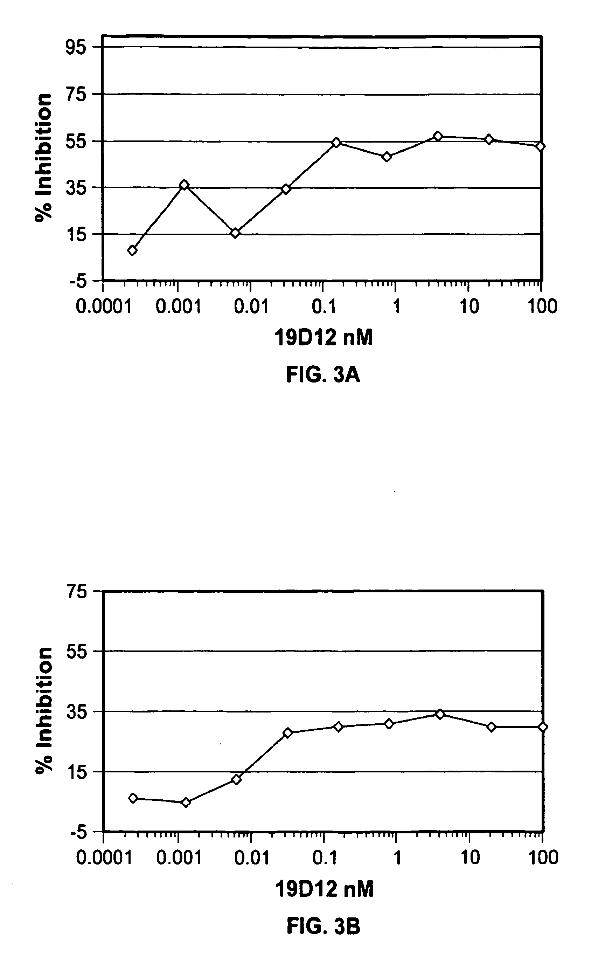

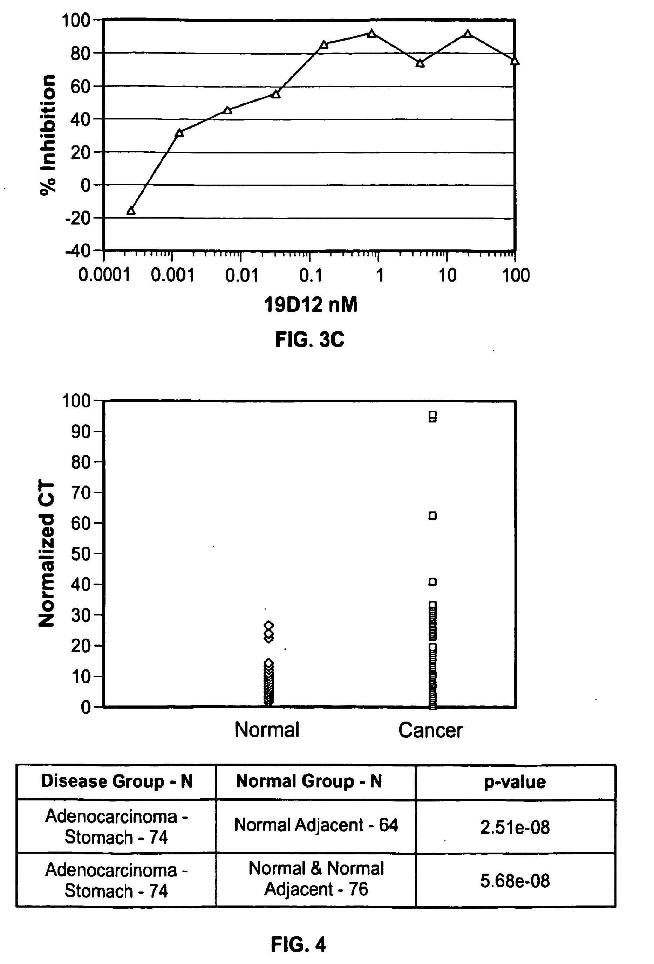

The results of this work is set forth in Figures 3a-3c which demonstrates that proliferation of squamous cell carcinoma cell lines was inhibited by exposure to various concentrations of LCF/HCA (indicated in the figures as 19D12). The data in figures (a), (b), and (c) correspond to the cell lines discussed above as follows: (a): cell line SCC 15; (b): cell line SCC 25; (c): cell line SCC 9.

### Example 3: IGF2 and IGF1R are highly expressed in gastric and ovarian cancer cell lines.

In this example, *IGF2* mRNA was demonstrated to be expressed at a high level in primary gastric tumor samples and in stomach adenocarcinoma cell lines as compared to normal samples and cell lines. High expression of IGF2 in a tumor sample is a marker for autocrine stimulation through the IGF1R growth pathway and indicates that the tumor's growth will be inhibited by anti-IGF1R (LCF(κ)/HCA(γ1)) antibody treatment.

Cell lines in which expression levels were to be determined were grown, prepared and assayed essentially as set forth above. Protein levels were analyzed by western blot and mRNA levels were analyzed by Taqman analysis. Quantitative flow cytometry was conducted using cells labeled with anti-IGF1 R, IR (insulin receptor), EGFR (epidermal growth factor receptor) or HER2 antibodies using the Quantitative Simply Cellular system of Bangs Laboratories, Inc. (Fishers, IN).

A2780 is a human ovarian cancer cell line and NCl-N87, SNU-16, SNU-1 and Hs746T are human gastric cancer cell lines.

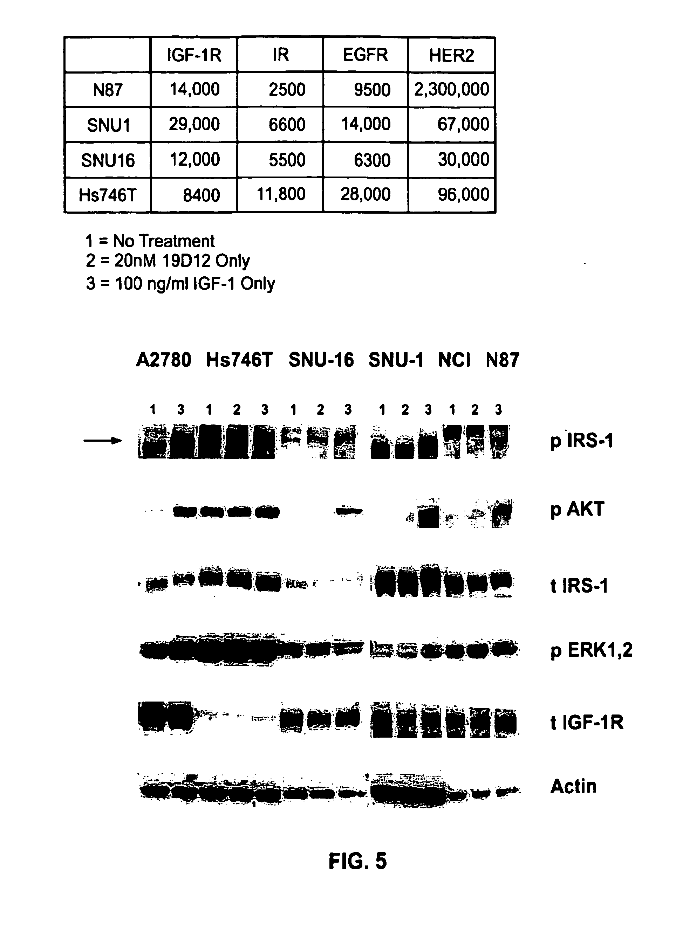

The results of this work are set forth in figures 4 and 5. Figure 4 demonstrates that IGF2 was expressed at high levels in primary gastric tumor cells as compared to that of normal cells and normal adjacent cells. Each point is the normalized level of *IGF2* mRNA expressed in a single tumor tissue sample (normalized against the expression levels of ubiquitin).

The Figure 5 top panel is quantitative flow cytometry data, relating to several cell lines, indicating the number of receptors/cell for IGF1 R, IR, EGFR and HER2. The figure 5 bottom panel is western blot data for several cell lines demonstrating the expression level of pIRS-1 (phosphorylated insulin receptor substrate-1 (IRS-1)), pAKT (phosphorylated AKT (also known as protein kinase-B) (non-isoform specific)), tIRS-1 (total IRS-1), pERK1, 2 (phosphorylated ERK1 (extracellular signal-regulated kinase 1) and ERK2 (extracellular signal-regulated kinase 2)), tIGR1 R (total IGFR1) and actin. The band under lane 3 of NCl N87 in the t IRS-1 row migrates slightly slower than expected suggesting that it is HER2 which cross reacts with the anti-IRS-1 antibodies used in this experiment.

### Example 4: Anti-IGF1R (LCF(κ)/HCA(γ1)) antibody inhibits in vitro proliferation of gastric carcinoma and myeloma cell lines.

This example demonstrates that proliferation of the gastric cancer cell line SNU-16 cells and the myeloma cell line RPMI8226 was inhibitied by the anti-IGF1 R antibody LCF/HCA. Cells were grown, prepared and assayed using the Cell-Titer Glo assay essentially as set forth above. The anti-IGF1 R antibody concentrations used in each experiment were 100 nm, 20 nm, 5 nm, 0.8 nm, 0.16 nm, 0.032 nm, 0.0064 nm, 0.00128 nm, and 0.000256 nM

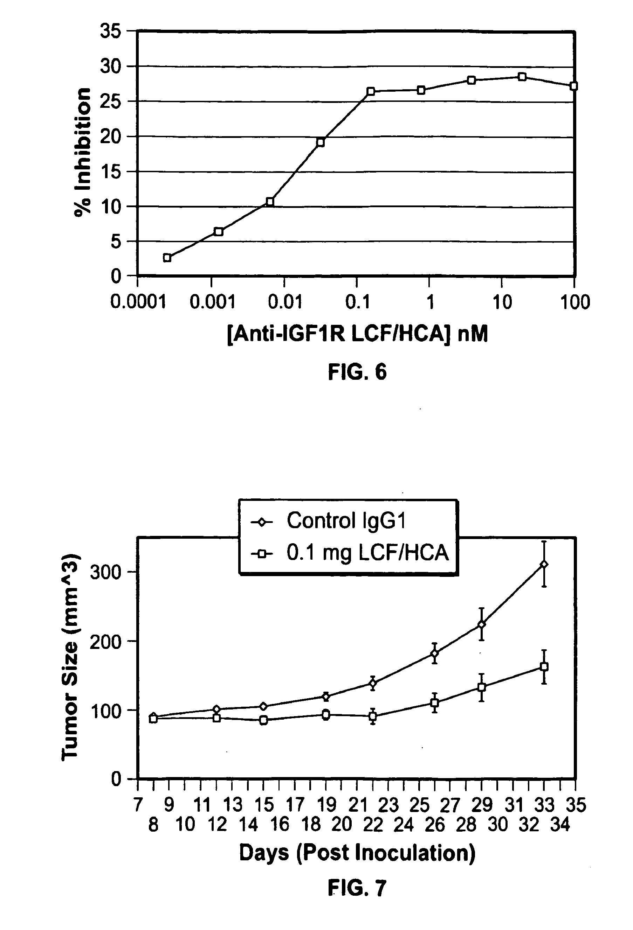

The results of this work are set forth in figure 6. Figure 6 demonstrates *in vitro* growth inhibition of the SNU-16 cell line at several concentrations of the antibody. A 40%-45% level of growth inhibition of the myeloma cell line was also observed *in vitro.*

### Example 5: Anti-IGF1R (LCF(κ)/HCA(γ1)) antibody inhibits in vivo renal cell carcinoma and in vitro melanoma tumor cell growth.

This example demonstrates the efficacy of anti-IGF1R therapy for the treatment or prevention of renal cell cancer or melanoma.

Athymic nude mice were inoculated with A498 human kidney carcinoma tumor cells in the right flank, subcutaneously, along with Matrigel (1:1::cells:gel). In these experiments, 5 x 10⁶ cells/mouse in a 1:1 mix with regular matrigel. The LCF/HCA anti-IGF1R antibody was inoculated intraperitoneally (ip) (0.1 mg/injection). Mice were dosed with antibody two times per week, by intraperitoneal injection, seven times starting on day 12. Tumor size was measured with calipers and the data was entered into the LABCAT data experimental collection and analysis program program (Innovative Programming Associates, Inc.; Princeton, NJ). Mice were grouped with average size of 100 mm³. Tumor size and body weight were measured twice weekly.

Human melanoma cells (A375-SM) were also assayed *in vitro* for responsiveness to anti-IGF1R antibody at 100 nM, 20 nM, 5 nM, 0.8 nM, 0.16 nM, 0.032 nM, 0.0064 nM, 0.00128 nM, and 0.000256 nM concentrations. Assays were conducted as set forth above by Cell-Titer Glo assay.

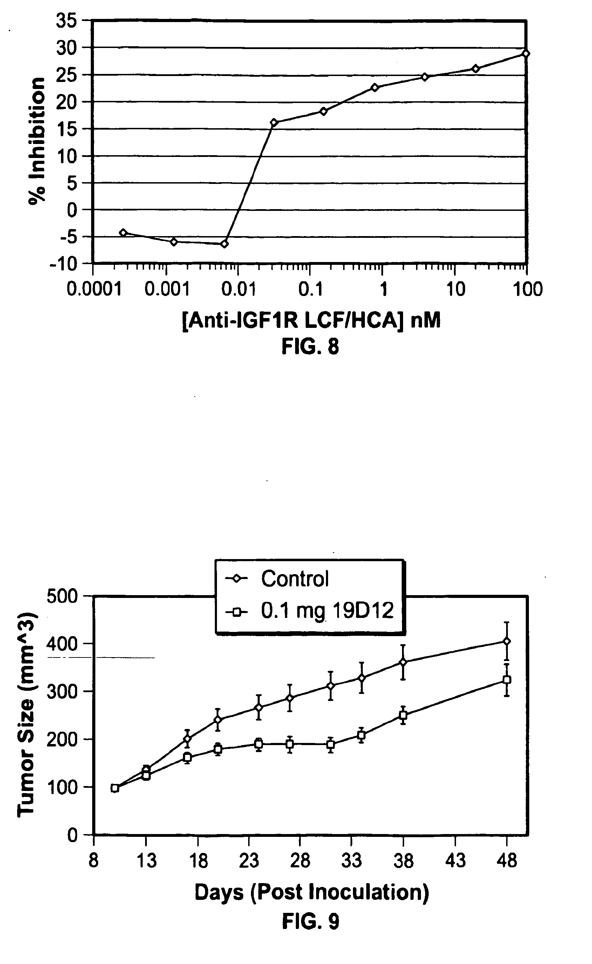

The results of this work are set forth in figures 7 and 8. Figure 7 demonstrates *in vivo* growth inhibition of the renal cell carcinoma cell line over time when the LCF/HCA antibody was administered. Figure 8 demonstrates the *in vitro* growth inhibition of the melanoma cell line at various concentions of LCF/HCA antibody.

### Example 6: Anti-IGF1R (LCF(κ)/HCA(γ1)) antibody inhibits in vivo squamous cell carcinoma cell growth.

This example demonstrates the efficacy of anti-IGF1R therapy for the treatment or prevention of head & neck cancers such as squamous cell carcinoma.

Immune-deficient SCID mice (strain SCID) were inoculated with SCC15 human squamous cell carcinoma tumor cells in the right flank, subcutaneously, along with Matrigel (1:1 cells:gel). In these experiments, 5 x 10⁶ cells/mouse in a 1:1 mix with regular matrigel were inoculated. The LCF/HCA anti-IGF1R antibody was inoculated intraperitoneally (ip) (0.1 mg/injection). Mice were dosed with the antibody two times per week, by intraperitoneal injection, seven times starting on day 10 with the last dose on day 31. Tumor size was measured with calipers and the data was entered into the labcat program. Mice were grouped with average size of 100 mm³. Tumor size and body weight were measured twice weekly.

The data generated from these experiments are set forth in figure 9. Tumor growth in the mice inoculated with the LCF/HCA antibody (indicated as "19D12" on the graph) was less than that observed in mice only receiving a control injection without the antibody.

### Example 7: PPTP panel testing of LCF(κ)1HCA(γ1) anti-IGF1R antibody.

LCF/HCA is a fully human antibody directed against the insulin-like growth factor 1 receptor (IGF1R), which is implicated in the growth and metastatic phenotype of a broad range of malignancies. IGF1 R signaling is of particular importance in the childhood cancer setting, *e.g.,* in connection with pediatric cancers such as neuroblastoma, Ewing sarcoma, rhabdomyosarcoma, Wilms tumor, and osteosarcoma. The activity of the antibody was evaluated against the *in vitro* and *in vivo panels* of the Pediatric Preclinical Testing Program (PPTP). PPTP includes a panel of *in vitro* cell line and *in vivo* mouse xenograft panels, established and organized by the National Cancer Institute (NCI), which allows testing of therapies in the most common pediatric cancers (see *e.g.,* Houghton et al., Pediatric Blood & Cancer (2007) 49(7):928-40).

The PPTP includes a molecularly characterized *in vitro* panel of cell lines (n=27) and *in vivo* panel of xenografts (n=61) representing most of the common types of childhood solid tumors and childhood ALL (acute lymphoblastic leukemia). LCF/HCA anti-IGF1R antibody was tested against the PPTP *in vitro* panel at concentrations ranging from 0.01 nM to 100 nM; and was tested against the PPTP *in vivo* panel at a dose of 0.5 mg per mouse administered twice weekly for four weeks via intraperitoneal injection. Three measures of antitumor activity were used: 1) response criteria modeled after the clinical setting; 2) treated-to-control (T/C) tumor volume at day 21; and 3) a time to event (4-fold increase in tumor volume) measure based on the median EFS of treated and control lines (intermediate activity required EFS (event-free survival) T/C > 2, and high activity additionally required a net reduction in median tumor volume at the end of the experiment).

### Methods

*In vitro* testing. *In vitro* testing was performed using DIMSCAN, a semiautomatic fluorescence-based digital image microscopy system that quantifies viable cell numbers (using fluorescein diacetate [FDA]) in tissue culture multiwell plates (Keshelava et al., Methods Mol.Med. (2005) 110:139-153). Testing was for 96 hours at concentrations from 0.01 nM to 0.1 mM with replicates of 6 per data point. Data were analyzed using Kaleidagraph software (Synergy Software; Reading, PA), fitting a non-linear regression, sigmoidal dose-response model to the response; relative fluorescence values vs. the concentration. The PPTP *in vitro* panel contains cell lines for neuroblastoma (4), Ewing sarcoma (4), rhabdomyosarcoma (4), acute lymphoblastic leukemia (5), NHL (2), and others.

Stage 1 testing involved testing an agent across the entire PPTP panel of childhood cancer xenograft lines at its maximum tolerated dose (MTD) or at a dose selected based on PK/PD (pharmacokinetic/pharmacodynamic) studies using adult preclinical models.

*In vivo* solid tumor testing: For each xenograft line, 10 mice bearing subcutaneous (SC) tumors initiated treatment when the tumors were between 0.2-0.5 cm³ in volume. Two perpendicular tumor diameters were measured at once weekly intervals with digital vernier calipers. Assuming tumors to be spherical, volumes were calculated from the formula (π/6)×d3, where d represented the mean diameter.

*In vivo* acute lymphoblastic leukemia testing: For each xenograft line, 8 mice were inoculated with 3-5 x 10⁶ mononuclear cells purified from the spleens of secondary recipient mice. Engraftment was monitored weekly by flow cytometry, and treatment was initiated when the proportion of human CD45+ cells in the peripheral blood reached 1%. The proportion of human CD45+ cells in the peripheral blood was monitored weekly throughout the course of treatment.

Drug: LCF/HCA was dissolved in 20mM sodium acetate pH 5 buffer containing 150mM sodium chloride, and administered intraperitoneally twice weekly for 4 consecutive weeks at a dose of 0.5 mg per animal. The antibody was provided to each testing site in coded vials for blinded testing according the PPTP's standard operating procedures.

### Solid Tumor Response Criteria:

| **Response** | **Definition** | **Score** |
|---|---|---|
| **PD1 (Progressive Disease 1)** | **>25% ↑ in tumor volume, TGD value ≤1.5** | **0** |
| **PD2 (Progressive Disease 2)** | **>25% ↑ in tumor volume, TGD value >1.5** | **2** |
| **SD (Stable Disease)** | **<25% ↑ in tumor volume, <50% regression** | **4** |
| **PR (Partial Response)** | **≥50% regression, but no CR** | **6** |
| **CR (Complete Response)** | **<0.1 cm3 tumor volume** | **8** |
| **MCR (Maintained CR)** | **<0.1 cm3 tumor volume at the end of study** | **10** |

**Leukemia Response Criteria:**

| **Response** | **Definition** | **Score** |
|---|---|---|
| PD1 (Progressive Disease 1) | No PR & TGD value of ≤1.5 & events at EOS | 0 |
| PD2 (Progressive Disease 2) | No PR & TGD value >1.5 & events at EOS | 2 |
| SD (Stable Disease) | No PR and no events at EOS | 4 |
| PR (Partial Response) | CD45% <1 % for only 1 week | 6 |
| CR (Complete Response) | CD45% <1 % for 2 consecutive weeks | 8 |
| MCR (Maintained CR) | CD45% <1 % for last 3 weeks of study | 10 |

Median Group Response: Each individual mouse in each treatment group was assigned a response score (see Tables above) and a median score for the treatment group was calculated and then each treatment group was assigned an overall response according to the table below.

| **If Median Score (MS) from (1):** | **Overall Group Response** |
|---|---|
| **0 ≤ MS ≤1** | **PD1** |
| **1 < MS ≤3** | **PD2** |
| **3 < MS ≤5** | **SD** |
| **5 < MS ≤7** | **PR** |
| **7 < MS ≤9** | **CR** |
| **9 < MS** | **MCR** |

Statistical Methods: Event-free survival (EFS) distributions of each treatment group were compared to the EFS distribution of the respective control group using the exact log rank test. P-values were 2-sided & were not adjusted for multiple comparisons given the exploratory nature of this study. P-values < 0.05 were considered to be significant.

### Results

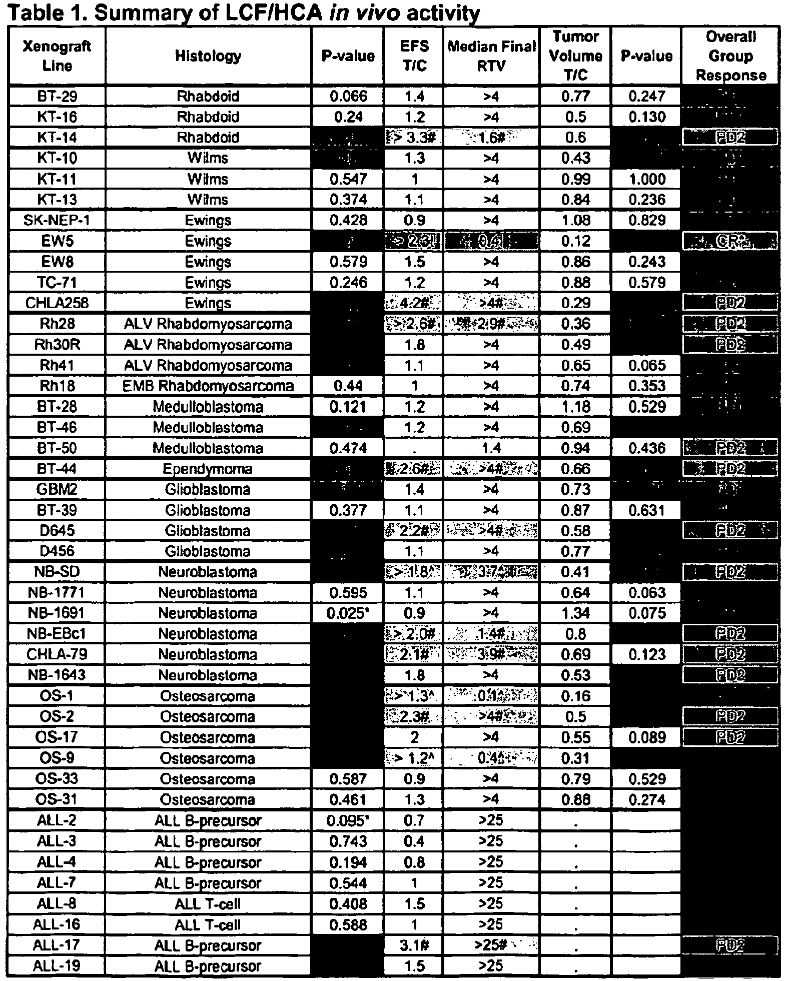

An * in the p-value columns indicates a statistically significant difference between treated and control groups. Notations in the EFS columns indicate xenografts that have either high (!), intermediate (#), or indeterminate (^) activity. RTV is relative tumor volume.

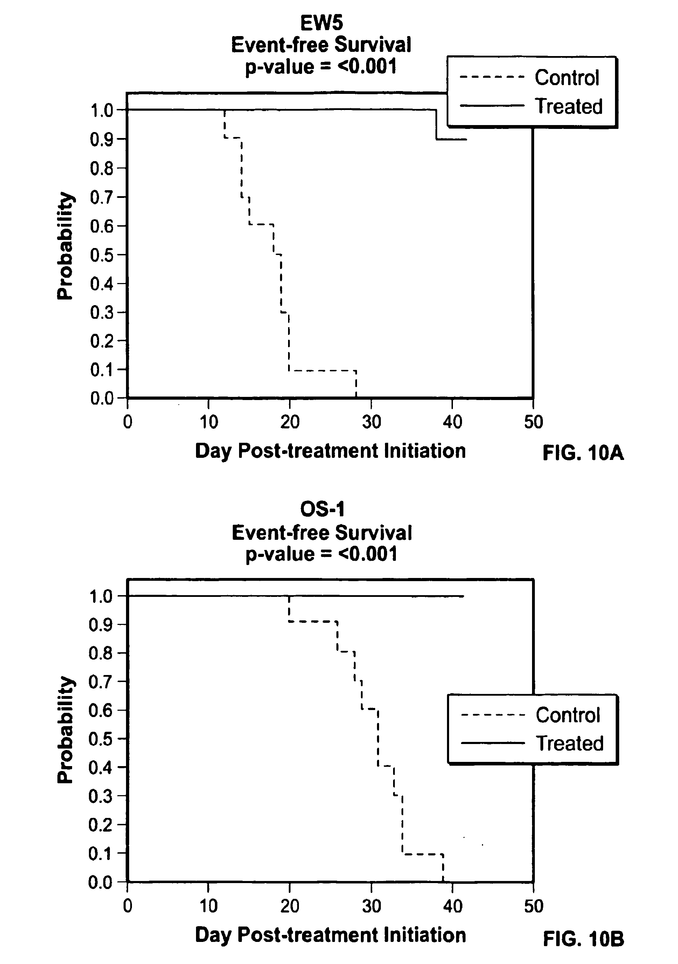

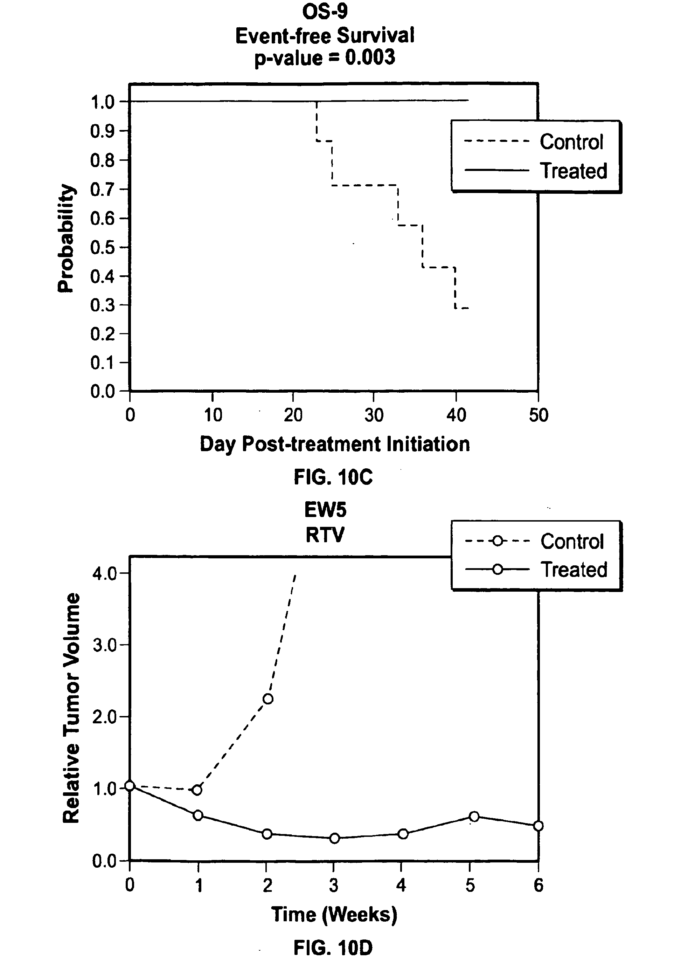

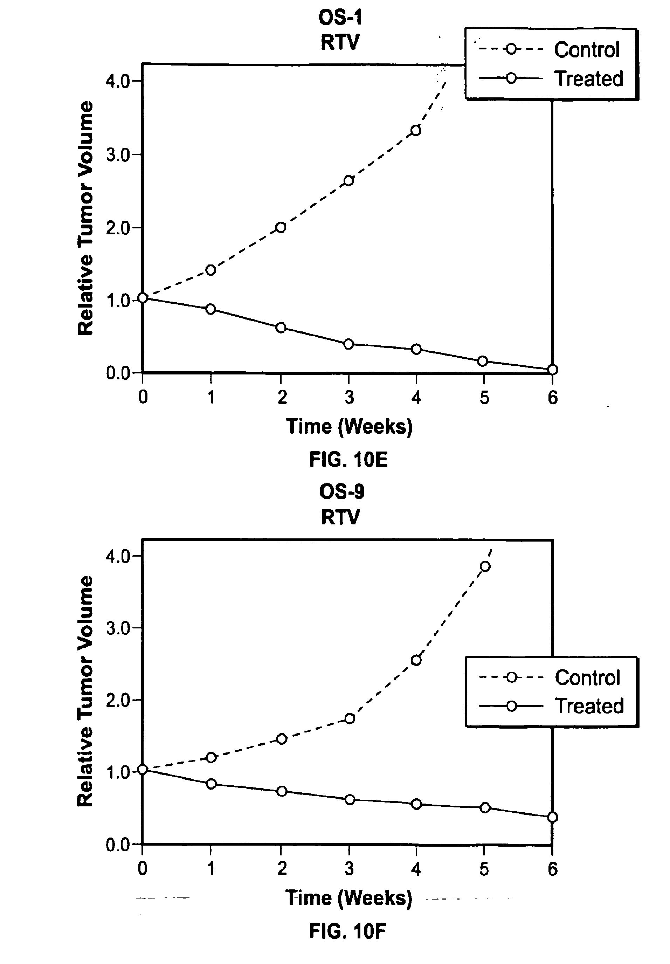

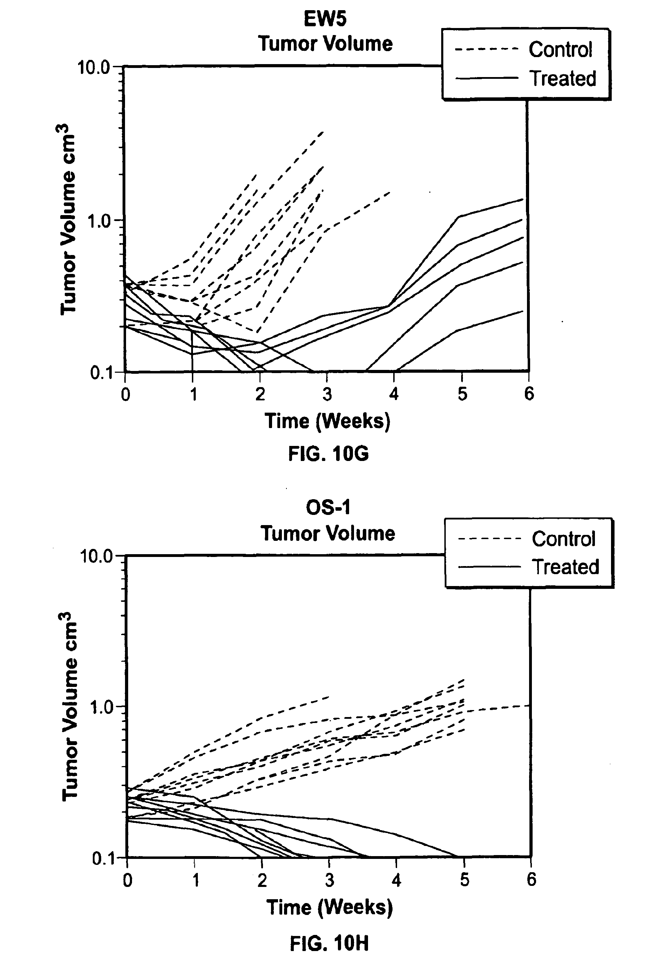

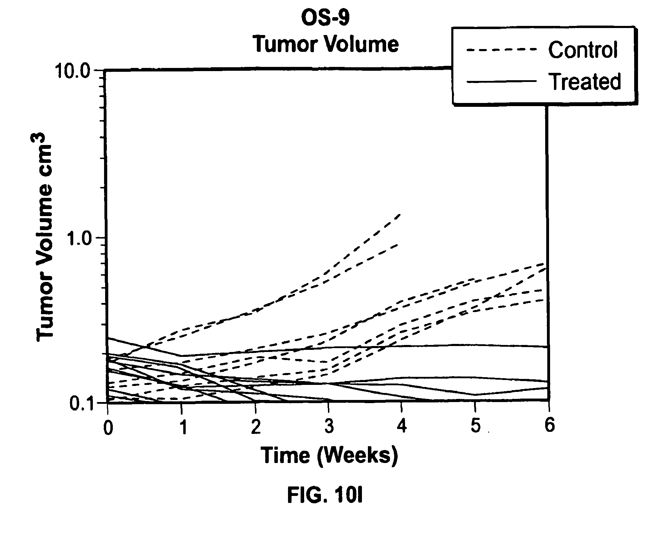

See also Figure 10 which sets forth a graphical analysis of the *in vivo* tumor volume of various cell types over time.

IGF-1R expression was also evaluated using Affymetrix U133 Plus2 Arrays.

Five xenografts with very low IGF1R expression did not respond (*i.e.,* PD1) to the antibody. For example, OS-33 had very low expression and was one of two osteosarcoma xenografts that showed no response to the antibody. The 3 xenografts with CR or MGR responses had high IGF1R expression. Some xenografts with moderate to high IGF1R expression did not respond to the antibody (*e.g.,* Rh41).

The maximal growth inhibition achieved in the *in vitro* assays was 30% and was observe for the T-cell ALL line, MOLT-4. The median growth inhibition for the *in vitro* panel, at the highest concentration tested, was 5%.

## Claims

1. An isolated antibody or antigen-binding fragment thereof comprising CDR-H1, CDR-H2 and CDR-H3 from a heavy chain immunoglobulin comprising the amino acid sequence set forth in SEQ ID NO: 10; or CDR-L1, CDR-L2 and CDRL3 from the a light chain immunoglobulin comprising the amino acid sequence set forth in SEQ ID NO: 8; or both, for use in treating or preventing a medical condition, by administration to a mammalian subject, wherein the medical condition is squamous cell carcinoma or renal cell cancer.

2. The isolated antibody or antigen-binding fragment thereof according to claim 1, for the use according to claim 1, wherein the antibody or antigen-binding fragment thereof comprises
(i)
CDR-L1 comprising the amino acid sequence: RASQSIGSSLH; and
CDR-L2 comprising the amino acid sequence: YASQSLS; and
CDR-L3 comprising the amino acid sequence: HQSSRLPHT;
or
(ii)
CDR-H1 comprising the amino acid sequence: SFAMH; and
CDR-H2 comprising the amino acid sequence: VIDTRGATYYADSVKG; and
CDR-H3 comprising the amino acid sequence: LGNFYYGMDV;
or both (i) and (ii).

3. The isolated antibody or antigen-binding fragment thereof according to claim 1, for the use according to claim 1, wherein the antibody or antigen-binding fragment thereof is a monoclonal antibody or antigen binding fragment thereof comprising a light chain immunoglobulin variable region comprising amino acids 20-128 of SEQ ID NO: 8; and a heavy chain immunoglobulin variable region comprising amino acids 20-137 of SEQ ID NO: 10 or 12.

4. The isolated antibody or antigen-binding fragment thereof according to claim 1, for the use according to claim 1, wherein the antibody or antigen binding fragment comprises:
(a) a light chain variable region comprising amino acids 20-128 of SEQ ID NO: 2 and a heavy chain variable region comprising amino acids 20-137 of SEQ ID NO: 10 or 12; or
(b) a light chain variable region comprising amino acids 20-128 of SEQ ID NO: 4 and a heavy chain variable region comprising amino acids 20-137 of SEQ ID NO: 10 or 12; or
(c) a light chain variable region comprising amino acids 20-128 of SEQ ID NO: 6 and a heavy chain variable region comprising amino acids 20-137 of SEQ ID NO: 10 or 12; or
(d) a light chain variable region comprising amino acids 20-128 of SEQ ID NO: 8 and a heavy chain variable region comprising amino acids 20-137 of SEQ ID NO: 10 or 12.

5. The isolated antibody or antigen-binding fragment thereof according to claim 1, for the use according to claim 1, wherein antibody or antigen-binding fragment is administered in association with one or more further chemotherapeutic agents or a pharmaceutical composition thereof.

6. The isolated antibody or antigen-binding fragment thereof according to claim 1, for the use according to claim 5, wherein the further chemotherapeutic agent is an anti-cancer agent, an anti-emetic agent, an anti-anemic agent or an anti-mucositis agent.

7. The isolated antibody or antigen-binding fragment thereof according to claim 1, for the use according to claim 6, wherein the further chemotherapeutic agent is one or more members selected from the group consisting of everolimus, trabectedin, cytosine arabinoside, 6-mecaptopurine, deoxycoformycin, calcitriol, valrubicin, mithramycin, vinblasfine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK1886619, topotecan, PTK787/ZK 222584, VX-745, PD 184352, rapamycin, 40-O-(2-hydroxyethyl)-rapamycin, temsirolimus, AP-23573, RAD001, ABT-578, BC-210, LY294002, LY292223, LY292696, LY293684, LY293646, wortmannin, ZM336372, L-779,450, PEG-filgrastim, darbepoetin, 5-fluorouracil, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonists, palonosetron, aprepitant, diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa and darbepoetin alfa

8. The isolated antibody or antigen-binding fragment thereof according to claim 1, for the use according to claim 5, wherein the antibody or antigen-binding fragment and the further chemotherapeutic agent are administered simultaneously.

9. The isolated antibody or antigen-binding fragment thereof according to claim 1, for the use according to claim 5, wherein the antibody or antigen-binding fragment and the further chemotherapeutic agent are administered non-simultaneously.

10. The isolated antibody or antigen-binding fragment thereof according to claim 3, for the use according to claim 1, wherein the light chain immunoglobulin variable region is linked to a kappa immunoglobulin constant region; and the heavy chain immunoglobulin variable region is linked to a gamma-1 immunoglobulin constant region.

11. The isolated antibody or antigen-binding fragment thereof according to claim 1, for the use according to claims 1, wherein the subject is a human.

12. The isolated antibody or antigen-binding fragment thereof according to claim 1, for the use according to claim 11, wherein the subject is a child.

13. The isolated antibody or antigen-binding fragment thereof according to claim 1, for the use according to claim 1, wherein the antibody or antigen-binding fragment is administered in association with an anti-cancer therapeutic procedure.

14. The isolated antibody or antigen-binding fragment thereof according to claim 1, for the use according to claim 13, wherein the anti-cancer therapeutic procedure is surgical tumorectomy and/or anti-cancer radiation treatment.

## Patentansprüche

1. Ein isolierter Antikörper oder ein antigenbindendes Fragment davon, der/das CDR-H1, CDR-H2 und CDR-H3 der schweren Kette des Immunglobulins, umfassend die in SEQ ID NO: 10 gezeigte Aminosäuresequenz, oder CDR-L1, CDR-L2 und CDR-L3 der leichten Kette des Immunglobulins, umfassend die in SEQ ID NO: 8 gezeigte Aminosäuresequenz, oder beides umfasst, zur Verwendung bei der Behandlung oder Prävention eines medizinischen Zustands durch Verabreichung an ein Säuger-Subjekt, wobei der medizinische Zustand Plattenepithelkarzinom oder Nierenzellkrebs ist.

2. Der isolierte Antikörper oder das antigenbindende Fragment davon gemäß Anspruch 1 zur Verwendung gemäß Anspruch 1, wobei der Antikörper oder das antigenbindende Fragment davon umfasst
(i)
CDR-L1, umfassend die Aminosäuresequenz: RASQSIGSSLH, und
CDR-L2, umfassend die Aminosäuresequenz: YASQSLS, und
CDR-L3, umfassend die Aminosäuresequenz: HQSSRLPHT,
oder
(ii)
CDR-H1, umfassend die Aminosäuresequenz: SFAMH, und
CDR-H2, umfassend die Aminosäuresequenz: VIDTRGATYYADSVKG, und
CDR-H3, umfassend die Aminosäuresequenz: LGNFYYGMDV,
oder sowohl (i) als auch (ii).

3. Der isolierte Antikörper oder das antigenbindende Fragment davon gemäß Anspruch 1 zur Verwendung gemäß Anspruch 1, wobei der Antikörper oder das antigenbindende Fragment davon ein monoklonaler Antikörper oder ein antigenbindendes Fragment davon ist, der/das eine variable Region der leichten Kette des Immunglobulins, umfassend die Aminosäuren 20 - 128 von SEQ ID NO: 8, und eine variable Region der schweren Kette des Immunglobulins, umfassend die Aminosäuren 20 - 137 von SEQ ID NO: 10 oder 12, umfasst.

4. Der isolierte Antikörper oder das antigenbindende Fragment davon gemäß Anspruch 1 zur Verwendung gemäß Anspruch 1, wobei der Antikörper oder das antigenbindende Fragment umfasst:
(a) eine variable Region der leichten Kette, umfassend die Aminosäuren 20 - 128 von SEQ ID NO: 2, und
eine variable Region der schweren Kette, umfassend die Aminosäuren 20 - 137 von SEQ ID NO: 10 oder 12, oder
(b) eine variable Region der leichten Kette umfassend die Aminosäuren 20 - 128 von SEQ ID NO: 4, und
eine variable Region der schweren Kette, umfassend die Aminosäuren 20 - 137 von SEQ ID NO: 10 oder 12, oder
(c) eine variable Region der leichten Kette, umfassend die die Aminosäuren 20 - 128 von SEQ ID NO: 6,
und eine variable Region der schweren Kette, umfassend die Aminosäuren 20 - 137 von SEQ ID NO: 10 oder 12, oder
(d) eine variable Region einer leichten Kette, umfassend die Aminosäuren 20 - 128 von SEQ ID NO: 8, und
eine variable Region der schweren Kette, umfassend die Aminosäuren 20 - 137 von SEQ ID NO: 10 oder 12.

5. Der isolierte Antikörper oder das antigenbindende Fragment davon gemäß Anspruch 1 zur Verwendung gemäß Anspruch 1, wobei der Antikörper oder das antigenbindende Fragment in Verbindung mit einem oder mehreren weiteren chemotherapeutischen Mitteln oder einer pharmazeutisch annehmbaren Zusammensetzung davon verabreicht wird.

6. Der isolierte Antikörper oder das antigenbindende Fragment davon gemäß Anspruch 1 zur Verwendung gemäß Anspruch 5, wobei das weitere chemotherapeutische Mittel ein Mittel gegen Krebs, ein Antiemetikum, ein Antianämikum oder ein Mittel gegen Mukositis ist.

7. Der isolierte Antikörper oder das antigenbindende Fragment davon gemäß Anspruch 1 zur Verwendung gemäß Anspruch 6, wobei das weitere chemotherapeutische Mittel ein oder mehrere Elemente ist, ausgewählt aus der Gruppe, bestehend aus Everolimus, Trabectedin, Cytosinarabinosid, 6-Mercaptopurin, Desoxycoformycin, Calcitriol, Valrubicin, Mithramycin, Vinblastin, Vinorelbin, Topotecan, Razoxin, Marimastat, COL-3, Neovastat, BMS-275291, Squalamin, Endostatin, SU5416, SU6668, EMD121974, Interleukin-12, IM862, Angiostatin, Vitaxin, Droloxifen, Idoxyfen, Spironolacton, Finasterid, Cimitidin, Trastuzumab, Denileukin-Difitox, Gefitinib, Bortezimib, Paclitaxel, Cremophor-freies Paclitaxel, Docetaxel, Epithilon B, BMS-247550, BMS-310705, Droloxifen, 4-Hydroxytamoxifen, Pipendoxifen, ERA-923, Arzoxifen, Fulvestrant, Acolbifen, Lasofoxifen, Idoxifen, TSE-424, HMR-3339, ZK186619, Topotecan, PTK787/ZK 222584, VX-745, PD 184352, Rapamycin, 40-O-(2-Hydroxyethyl)rapamycin, Temsirolimus, AP-23573, RAD001, ABT-578, BC-210, LY294002, LY292223, LY292696, LY293684, LY293646; Wortmannin, ZM336372, L-779450, PEG-Filgrastim, Darbepoetin, 5-Fluoruracil, Erythropoietin, Granulozyten-Kolonie-stimulierender Faktor, Zolendronat, Prednison, Cetuximab, Granulozyten-Makrophagen-Kolonie-stimulierender Faktor, Histrelin, pegyliertes Interferon alfa-2a, Interferon alfa-2a, pegyliertes Interferon alfa-2b, Interferon alfa-2b, Azacitidin, PEG-L-Asparaginase, Lenalidomid, Gemtuzumab, Hydrocortison, Interleukin-11, Dexrazoxan, Alemtuzumab, All-trans-Retinsäure, Ketoconazol, Interleukin-2, Megestrol, Immunglobulin, Stickstofflost, Methylprednisolon, Ibritgumomab-Tiuxetan, Androgene, Decitabin, Hexamethylmelamin, Bexaroten, Tositumomab, Arsentrioxid, Kortison, Editronat, Mitotan, Cyclosporin, Liposomal-Daunorubicin, Edwina-Asparaginase, Strontium 89, Casopitant, Netupitant, einem NK-1-Rezeptor-Antagonist, Palonosetron, Aprepitant, Diphenhydramin, Hydroxyzin, Metoclopramid, Lorazepam, Alprazolam, Haloperidol, Droperidol, Dronabinol, Dexamethason, Methylprednisolon, Prochlorperazin, Granisetron, Ondansetron, Dolasetron, Tropisetron, Pegfilgrastim, Erythropoietin, Epoetin-alfa und Darbepoetin-alfa.

8. Der isolierte Antikörper oder das antigenbindende Fragment davon gemäß Anspruch 1 zur Verwendung gemäß Anspruch 5, wobei der Antikörper oder das antigenbindende Fragment und das weitere chemotherapeutische Mittel gleichzeitig verabreicht werden.

9. Der isolierte Antikörper oder das antigenbindende Fragment davon gemäß Anspruch 1 zur Verwendung gemäß Anspruch 5, wobei der Antikörper oder das antigenbindende Fragment und das weitere chemotherapeutische Mittel nicht gleichzeitig verabreicht werden.

10. Der isolierte Antikörper oder das antigenbindende Fragment davon gemäß Anspruch 3 zur Verwendung gemäß Anspruch 1, wobei die variable Region der leichten Kette des Immunglobulins an eine konstante Region des Kappa-Immunglobulins gebunden ist und die variable Reaktion der schweren Kette des Immunglobulins an eine konstante Region des Gamma-1-Immunglobulins gebunden ist.

11. Der isolierte Antikörper oder das antigenbindende Fragment davon gemäß Anspruch 1 zur Verwendung gemäß Anspruch 1, wobei das Subjekt ein Mensch ist.

12. Der isolierte Antikörper oder das antigenbindende Fragment davon gemäß Anspruch 1 zur Verwendung gemäß Anspruch 11, wobei das Subjekt ein Kind ist.

13. Der isolierte Antikörper oder das antigenbindende Fragment davon gemäß Anspruch 1 zur Verwendung gemäß Anspruch 1, wobei der Antikörper oder das antigenbindende Fragment ein Verbindung mit einem therapeutischen Verfahren gegen Krebs verabreicht wird.

14. Der isolierte Antikörper oder das antigenbindende Fragment davon gemäß Anspruch 1 zur Verwendung gemäß Anspruch 13, wobei das therapeutische Verfahren gegen Krebs eine operative Tumorektomie und/oder eine Strahlenbehandlung gegen Krebs ist.

## Revendications

1. Anticorps isolé ou fragment de liaison à l'antigène de celui-ci comprenant CDR-H1, CDR-H2 et CDR-H3 d'une chaîne lourde d'immunoglobuline comprenant la séquence d'acides aminés présentée dans la SEQ ID NO: 10; ou bien CDR-L1, CDR-L2 et CDR-L3 d'une chaîne légère d'immunoglobuline comprenant la séquence d'acides aminés présentée dans la SEQ ID NO: 8, ou les deux, à utiliser dans le traitement ou la prévention d'une condition médicale, par l'administration à un sujet mammalien, où la condition médicale est un carcinome à cellules squameuses ou un cancer à cellules rénales.

2. Anticorps isolé ou fragment de liaison à l'antigène de celui-ci selon la revendication 1, à utiliser selon la revendication 1, où l'anticorps ou le fragment de liaison à l'antigène de celui-ci comprend:
(i)
CDR-L1 comprenant la séquence d'acides aminés: RASQSIGSSLH; et
CDR-L2 comprenant la séquence d'acides aminés: YASQSLS; et
CDR-L3 comprenant la séquence d'acides aminés: HQSSRLPHT;
ou
(ii)
CDR-H1 comprenant la séquence d'acides aminés: SFAMH; et
CDR-H2 comprenant la séquence d'acides aminés: VIDTRGATYYADSVKG; et
CDR-H3 comprenant la séquence d'acides aminés: LGNFYYGMDV;
ou les deux (i) et (ii).

3. Anticorps isolé ou fragment de liaison à l'antigène de celui-ci selon la revendication 1, à utiliser selon la revendication 1, où l'anticorps ou le fragment de liaison à l'antigène de celui-ci est un anticorps monoclonal ou un fragment de liaison à l'antigène de celui-ci comprenant une région variable de chaîne légère d'immunoglobuline comprenant les acides aminés 20-128 de la SEQ ID NO: 8; et une région variable de chaîne lourde d'immunoglobuline comprenant les acides aminés 20-137 de la SEQ ID NO: 10 ou 12.

4. Anticorps isolé ou fragment de liaison à l'antigène de celui-ci selon la revendication 1, à utiliser selon la revendication 1, où l'anticorps ou le fragment de liaison à l'antigène comprend:
(a) une région variable de chaîne légère comprenant les acides aminés 20-128 de la SEQ ID NO: 2 et une région variable de chaîne lourde comprenant les acides aminés 20-137 de la SEQ ID NO: 10 ou 12; ou bien
(b) une région variable de chaîne légère comprenant les acides aminés 20-128 de la SEQ ID NO: 4 et une région variable de chaîne lourde comprenant les acides aminés 20-137 de la SEQ ID NO: 10 ou 12; ou bien
(c) une région variable de chaîne légère comprenant les acides aminés 20-128 de la SEQ ID NO: 6 et une région variable de chaîne lourde comprenant les acides aminés 20-137 de la SEQ ID NO: 10 ou 12; ou bien
(d) une région variable de chaîne légère comprenant les acides aminés 20-128 de la SEQ ID NO: 8 et une région variable de chaîne lourde comprenant les acides aminés 20-137 de la SEQ ID NO: 10 ou 12.

5. Anticorps isolé ou fragment de liaison à l'antigène de celui-ci selon la revendication 1, à utiliser selon la revendication 1, où l'anticorps ou le fragment de liaison à l'antigène est administré en association avec un ou plusieurs agents chimiothérapeutiques supplémentaires ou une composition pharmaceutique de ceux-ci.

6. Anticorps isolé ou fragment de liaison à l'antigène de celui-ci selon la revendication 1, à utiliser selon la revendication 5, où l'agent chimiothérapeutique supplémentaire est un agent anticancéreux, un agent antiémétique, un agent antianémique ou un agent antimucite.

7. Anticorps isolé ou fragment de liaison à l'antigène de celui-ci selon la revendication 1, à utiliser selon la revendication 6, où l'agent chimiothérapeutique supplémentaire est un ou plusieurs membres sélectionnés parmi le groupe consistant en évérolimus, trabectédine, cytosine-arabinoside, 6-mercaptopurine, déoxycoformycine, calcitriol, valrubicine, mithramycine, vinblastine, vinorelbine, topotécan, razoxane, marimastat, COL-3, néovastat, BMS-275291, squalamine, endostatine, SU5416, SU6668, EMD121974, interleukine-12, IM862, angiostatine, vitaxine, droloxifène, idoxyfène, spironolactone, finastéride, cimitidine, trastuzumab, dénileukine diftitox, géfitinib, bortézimib, paclitaxel, paclitaxel sans crémophore, docétaxel, épithilone B, BMS-247550, BMS-310705, droloxifène, 4-hydrotamoxifène, pipendoxifène, ERA-923, arzoxifène, fulvestrant, acolbifène, lasofoxifène, idoxifène, TSE-424, HMR-3339, ZK186619, topotécan, PTK787/ZK222584, VX-745, PD 184352, rapamycine, 40-O-(2-hydroxyéthyle)-rapamycine, temsirolimus, AP-23573, RAD001, ABT-578, BC-210, LY294002, LY292223, LY292696, LY293684, LY293646, wortmannin, ZM336372, L-779,450, PEG-filgrastim, darbépoétine, 5-fluorouracile, érythropoïétine, facteur de stimulation des colonies de granulocytes, zolendronate, prednisone, cétuximab, facteur de stimulation des colonies de macrophages, histréline, interféron alfa-2a pégylé, interféron alfa-2a, interféron alfa-2b pégylé, interféron alfa-2b azacitidine, PEG-L-asparaginase, lénalidomide, gemtuzumab, hydrocortisone, interleukine 11, dexrazoxane, alemtuzumab, acide tout-trans rétinoïque, kétoconazole, interleukine 2, mégestrol, globuline immune, moutarde azotée, méthylprednisolone, ibritumomab tiuxétan, androgènes, décitabine, hexaméthylmélamine, bexarotène, tositumomab, trioxyde d'arsenic, cortisone, éditronate, mitotane, cyclosporine, daunorubicine liposomale, Edwina-asparaginase, strontium 89, casopitant, nétupitant, antagonistes d'un récepteur NK-1, palonosétron, aprépitant, diphenhydramine, hydroxyzine, métoclopramide, lorazépam, alprazolam, halopéridol, dropéridol, dronabinol, dexaméthasone, méthylprednisolone, prochlorpérazine, granisétron, ondansétron, dolasétron, tropisétron, pegfilgrastim, érythropoïétine, époétine alfa et darbépoétine alfa.

8. Anticorps isolé ou fragment de liaison à l'antigène de celui-ci selon la revendication 1, à utiliser selon la revendication 5, où l'anticorps ou le fragment de liaison à l'antigène et l'agent chimiothérapeutique supplémentaire sont administrés simultanément.

9. Anticorps isolé ou fragment de liaison à l'antigène de celui-ci selon la revendication 1, à utiliser selon la revendication 5, où l'anticorps ou le fragment de liaison à l'antigène et l'agent chimiothérapeutique supplémentaire sont administrés non simultanément.

10. Anticorps isolé ou fragment de liaison à l'antigène de celui-ci selon la revendication 3, à utiliser selon la revendication 1, où la région variable de la chaîne légère d'immunoglobuline est liée à une région constante d'immunoglobuline kappa et la région variable de la chaîne lourde d'immunoglobuline est liée à une région constante d'immunoglobuline gamma 1.

11. Anticorps isolé ou fragment de liaison à l'antigène de celui-ci selon la revendication 1, à utiliser selon la revendication 1, où le sujet est un être humain.

12. Anticorps isolé ou fragment de liaison à l'antigène de celui-ci selon la revendication 1, à utiliser selon la revendication 11, où le sujet est un enfant.

13. Anticorps isolé ou fragment de liaison à l'antigène de celui-ci selon la revendication 1, à utiliser selon la revendication 1, où l'anticorps ou fragment de liaison à l'antigène est administré en association avec une procédure thérapeutique anticancéreuse.

14. Anticorps isolé ou fragment de liaison à l'antigène de celui-ci selon la revendication 1, à utiliser selon la revendication 13, où la procédure thérapeutique anticancéreuse est une tumorectomie chirurgicale et/ou un traitement anticancéreux par rayonnements.
